# EUROPEAN PATENT APPLICATION

(11) **EP 4 137 488 A1**
(43) Date of publication of application: **22.02.2023**
(21) Application number: 22188641.9
(22) Date of filing: 04.08.2022
(51) Int. Cl.: C07D 405/04, C07D 405/10, C07D 405/14, C07D 409/04, C07D 409/10, C07D 409/14, C07D 491/048, C07D 495/04, H01L 51/00

(54) **HETEROCYCLIC COMPOUND, ORGANIC LIGHT EMITTING DEVICE COMPRISING SAME, COMPOSITION FOR ORGANIC LAYER OF ORGANIC LIGHT EMITTING DEVICE**

(30) Priority: 18.08.2021 KR 20210108741
(71) Applicant: LT Materials Co., Ltd., Yongin-City 17118 (KR)
(72) Inventor: HEO, Yu-Jin, 17118 Yongin-City, Gyeonggi-Do (KR); LEE, Sol, 17118 Yongin-City, Gyeonggi-Do (KR); LEE, Yong-Hui, 17118 Yongin-City, Gyeonggi-Do (KR); MO, Jun-Tae, 17118 Yongin-City, Gyeonggi-Do (KR); KIM, Dong-Jun, 17118 Yongin-City, Gyeonggi-Do (KR)
(74) Representative: Goddar, Heinz J.

(57) **Abstract**

The present specification provides a heterocyclic compound represented by Chemical Formula 1, an organic light emitting device including the same, and a composition for an organic material layer of the organic light emitting device.

## Description

### [Technical Field]

This application claims priority to and the benefits of Korean Patent Application No. 10-2021-0108741, filed with the Korean Intellectual Property Office on August 18, 2021, the entire contents of which are incorporated herein by reference.

The present specification relates to a heterocyclic compound, an organic light emitting device including the same and a composition for an organic material layer of the organic light emitting device.

### [Background Art]

An organic electroluminescence device is a kind of self-emitting type display device, and has an advantage in that the viewing angle is wide, the contrast is excellent, and the response speed is fast.

An organic light emitting device has a structure in which an organic thin film is disposed between two electrodes. When a voltage is applied to an organic light emitting device having the structure, electrons and holes injected from the two electrodes combine with each other in an organic thin film to make a pair, and then, emit light while being extinguished. The organic thin film may be composed of a single layer or multi layers, if necessary.

A material for the organic thin film may have a light emitting function, if necessary. For example, as the material for the organic thin film, it is also possible to use a compound, which may itself constitute a light emitting layer alone, or it is also possible to use a compound, which may serve as a host or a dopant of a host-dopant-based light emitting layer. In addition, as a material for the organic thin film, it is also possible to use a compound, which may perform a function such as hole injection, hole transport, electron blocking, hole blocking, electron transport or electron injection.

In order to improve the performance, service life, or efficiency of the organic light emitting device, there is a continuous need for developing a material for an organic thin film.

It is necessary to perform studies on an organic light emitting device including a compound having a chemical structure, which may satisfy conditions required for a material which is available for the organic light emitting device, for example, appropriate energy levels, electrochemical stability, thermal stability, and the like, and may perform various functions required for the organic light emitting device according to the substituent.

### [Related Art Document]

### [Patent Document]

(Patent Document 1) US Patent No. 4,356,429

### [Disclosure]

### [Technical Problem]

The present application relates to a heterocyclic compound, an organic light emitting device including the same and a composition for an organic material layer of the organic light emitting device.

### [Technical Solution]

In an exemplary embodiment of the present application, provided is a heterocyclic compound represented by the following Chemical Formula 1.

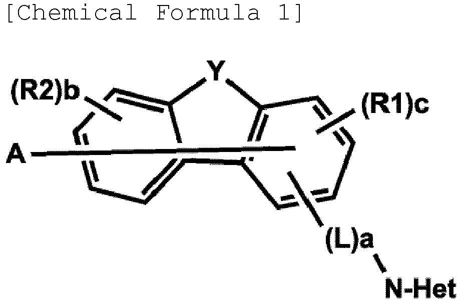

In Chemical Formula 1,
Y is O; or S,
N-Het is a heteroaryl group which is substituted or unsubstituted and includes at least one N,
L is a direct bond; a substituted or unsubstituted C6 to C60 arylene group; or a substituted or unsubstituted C2 to C60 heteroarylene group,
R1 and R2 are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R"; and a substituted or unsubstituted amine group, or two or more adjacent groups are bonded to each other to form a substituted or unsubstituted C6 to C60 aliphatic or aromatic hydrocarbon ring, or a substituted or unsubstituted C2 to C60 aliphatic or aromatic hetero ring,
b is an integer from 0 to 4, c is an integer from 0 to 3, and b and c satisfy 0≤b+c ≤6,
a is an integer from 0 to 4,
when a, b and c are 2 or higher, substituents in the parenthesis are the same as or different from each other,
A is represented by the following Chemical Formula 1-1 or 1-2,

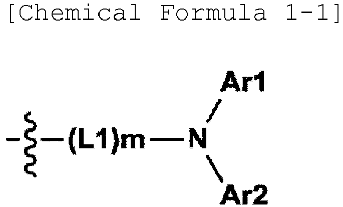

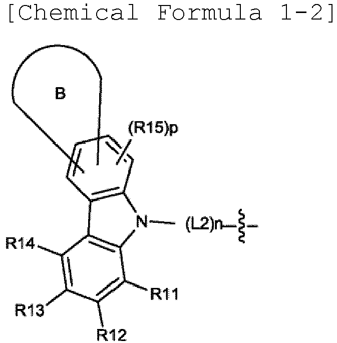

In Chemical Formulae 1-1 and 1-2,
Ar1 and Ar2 are the same as or different from each other, and are each independently selected from the group consisting of a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R"; and a substituted or unsubstituted amine group,
L1 and L2 are the same as or different from each other, and are each independently a direct bond; a substituted or unsubstituted C6 to C60 arylene group; or a substituted or unsubstituted C2 to C60 heteroarylene group,
R11 to R15 are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R"; and a substituted or unsubstituted amine group, or two or more adjacent groups are bonded to each other to form a substituted or unsubstituted C6 to C60 aliphatic or aromatic hydrocarbon ring, or a substituted or unsubstituted C2 to C60 aliphatic or aromatic hetero ring,
B is a substituted or unsubstituted C6 to C60 aliphatic or aromatic hydrocarbon ring; or a substituted or unsubstituted C2 to C60 aliphatic or aromatic hetero ring,
p is an integer from 0 to 2,
m and n are an integer from 0 to 4,
when p is 2 and when m and n are 2 or higher, substituents in the parenthesis are the same as or different from each other,
R, R' and R" are the same as or different from each other, and are each independently a substituted or unsubstituted C1 to C60 alkyl group; a substituted or unsubstituted C6 to C60 aryl group; or a substituted or unsubstituted C2 to C60 heteroaryl group, and
the deuterium content of Chemical Formula 1 is more than 0% and 100% or less.

Further, according to an exemplary embodiment of the present application, provided is an organic light emitting device including: a first electrode; a second electrode provided to face the first electrode; and an organic material layer having one or more layers provided between the first electrode and the second electrode, in which one or more layers of the organic material layer include the heterocyclic compound represented by Chemical Formula 1.

In addition, an exemplary embodiment of the present application provides an organic light emitting device in which an organic material layer including the heterocyclic compound of Chemical Formula 1 further includes: a compound represented by the following Chemical Formula A; or a compound represented by the following Chemical Formula B.

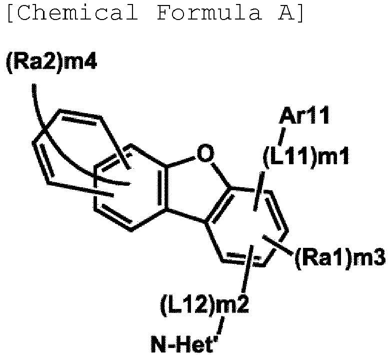

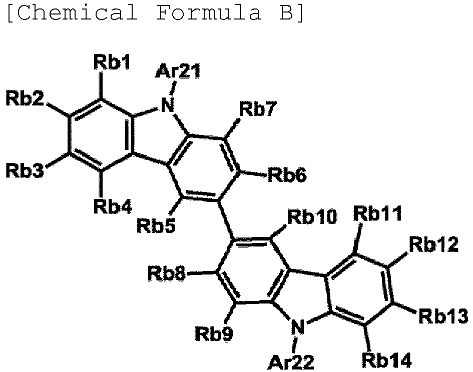

In Chemical Formulae A and B,
Ra1, Ra2 and Rb1 to Rb14 are the same as or different from each other, and are each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group, or two or more adjacent groups are bonded to each other to form a substituted or unsubstituted C6 to C60 aromatic hydrocarbon ring or a substituted or unsubstituted C2 to C60 hetero ring.
L11 and L12 are the same as or different from each other, and are each independently a direct bond; a substituted or unsubstituted C6 to C60 arylene group; or a substituted or unsubstituted C2 to C60 heteroarylene group,
N-Het' is a heteroaryl group which is substituted or unsubstituted and includes at least one N,
Ar11, Ar21 and Ar22 are the same as or different from each other, and each independently a substituted or unsubstituted C6 to C60 aryl group; or a substituted or unsubstituted C2 to C60 heteroaryl group,
m1 and m2 are an integer from 0 to 4,
m3 is an integer from 0 to 2,
m4 is an integer from 0 to 6, and
when m1, m2 and m4 are an integer of 2 or higher, or m3 is an integer of 2, substituents in the parenthesis are the same as or different from each other.

Furthermore, another exemplary embodiment of the present application provides a composition for an organic material layer of an organic light emitting device, which includes both the heterocyclic compound represented by Chemical Formula 1; and the compound represented by Chemical Formula A or the compound represented by Chemical Formula B.

Finally, an exemplary embodiment of the present application provides a method for manufacturing an organic light emitting device, the method including: preparing a substrate; forming a first electrode on the substrate; forming an organic material layer having one or more layers on the first electrode; and forming a second electrode on the organic material layer, in which the forming of the organic material layer includes forming the organic material layer having one or more layers by using the composition for an organic material layer according to an exemplary embodiment of the present application.

### [Advantageous Effects]

The compound described in the present specification can be used as a material for the organic material layer of the organic light emitting device. The compound can serve as a hole injection material, a hole transport material, a light emitting material, an electron transport material, an electron injection material, an electron blocking material, a hole blocking material, and the like in an organic light emitting device. In particular, the compound can be used as a light emitting material of an organic light emitting device, and a compound of Chemical Formula 1 can be used as a bipolar host.

Further, the heterocyclic compound represented by Chemical Formula 1 and the heterocyclic compound represented by Chemical Formula A or B can be used simultaneously as a material for a light emitting layer of an organic light emitting device. In this case, it is possible to lower a driving voltage of the device, improve the light efficiency, and particularly improve the service life characteristics of the device by the thermal stability of the compound.

### [Description of Drawings]

FIGS. 1 to 3 each are views schematically illustrating a stacking structure of an organic light emitting device according to an exemplary embodiment of the present application.

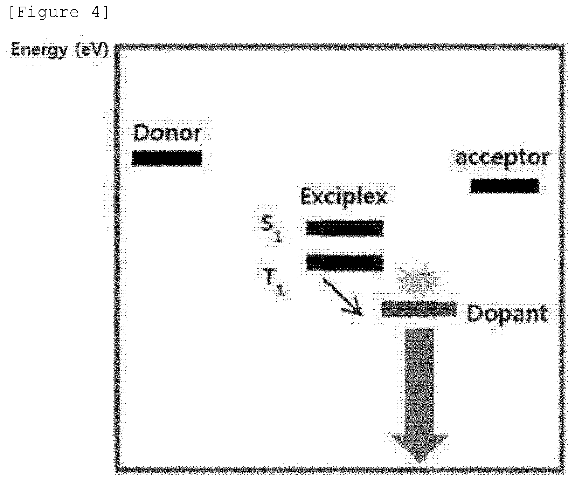

FIG. 4 is a view illustrating a description on the exciplex phenomenon.

### [Mode for Invention]

Hereinafter, the present application will be described in detail.

In the present specification, "when a substituent is not indicated in the structure of a chemical formula or compound" means that a hydrogen atom is bonded to a carbon atom. However, since deuterium (²H) is an isotope of hydrogen, some hydrogen atoms may be deuterium.

In an exemplary embodiment of the present application, "when a substituent is not indicated in the structure of a chemical formula or compound" may mean that all the positions that may be reached by the substituent are hydrogen or deuterium. That is, deuterium is an isotope of hydrogen, and some hydrogen atoms may be deuterium which is an isotope, and in this case, the content of deuterium may be 0% to 100%.

In an exemplary embodiment of the present application, in "the case where a substituent is not indicated in the structure of a chemical formula or compound", when deuterium is not explicitly excluded such as when the content of deuterium is 0% and the content of hydrogen is 100%, hydrogen and deuterium may be mixed and used in the compound. That is, the expression "substituent X is hydrogen" does not exclude deuterium such as the case where the content of hydrogen is 100% and the content of deuterium is 0%, and may mean a state in which hydrogen and deuterium are mixed.

In an exemplary embodiment of the present application, deuterium is one of the isotopes of hydrogen, is an element that has a deuteron composed of one proton and one neutron as a nucleus, and may be represented by hydrogen-2, and the element symbol may also be expressed as D or 2H.

In an exemplary embodiment of the present application, the isotope means an atom with the same atomic number (Z), but different mass numbers (A), and the isotope may be interpreted as an element which has the same number of protons, but different number of neutrons.

In an exemplary embodiment of the present application, when the total number of substituents of a basic compound is defined as T1 and the number of specific substituents among the substituents is defined as T2, the content T% of the specific substituent may be defined as T2/T1×100 = T%.

That is, in an example, the deuterium content of 20% in a phenyl group represented by 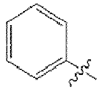 may be represented by 20% when the total number of substituents that the phenyl group can have is 5 (T1 in the formula) and the number of deuteriums among the substituents is 1 (T2 in the formula). That is, a deuterium content of 20% in the phenyl group may be represented by the following structural formula.

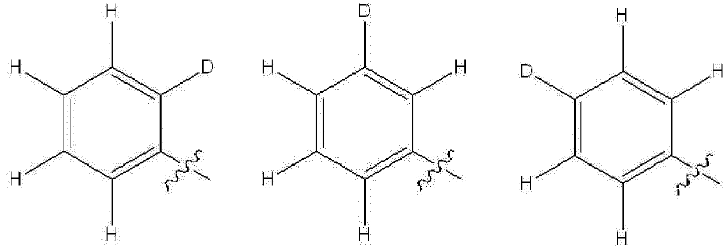

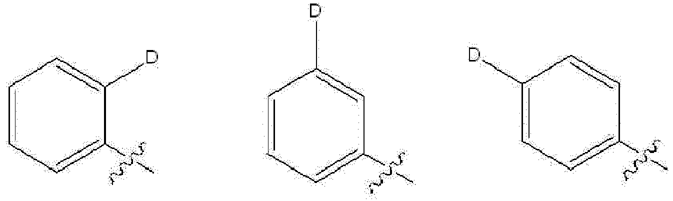

Further, in an exemplary embodiment of the present application, "a phenyl group having a deuterium content of 0%" may mean a phenyl group that does not include a deuterium atom, that is, has five hydrogen atoms.

In the present specification, the halogen may be fluorine, chlorine, bromine or iodine.

In the present specification, the alkyl group includes a straight-chain or branched-chain having 1 to 60 carbon atoms, and may be additionally substituted with another substituent. The number of carbon atoms of the alkyl group may be 1 to 60, specifically 1 to 40, and more specifically 1 to 20. Specific examples thereof include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like, but are not limited thereto.

In the present specification, the alkenyl group includes a straight-chain or branched-chain having 2 to 60 carbon atoms, and may be additionally substituted with another substituent. The number of carbon atoms of the alkenyl group may be 2 to 60, specifically 2 to 40, and more specifically 2 to 20. Specific examples thereof include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like, but are not limited thereto.

In the present specification, the alkynyl group includes a straight-chain or branched-chain having 2 to 60 carbon atoms, and may be additionally substituted with another substituent. The number of carbon atoms of the alkynyl group may be 2 to 60, specifically 2 to 40, and more specifically 2 to 20.

In the present specification, an alkoxy group may be straight-chained, branched, or cyclic. The number of carbon atoms of the alkoxy group is not particularly limited, but is preferably 1 to 20. Specific examples thereof include methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and the like, but are not limited thereto.

In the present specification, the cycloalkyl group includes a monocycle or polycycle having 3 to 60 carbon atoms, and may be additionally substituted with another substituent. Here, the polycycle means a group in which a cycloalkyl group is directly linked to or fused with another cyclic group. Here, another cyclic group may also be a cycloalkyl group, but may also be another kind of cyclic group, for example, a heterocycloalkyl group, an aryl group, a heteroaryl group, and the like. The number of carbon atoms of the cycloalkyl group may be 3 to 60, specifically 3 to 40, and more specifically 5 to 20. Specific examples thereof include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, and the like, but are not limited thereto.

In the present specification, the heterocycloalkyl group includes O, S, Se, N, or Si as a heteroatom, includes a monocycle or polycycle having 2 to 60 carbon atoms, and may be additionally substituted with another substituent. Here, the polycycle means a group in which a heterocycloalkyl group is directly linked to or fused with another cyclic group. Here, another cyclic group may also be a heterocycloalkyl group, but may also be another kind of cyclic group, for example, a cycloalkyl group, an aryl group, a heteroaryl group, and the like. The number of carbon atoms of the heterocycloalkyl group may be 2 to 60, specifically 2 to 40, and more specifically 3 to 20.

In the present specification, the aryl group includes a monocycle or polycycle having 6 to 60 carbon atoms, and may be additionally substituted with another substituent. Here, the polycycle means a group in which an aryl group is directly linked to or fused with another cyclic group. Here, another cyclic group may also be an aryl group, but may also be another kind of cyclic group, for example, a cycloalkyl group, a heterocycloalkyl group, a heteroaryl group, and the like. The aryl group includes a spiro group. The number of carbon atoms of the aryl group may be 6 to 60, specifically 6 to 40, and more specifically 6 to 25. Specific examples of the aryl group include a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused cyclic group thereof, and the like, but are not limited thereto.

In the present specification, the fluorenyl group may be substituted, and adjacent substituents may be bonded to each other to form a ring.

When the fluorenyl group is substituted, the substituent may be 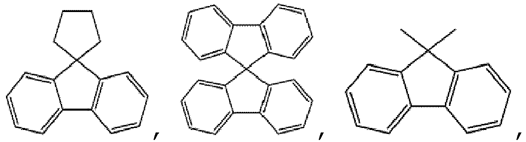 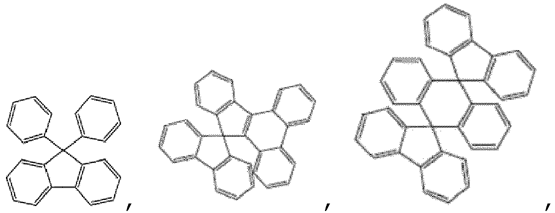 and the like, but is not limited thereto.

In the present specification, the heteroaryl group includes S, O, Se, N, or Si as a heteroatom, includes a monocycle or polycycle having 2 to 60 carbon atoms, and may be additionally substituted with another substituent. Here, the polycycle means a group in which a heteroaryl group is directly linked to or fused with another cyclic group. Here, another cyclic group may also be a heteroaryl group, but may also be another kind of cyclic group, for example, a cycloalkyl group, a heterocycloalkyl group, an aryl group, and the like. The number of carbon atoms of the heteroaryl group may be 2 to 60, specifically 2 to 40, and more specifically 3 to 25. Specific examples of the heteroaryl group include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a quinozolilyl group, a naphthyridyl group, an acridinyl group, a phenanthridinyl group, an imidazopyridinyl group, a diaza naphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi (dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepin group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, a 5,10-dihydrodibenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group, and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and the number of carbon atoms thereof is not particularly limited, but is preferably 1 to 30. Specific examples of the amine group include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group, and the like, but are not limited thereto.

In the present specification, an arylene group means that there are two bonding positions in an aryl group, that is, a divalent group. The above-described description on the aryl group may be applied to the arylene group, except that the arylene groups are each a divalent group. Further, a heteroarylene group means that there are two bonding positions in a heteroaryl group, that is, a divalent group. The above-described description on the heteroaryl group may be applied to the heteroarylene group, except for a divalent heteroarylene group.

In the present specification, a phosphine oxide group is represented by -P(=O)R101R102, and R101 and R102 are the same as or different from each other, and may be each independently a substituent composed of at least one of hydrogen; deuterium; a halogen group; an alkyl group; an alkenyl group; an alkoxy group; a cycloalkyl group; an aryl group; and a heterocyclic group. Specific examples of the phosphine oxide group include a diphenylphosphine oxide group, dinaphthylphosphine oxide, and the like, but are not limited thereto.

In the present specification, a silyl group includes Si and is a substituent to which the Si atom is directly linked as a radical, and is represented by -SiR104R105R106, and R104 to R106 are the same as or different from each other, and may be each independently a substituent composed of at least one of hydrogen; deuterium; a halogen group; an alkyl group; an alkenyl group; an alkoxy group; a cycloalkyl group; an aryl group; and a heterocyclic group. Specific examples of the silyl group include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but are not limited thereto.

In the present specification, the "adjacent" group may mean a substituent substituted with an atom directly linked to an atom in which the corresponding substituent is substituted, a substituent disposed to be sterically closest to the corresponding substituent, or another substituent substituted with an atom in which the corresponding substituent is substituted. For example, two substituents substituted at the ortho position in a benzene ring and two substituents substituted with the same carbon in an aliphatic ring may be interpreted as groups which are "adjacent" to each other.

Structures exemplified by the above-described cycloalkyl group, cycloheteroalkyl group, aryl group and heteroaryl group may be applied, except that an aliphatic or aromatic hydrocarbon ring or hetero ring which adjacent groups may form is not a monovalent group.

In the present specification, the term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more substituents are substituted, the two or more substituents may be the same as or different from each other.

In the present specification, "substituted or unsubstituted" means being unsubstituted or substituted with one or more substituents selected from the group consisting of deuterium, a cyano group, a halogen group, a C1 to C60 straight-chained or branched alkyl; a C2 to C60 straight-chained or branched alkenyl; a C2 to C60 straight-chained or branched alkynyl; a C3 to C60 monocyclic or polycyclic cycloalkyl; a C2 to C60 monocyclic or polycyclic heterocycloalkyl; a C6 to C60 monocyclic or polycyclic aryl; a C2 to C60 monocyclic or polycyclic heteroaryl; - SiRR'R"; -P(=O)RR'; a C1 to C20 alkylamine; a C6 to C60 monocyclic or polycyclic arylamine; and a C2 to C60 monocyclic or polycyclic heteroarylamine, or being unsubstituted or substituted with a substituent to which two or more substituents selected among the exemplified substituents are linked.

The present application relates to the heterocyclic compound of Chemical Formula 1.

The compound described in the present specification can be used as a material for the organic material layer of the organic light emitting device. The compound can serve as a hole injection material, a hole transport material, a light emitting material, an electron transport material, an electron injection material, an electron blocking material, a hole blocking material, and the like in an organic light emitting device. In particular, the compound can be used as a light emitting material of an organic light emitting device, and a compound of Chemical Formula 1 can be used as a bipolar host.

The bipolar host is a compound having the characteristics of bipolarity, and means a compound in which the bipolar host may act as an N-type as long as the characteristic of a compound to be combined together is a P-type, and the bipolar host serves as a P-type as long as the characteristic of a compound to be combined together is an N-type.

The heterocyclic compound of Chemical Formula 1 according to the present application is used as a bipolar host, and has the characteristics of bipolarity and thus electrical stability characteristics against holes and electrons, thereby having characteristics useful even for the injection and transport of holes and electrons.

In an exemplary embodiment of the present application, the deuterium content of the heterocyclic compound of Chemical Formula 1 may be more than 0% and 100% or less.

In another exemplary embodiment, the deuterium content of the heterocyclic compound of Chemical Formula 1 may be 5% or more, 10% or more, 15% or more, and 20% or more, and may satisfy a range of 100% or less.

In still another exemplary embodiment, provided is a heterocyclic compound in which the deuterium content of the heterocyclic compound of Chemical Formula 1 is 20% to 100%.

In an exemplary embodiment of the present application, the deuterium content of the heterocyclic compound of Chemical Formula 1 includes all the contents of deuterium included in the specific example compound of Chemical Formula 1 to be described below, and may be calculated and represented by X% based on the compound to be described below, and the range corresponds to one example of the range according to the present application.

That is, all the range of deuterium may be created based on the specific examples to be described below, but only a part of the examples are described in the present specification, and the content of deuterium may be calculated and expressed in various ways based on the specific examples to be described below, and the content of deuterium may be expressed as X% or more and X'% or less based on some specific examples.

In yet another exemplary embodiment, the deuterium content of the heterocyclic compound of Chemical Formula 1 may be 100%.

In an exemplary embodiment of the present application, Chemical Formula 1 may be represented by the following Chemical Formula 2 or 3.

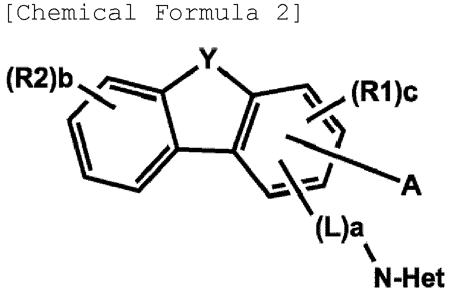

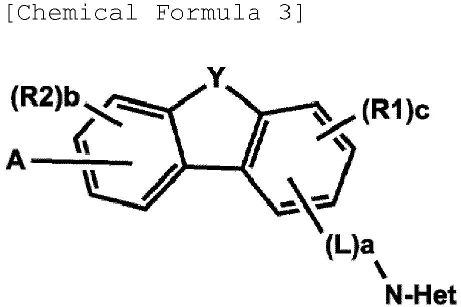

In Chemical Formulae 2 and 3,
the definitions of R1, R2, Y, L, N-Het, A, a, b and c are the same as the definitions in Chemical Formula 1.

In an exemplary embodiment of the present application, Chemical Formula 2 may be represented by any one of the following Chemical Formulae 2-1 to 2-4.

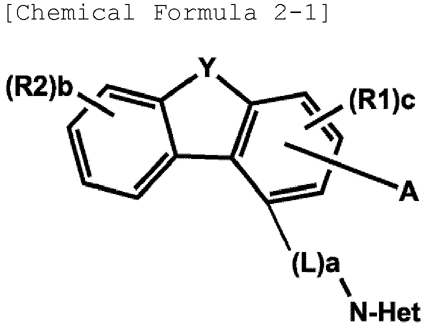

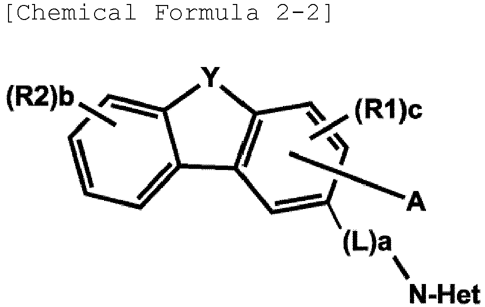

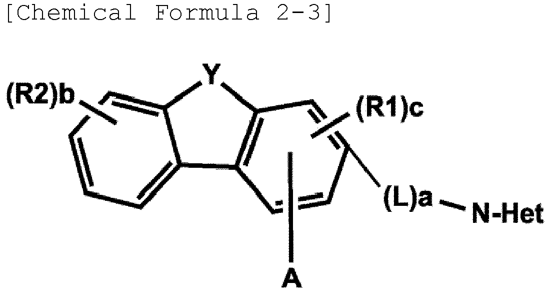

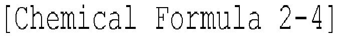

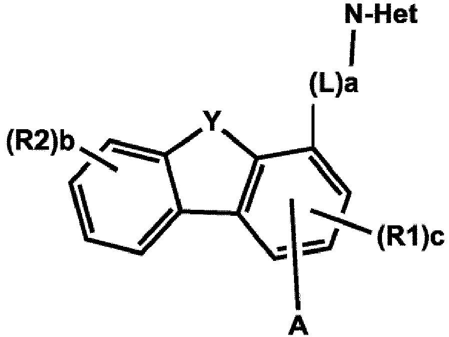

In Chemical Formulae 2-1 to 2-4,
the definitions of R1, R2, Y, L, N-Het, A, a, b and c are the same as the definitions in Chemical Formula 2. In an exemplary embodiment of the present application, Chemical Formula 3 may be represented by any one of the following Chemical Formulae 3-1 to 3-4.

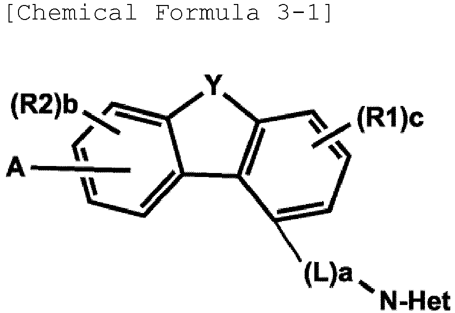

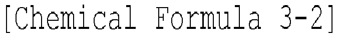

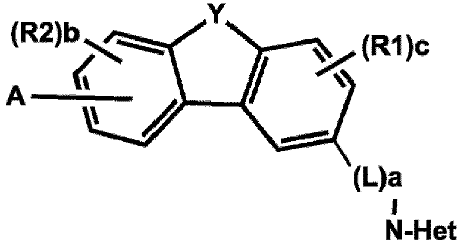

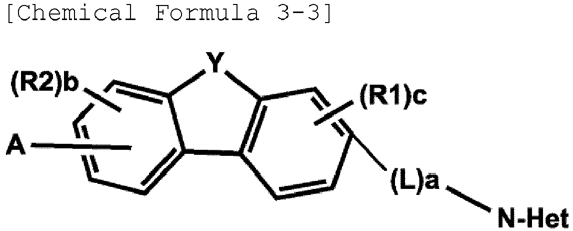

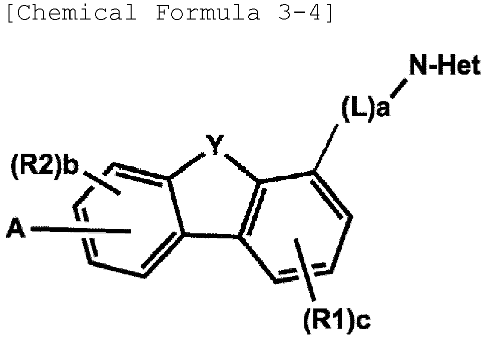

In Chemical Formulae 3-1 to 3-4,
the definitions of R1, R2, Y, L, N-Het, A, a, b and c are the same as the definitions in Chemical Formula 3.

In an exemplary embodiment of the present application, the structure of 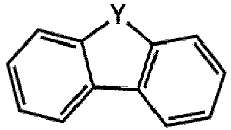 of Chemical Formula 1 may be represented by 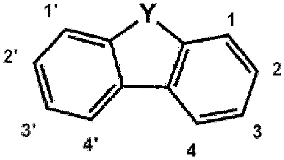 as a substituent position, and as an example, when a substituent represented by A is substituted at a No. 1 position and a group represented by -(L)a-N-Het is substituted at a No. 3 position, the following structure may be formed.

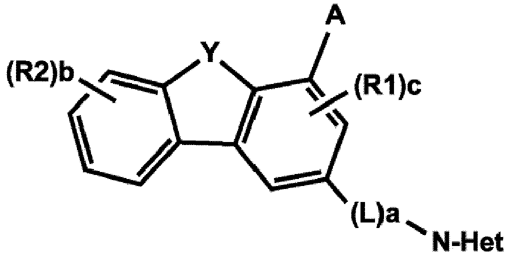

The substitution position definition of the substituent as described above may be equally applied to Chemical Formula 2, Chemical Formula 3, Chemical Formulas 2-1 to 2-4, and Chemical Formulas 3-1 to 3-4.

In an exemplary embodiment of the present application, R1 and R2 are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R"; and a substituted or unsubstituted amine group, or two or more adjacent groups may be bonded to each other to form a substituted or unsubstituted C6 to C60 aliphatic or aromatic hydrocarbon ring, or a substituted or unsubstituted C2 to C60 aliphatic or aromatic hetero ring.

In another exemplary embodiment, R1 and R2 are the same as or different from each other, and may be each independently hydrogen; deuterium; a substituted or unsubstituted C6 to C60 aryl group; or a substituted or unsubstituted C2 to C60 heteroaryl group.

In still another exemplary embodiment, R1 and R2 are the same as or different from each other, and may be each independently hydrogen; deuterium; a substituted or unsubstituted C6 to C40 aryl group; or a substituted or unsubstituted C2 to C40 heteroaryl group.

In yet another exemplary embodiment, R1 and R2 are the same as or different from each other, and may be each independently hydrogen; deuterium; a substituted or unsubstituted C6 to C20 aryl group; or a substituted or unsubstituted C2 to C20 heteroaryl group.

In yet another exemplary embodiment, R1 and R2 are the same as or different from each other, and may be each independently hydrogen; deuterium; a C6 to C20 aryl group; or a C2 to C20 heteroaryl group.

In yet another exemplary embodiment, R1 and R2 are the same as or different from each other, and may be each independently hydrogen; or deuterium.

In an exemplary embodiment of the present application, L may be a direct bond; a substituted or unsubstituted C6 to C60 arylene group; or a substituted or unsubstituted C2 to C60 heteroarylene group.

In another exemplary embodiment, L may be a direct bond; a substituted or unsubstituted C6 to C40 arylene group; or a substituted or unsubstituted C2 to C40 heteroarylene group.

In still another exemplary embodiment, L may be a direct bond; a C6 to C40 arylene group; or a C2 to C40 heteroarylene group.

In yet another exemplary embodiment, L may be a direct bond; or a C6 to C40 arylene group.

In yet another exemplary embodiment, L may be a direct bond; or a C6 to C20 arylene group.

In yet another exemplary embodiment, L may be a direct bond; a monocyclic C6 to C10 arylene group; or a polycyclic C10 to C20 arylene group.

In yet another exemplary embodiment, L may be a direct bond; a substituted or unsubstituted phenylene group; a substituted or unsubstituted biphenylene group; or a substituted or unsubstituted naphthalene group.

In yet another exemplary embodiment, L may be a direct bond; a phenylene group; a biphenylene group; or a naphthalene group.

In an exemplary embodiment of the present application, L may be further substituted with deuterium.

In an exemplary embodiment of the present application, N-Het may be a heteroaryl group which is substituted or unsubstituted, and includes at least one N.

In an exemplary embodiment of the present application, N-Het may be a heteroaryl group which is substituted or unsubstituted, and includes at least one or more and three or less N's.

In an exemplary embodiment of the present application, N-Het may be a heteroaryl group which is unsubstituted or substituted with one or more substituents selected from the group consisting of a C1 to C60 alkyl group; a C6 to C60 aryl group; and a C2 to C60 heteroaryl group, and includes at least one or more and three or less N's.

In an exemplary embodiment of the present application, N-Het may be a heteroaryl group which is unsubstituted or substituted with one or more substituents selected from the group consisting of a C1 to C60 alkyl group; a C6 to C60 aryl group; and a C2 to C60 heteroaryl group, and includes at least one or more and three or less N's.

In an exemplary embodiment of the present application, N-Het may be a substituted or unsubstituted pyridine group; a substituted or unsubstituted pyrimidine group; a substituted or unsubstituted triazine group; a substituted or unsubstituted quinoline group; a substituted or unsubstituted quinazoline group; a substituted or unsubstituted quinoxaline group; a substituted or unsubstituted benzofuro[3,2-d]pyrimidine group; or a substituted or unsubstituted benzo[4,5]thieno[3,2-d]pyrimidine group.

In an exemplary embodiment of the present application, N-Het may be a pyridine group unsubstituted or substituted with one or more substituents selected from the group consisting of a C6 to C60 aryl group and a C2 to C60 heteroaryl group; a pyrimidine group unsubstituted or substituted with one or more substituents selected from the group consisting of a C6 to C60 aryl group and a C2 to C60 heteroaryl group; a triazine group unsubstituted or substituted with one or more substituents selected from the group of a C6 to C60 aryl group and a C2 to C60 heteroaryl group; a quinoline group unsubstituted or substituted with one or more substituents selected from the group consisting of a C6 to C60 aryl group and a C2 to C60 heteroaryl group; a quinazoline group unsubstituted or substituted with one or more substituents selected from the group consisting of a C6 to C60 aryl group and a C2 to C60 heteroaryl group; a quinoxaline group unsubstituted or substituted with one or more substituents selected from the group consisting of a C6 to C60 aryl group and a C2 to C60 heteroaryl group; a benzofuro[3,2-d]pyrimidine group unsubstituted or substituted with one or more substituents selected from the group consisting of a C6 to C60 aryl group and a C2 to C60 heteroaryl group; or a benzo[4,5]thieno[3,2-d]pyrimidine group unsubstituted or substituted with one or more substituents selected from the group consisting of a C6 to C60 aryl group and a C2 to C60 heteroaryl group.

In an exemplary embodiment of the present application, N-Het may be further substituted with deuterium.

In an exemplary embodiment of the present application, the benzofuro[3,2-d]pyrimidine group and the benzo[4,5]thieno[3,2-d]pyrimidine group may have the following structure.

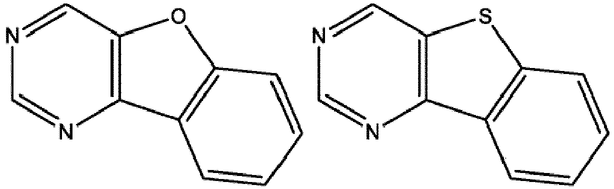

In an exemplary embodiment of the present application, Chemical Formula 1 is divided into and represented by the following Structural Formula A, Structural Formula B and Structural Formula C, and the deuterium content of the following Structural Formula A; the following Structural Formula B; the following Structural Formula C; the following Structural Formula A and the following Structural Formula B; the following Structural Formula A and the following Structural Formula C; the following Structural Formula B and the following Structural Formula C; and the following Structural Formulae A to C may be 100%.

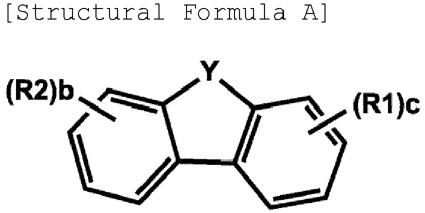

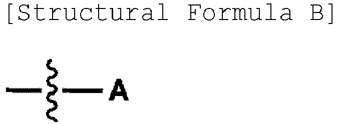

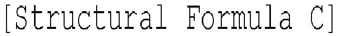

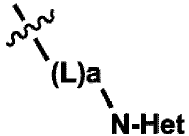

In Structural Formulae A to C,
the definition of each substituent is the same as the definition in Chemical Formula 1, and 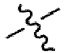 of Structural Formulae B and C each means a position bonded to Structural Formula A.

In an exemplary embodiment of the present application, the deuterium content of Chemical Formula A may be 0% to 100%.

In another exemplary embodiment, the deuterium content of Structural Formula A 20% to 100%.

In still another exemplary embodiment, the deuterium content of Structural Formula A 50% to 100%.

In yet another exemplary embodiment, the deuterium content of Structural Formula A may be 0% or 100%.

In an exemplary embodiment of the present application, the deuterium content of Structural Formula B may be 0% to 100%.

In another exemplary embodiment, the deuterium content of Structural Formula B may be 20% to 100%.

In still another exemplary embodiment, the deuterium content of Structural Formula B may be 50% to 100%.

In yet another exemplary embodiment, the deuterium content of Structural Formula B may be 0% or 100%.

In an exemplary embodiment of the present application, the deuterium content of Structural Formula C may be 0% to 100%.

In another exemplary embodiment, the deuterium content of Structural Formula C may be 20% to 100%.

In still another exemplary embodiment, the deuterium content of Structural Formula C may be 50% to 100%.

In yet another exemplary embodiment, the deuterium content of Chemical Formula C may be 0% or 100%.

In an exemplary embodiment of the present application, the deuterium content of the heterocyclic compound of Chemical Formula 1 satisfies the above range, the photochemical characteristics of a compound which includes deuterium and a compound which does not include deuterium are almost similar, but when deposited on a thin film, the deuterium-containing material tends to be packed with a narrower intermolecular distance.

Accordingly, when an electron only device (EOD) and a hole only device (HOD) are manufactured and the current density thereof according to voltage is confirmed, it can be confirmed that the compound of Chemical Formula 1 according to the present application, which includes deuterium, exhibits a much more balanced charge transport characteristics than the compound which does not include deuterium.

Further, when the surface of a thin film is observed using an atomic force microscope (AFM), it can be confirmed that the thin film made of a compound including deuterium is deposited with a more uniform surface without any aggregated portion.

Additionally, since the single bond dissociation energy of carbon and deuterium is higher than the single bond dissociation energy of carbon and hydrogen, the stability of the total molecules is enhanced as the deuterium content of the heterocyclic compound of Chemical Formula 1 according to the present application satisfies the above range, so that there is an effect that the service life of the device is improved.

When hydrogen is substituted with deuterium, the mass increases, but in this case, the compound has a lower vibrational energy than hydrogen to lower and stabilize the energy of the molecule. Therefore, since the bond dissociation energy is greater for carbon-deuterium bonds than for carbon-hydrogen bonds, the structure of the molecule is more stable when substituted with deuterium.

In an exemplary embodiment of the present application, A may be represented by the following Chemical Formula 1-1 or 1-2.

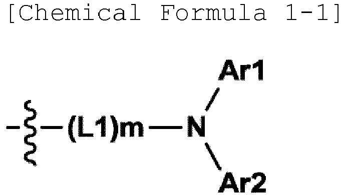

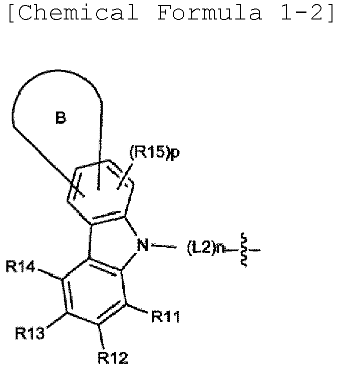

In Chemical Formulae 1-1 and 1-2,
Ar1 and Ar2 are the same as or different from each other, and are each independently selected from the group consisting of a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R"; and a substituted or unsubstituted amine group,
L1 and L2 are the same as or different from each other, and are each independently a direct bond; a substituted or unsubstituted C6 to C60 arylene group; or a substituted or unsubstituted C2 to C60 heteroarylene group,
R11 to R15 are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R"; and a substituted or unsubstituted amine group, or two or more adjacent groups are bonded to each other to form a substituted or unsubstituted C6 to C60 aliphatic or aromatic hydrocarbon ring, or a substituted or unsubstituted C2 to C60 aliphatic or aromatic hetero ring,
B is a substituted or unsubstituted C6 to C60 aliphatic or aromatic hydrocarbon ring; or a substituted or unsubstituted C2 to C60 aliphatic or aromatic hetero ring,
p is an integer from 0 to 2,
m and n are an integer from 0 to 4,
R, R' and R" are the same as or different from each other, and are each independently a substituted or unsubstituted C1 to C60 alkyl group; a substituted or unsubstituted C6 to C60 aryl group; or a substituted or unsubstituted C2 to C60 heteroaryl group.

In an exemplary embodiment of the present application, L1 and L2 are the same as or different from each other, and may be each independently a direct bond; a substituted or unsubstituted C6 to C60 arylene group; or a substituted or unsubstituted C2 to C60 heteroarylene group.

In another exemplary embodiment, L1 and L2 are the same as or different from each other, and may be each independently a direct bond; a substituted or unsubstituted C6 to C40 arylene group; or a substituted or unsubstituted C2 to C40 heteroarylene group.

In still another exemplary embodiment, L1 and L2 are the same as or different from each other, and may be each independently a direct bond; a C6 to C40 arylene group; or a C2 to C40 heteroarylene group.

In yet another exemplary embodiment, L1 and L2 are the same as or different from each other, and may be each independently a direct bond; or a C6 to C40 arylene group.

In yet another exemplary embodiment, L1 and L2 are the same as or different from each other, and may be each independently a direct bond; or a C6 to C20 arylene group.

In yet another exemplary embodiment, L1 and L2 are the same as or different from each other, and may be each independently a direct bond; a monocyclic C6 to C10 arylene group; or a polycyclic C10 to C20 arylene group.

In yet another exemplary embodiment, L1 and L12 are the same as or different from each other, and may be each independently a direct bond; a substituted or unsubstituted phenylene group; a substituted or unsubstituted biphenylene group; or a substituted or unsubstituted naphthalene group.

In yet another exemplary embodiment, L1 and L2 are the same as or different from each other, and may be each independently a direct bond; a phenylene group; a biphenylene group; or a naphthalene group.

In an exemplary embodiment of the present application, L1 and L2 may be further substituted with deuterium.

In an exemplary embodiment of the present application, Ar1 and Ar2 are the same as or different from each other, and may be each independently selected from the group consisting of a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R"; and a substituted or unsubstituted amine group,

In another exemplary embodiment, Ar1 and Ar2 are the same as or different from each other, and may be each independently selected from the group consisting of a substituted or unsubstituted C1 to C60 alkyl group; a substituted or unsubstituted C6 to C60 aryl group; and a substituted or unsubstituted C2 to C60 heteroaryl group.

In still another exemplary embodiment, Ar1 and Ar2 are the same as or different from each other, and may be each independently selected from the group consisting of a substituted or unsubstituted C1 to C40 alkyl group; a substituted or unsubstituted C6 to C40 aryl group; and a substituted or unsubstituted C2 to C40 heteroaryl group.

In yet another exemplary embodiment, Ar1 and Ar2 are the same as or different from each other, and may be each independently selected from the group consisting of a substituted or unsubstituted C1 to C20 alkyl group; a substituted or unsubstituted C6 to C20 aryl group; and a substituted or unsubstituted C2 to C20 heteroaryl group.

In yet another exemplary embodiment, Ar1 and Ar2 are the same as or different from each other, and may be each independently selected from the group consisting of a C1 to C20 alkyl group; a C6 to C20 aryl group; and a C2 to C20 heteroaryl group.

In yet another exemplary embodiment, Ar1 and Ar2 are the same as or different from each other, and may be each independently a substituted or unsubstituted phenyl group; a substituted or unsubstituted biphenyl group; a substituted or unsubstituted naphthyl group; a substituted or unsubstituted phenanthrenyl group; a substituted or unsubstituted terphenyl group; a substituted or unsubstituted dibenzofuran group; a substituted or unsubstituted dibenzothiophene group; or a substituted or unsubstituted dimethylfluorenyl group.

In yet another exemplary embodiment, Ar1 and Ar2 are the same as or different from each other, and may be each independently a phenyl group; a biphenyl group; a naphthyl group; a phenanthrenyl group; a terphenyl group; a dibenzofuran group; a dibenzothiophene group; or dimethylfluorenyl group.

In an exemplary embodiment of the present application, Ar1 and Ar2 may be further substituted with deuterium.

In an exemplary embodiment of the present application, B may be a substituted or unsubstituted C6 to C60 aliphatic or aromatic hydrocarbon ring; or a substituted or unsubstituted C2 to C60 aliphatic or aromatic hetero ring,

In another exemplary embodiment, B may be a substituted or unsubstituted C6 to C40 aliphatic or aromatic hydrocarbon ring; or a substituted or unsubstituted C2 to C40 aliphatic or aromatic hetero ring.

In another exemplary embodiment, B may be a C6 to C40 aliphatic or aromatic hydrocarbon ring.

In still another exemplary embodiment, B may be a C6 to C40 aliphatic or aromatic hydrocarbon ring.

In yet another exemplary embodiment, B may be a C6 to C10 aliphatic or aromatic hydrocarbon ring.

In yet another exemplary embodiment, B may be a C6 to C10 aromatic hydrocarbon ring.

In yet another exemplary embodiment, B may be a substituted or unsubstituted benzene ring.

In yet another exemplary embodiment, B may be a benzene ring.

In an exemplary embodiment of the present application, B may be further substituted with deuterium.

In an exemplary embodiment of the present application, R11 to R15 are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R"; and a substituted or unsubstituted amine group, or two or more adjacent groups may be bonded to each other to form a substituted or unsubstituted C6 to C60 aliphatic or aromatic hydrocarbon ring, or a substituted or unsubstituted C2 to C60 aliphatic or aromatic hetero ring.

In another exemplary embodiment, R11 to R15 are the same as or different from each other, and are each independently selected from the group consisting of hydrogen; deuterium; a substituted or unsubstituted C1 to C60 alkyl group; a substituted or unsubstituted C6 to C60 aryl group; a substituted or unsubstituted C2 to C60 heteroaryl group; -P(=O)RR'; -SiRR'R"; and a substituted or unsubstituted amine group, or two or more adjacent groups may be bonded to each other to form a substituted or unsubstituted C6 to C60 aliphatic or aromatic hydrocarbon ring or a substituted or unsubstituted C2 to C60 aliphatic or aromatic hetero ring.

In still another exemplary embodiment, R11 to R15 are the same as or different from each other, and are each independently selected from the group consisting of hydrogen; deuterium; and a substituted or unsubstituted C6 to C60 alkyl group, and two or more adjacent groups may be bonded to each other to form a substituted or unsubstituted C6 to C60 aromatic hydrocarbon ring.

In yet another exemplary embodiment, R11 to R15 are the same as or different from each other, and are each independently selected from the group consisting of hydrogen; deuterium; and a C6 to C60 aryl group, and two or more adjacent groups may be bonded to each other to form a C6 to C60 aromatic hydrocarbon ring.

In an exemplary embodiment of the present application, Chemical Formula 1-2 may be represented by any one of the following Chemical Formulae 1-2-1 to 1-2-8.

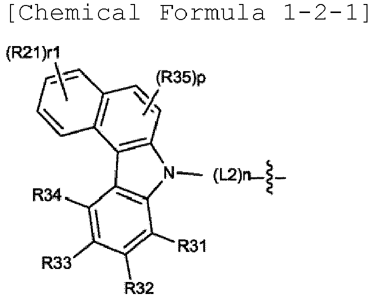

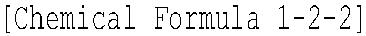

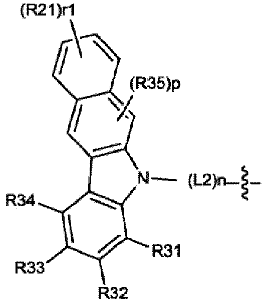

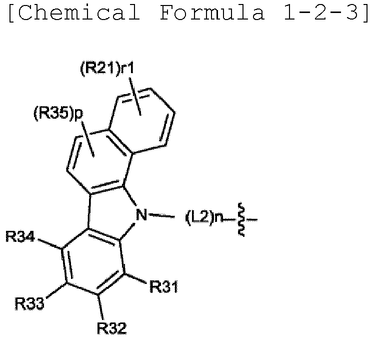

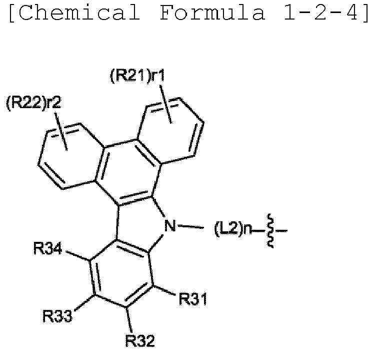

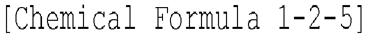

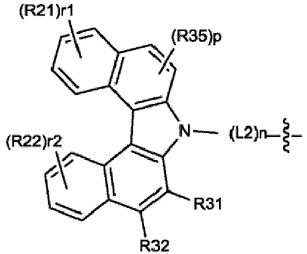

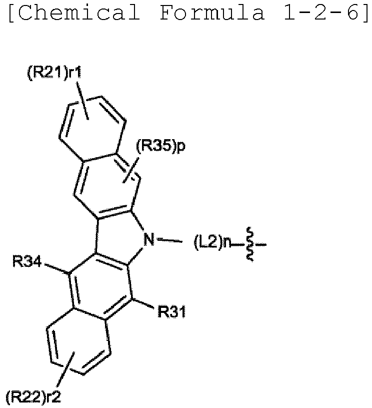

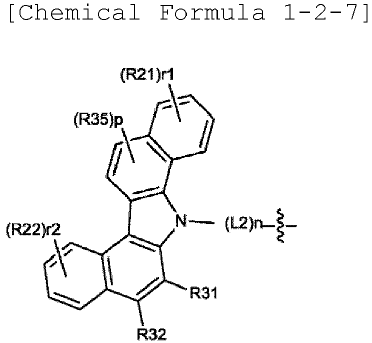

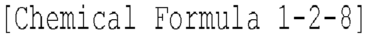

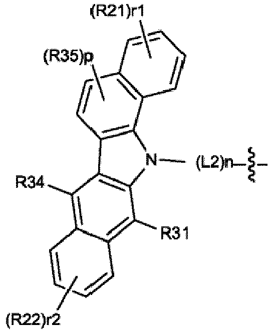

In Chemical Formulae 1-2-1 to 1-2-8,
the definitions of L2, n and p are the same as the definitions in Chemical Formula 1-2,
R21, R22 and R31 to R35 are the same as or different from each other, and are each independently selected from the group consisting of hydrogen; deuterium; a substituted or unsubstituted C1 to C60 alkyl group; a substituted or unsubstituted C6 to C60 aryl group; a substituted or unsubstituted C2 to C60 heteroaryl group; and a substituted or unsubstituted amine group, and
r1 and r2 are an integer from 0 to 4, and when r1 and r2 are 2 or higher, substituents in the parenthesis are the same as or different from each other.

In an exemplary embodiment of the present application, R21, R22 and R31 to R35 are the same as or different from each other, and are each independently selected from the group consisting of hydrogen; deuterium; a substituted or unsubstituted C1 to C60 alkyl group; a substituted or unsubstituted C6 to C60 aryl group; a substituted or unsubstituted C2 to C60 heteroaryl group; and a substituted or unsubstituted amine group.

In another exemplary embodiment, R21, R22 and R31 to R35 are the same as or different from each other, and may be each independently hydrogen; deuterium; or a substituted or unsubstituted C6 to C60 aryl group.

In still another exemplary embodiment, R21, R22 and R31 to R35 are the same as or different from each other, and may be each independently hydrogen; deuterium; or a substituted or unsubstituted C6 to C40 aryl group.

In yet another exemplary embodiment, R21, R22 and R31 to R35 are the same as or different from each other, and may be each independently hydrogen; deuterium; or a substituted or unsubstituted C6 to C20 aryl group.

In yet another exemplary embodiment, R21, R22 and R31 to R35 are the same as or different from each other, and may be each independently hydrogen; deuterium; or a C6 to C20 aryl group.

In yet another exemplary embodiment, R21, R22 and R31 to R35 are the same as or different from each other, and may be each independently hydrogen; deuterium; or a phenyl group.

In an exemplary embodiment of the present application, R, R', and R" are the same as or different from each other, and may be each independently a substituted or unsubstituted C1 to C60 alkyl group; a substituted or unsubstituted C6 to C60 aryl group; or a substituted or unsubstituted C2 to C60 heteroaryl group.

In another exemplary embodiment, R, R', and R" are the same as or different from each other, and may be each independently a substituted or unsubstituted C1 to C60 alkyl group; or a substituted or unsubstituted C6 to C60 aryl group.

In still another exemplary embodiment, R, R', and R" are the same as or different from each other, and may be each independently a C1 to C60 alkyl group; or a C6 to C60 aryl group.

In yet another exemplary embodiment, R, R', and R" are the same as or different from each other, and may be each independently a methyl group; or a phenyl group.

In yet another exemplary embodiment, R, R', and R" may be a phenyl group.

In an exemplary embodiment of the present application, the heterocyclic compound of Chemical Formula 1 may be represented by any one of the following compounds.

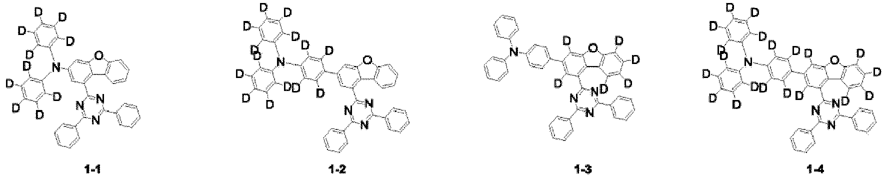

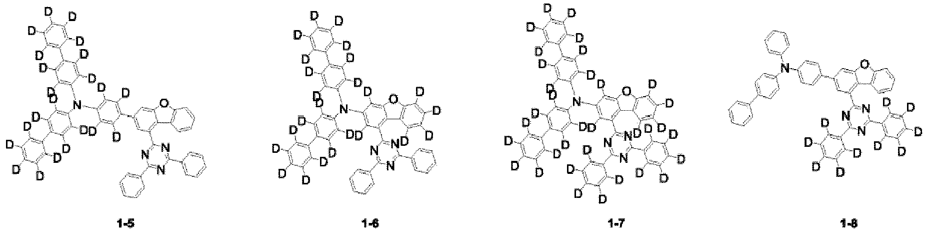

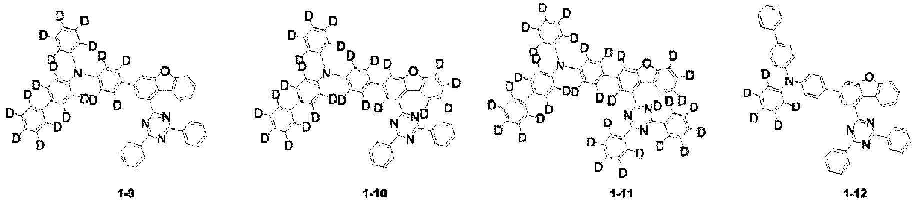

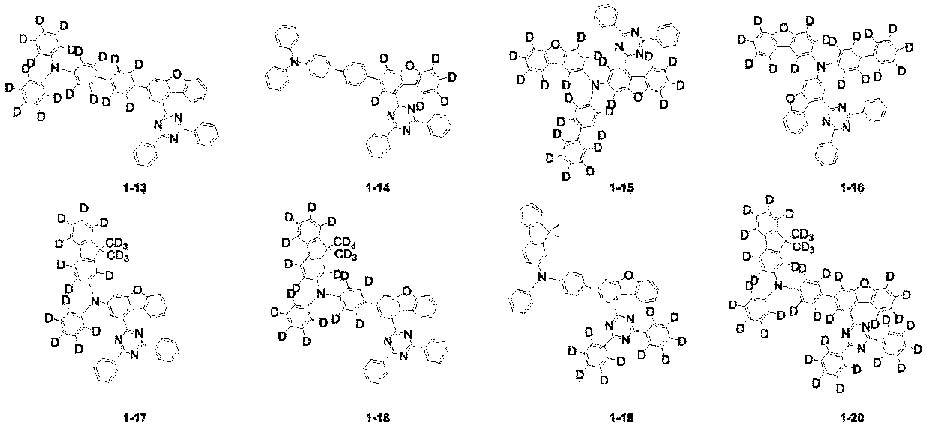

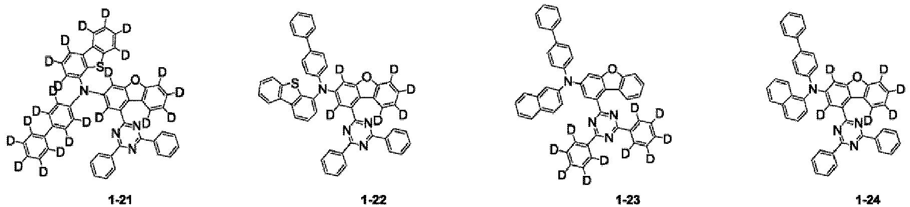

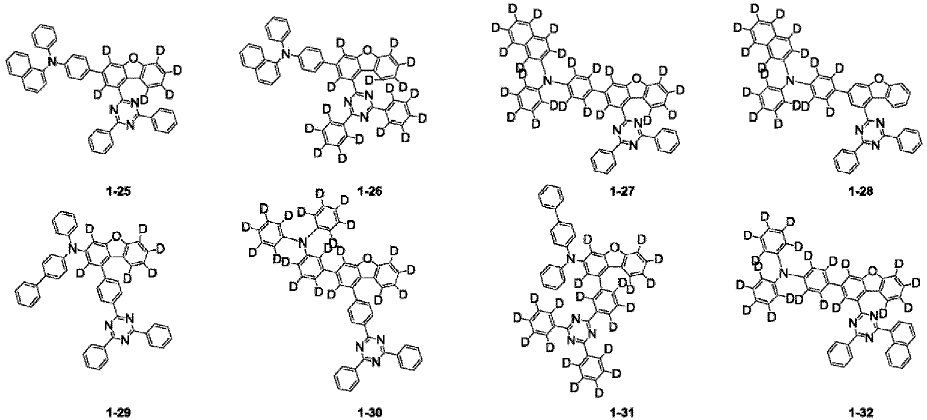

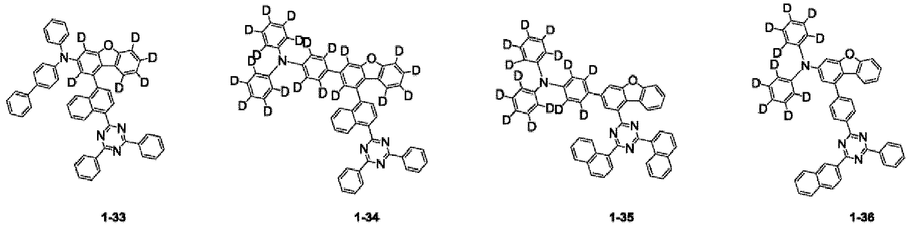

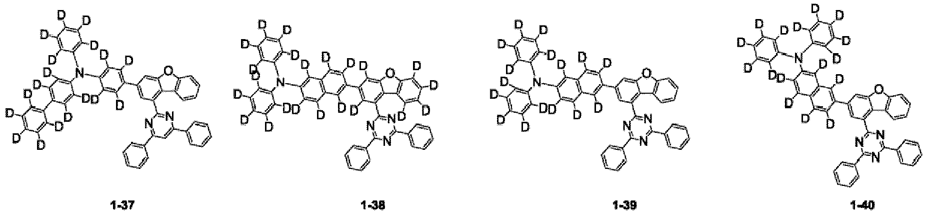

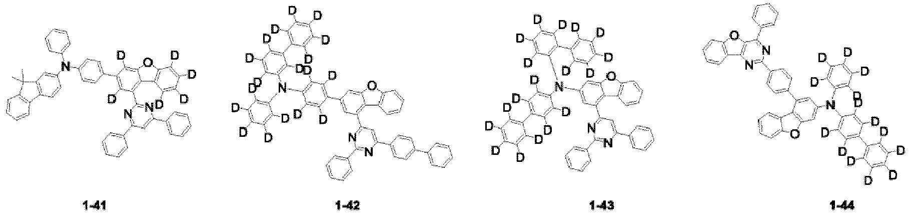

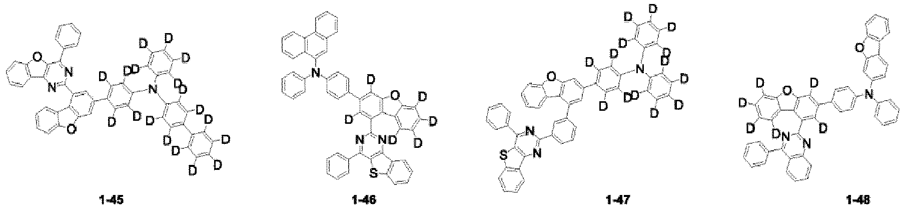

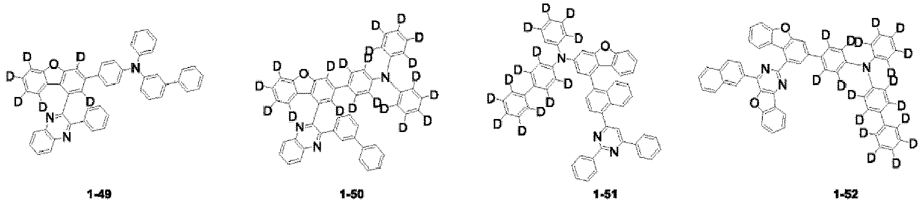

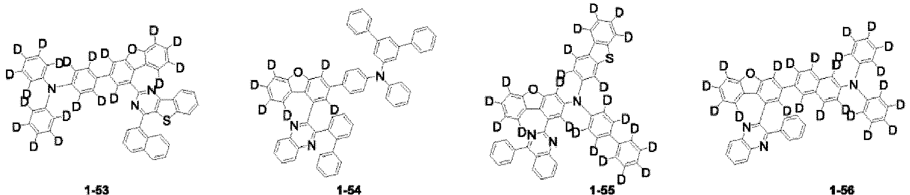

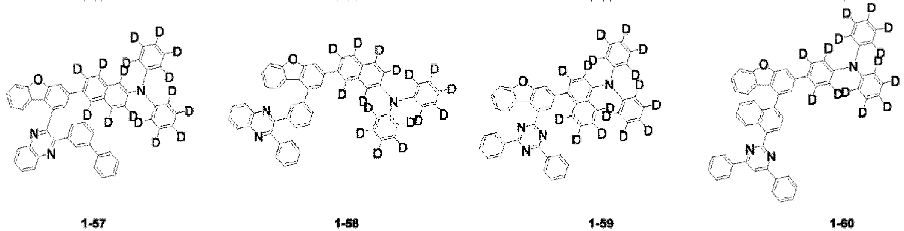

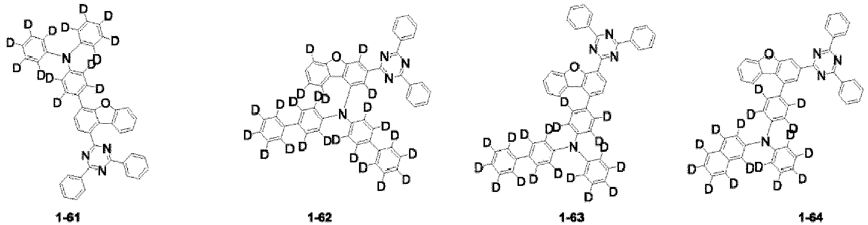

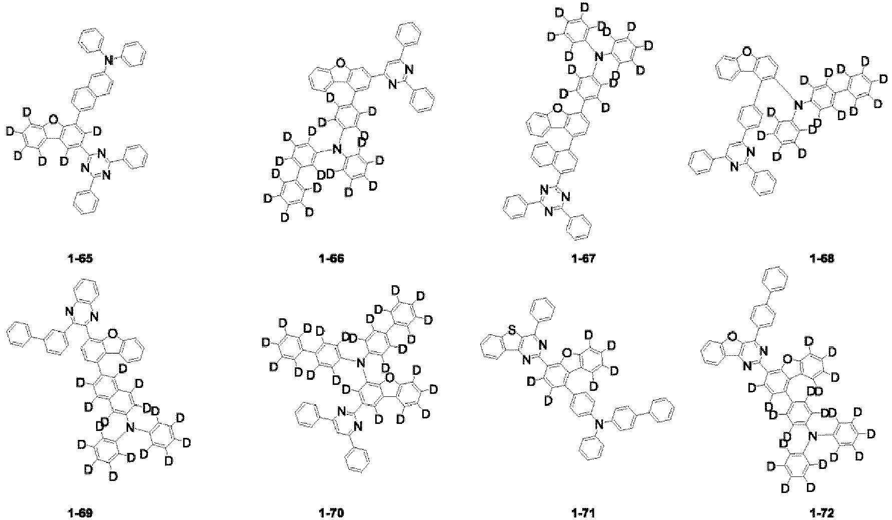

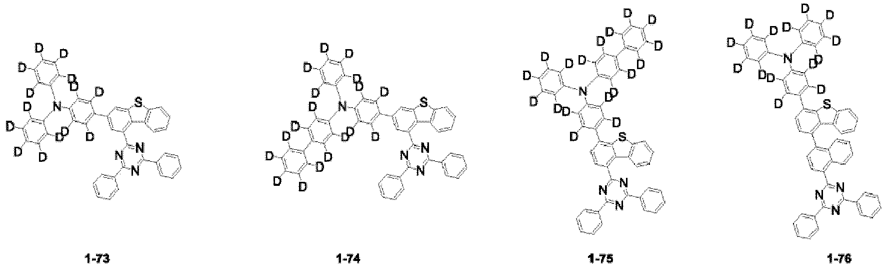

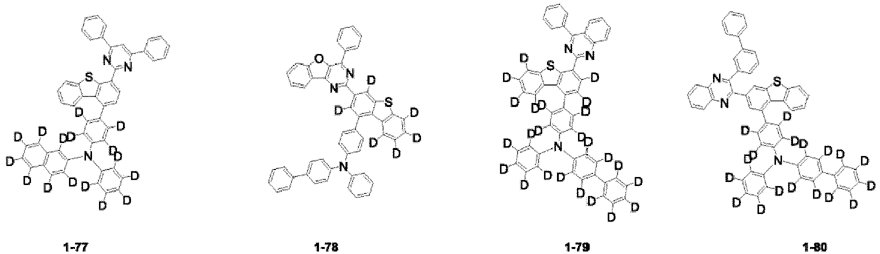

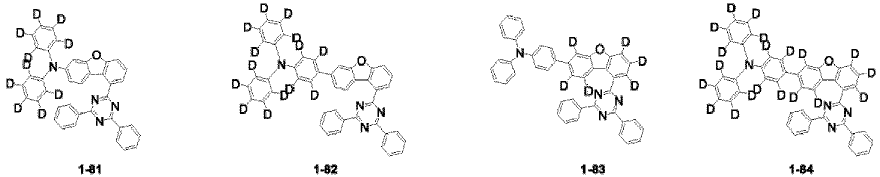

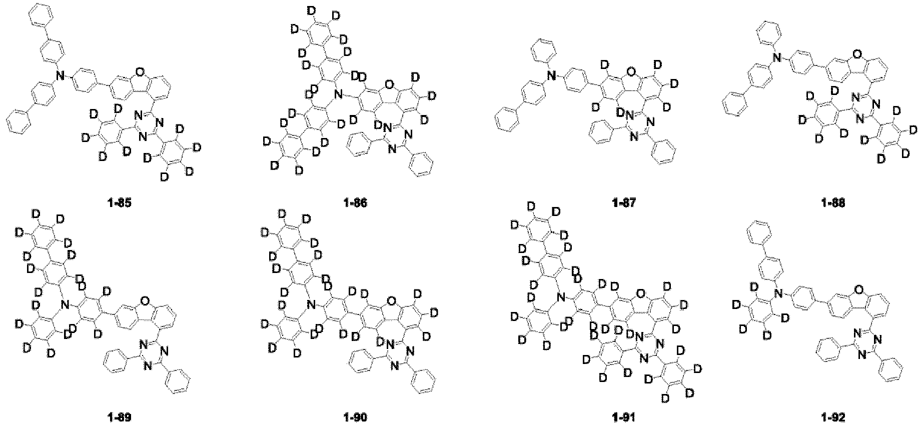

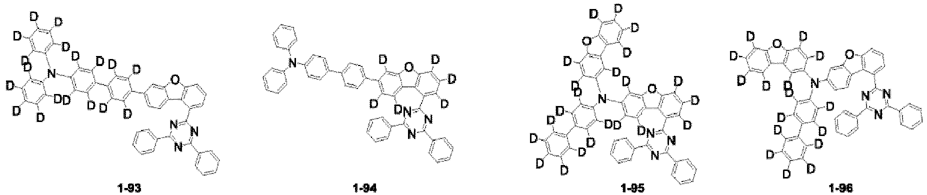

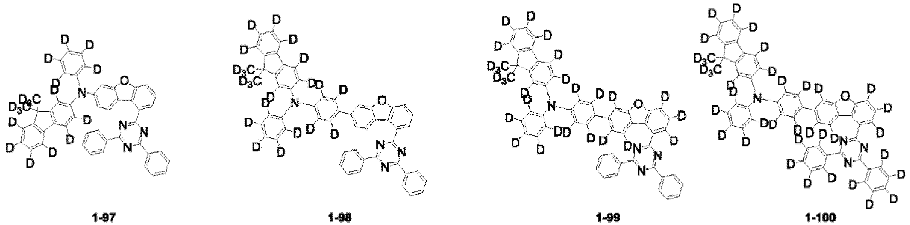

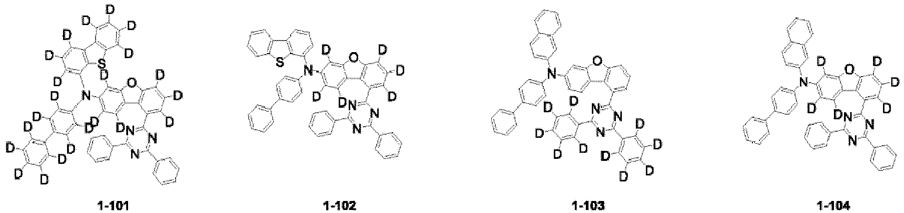

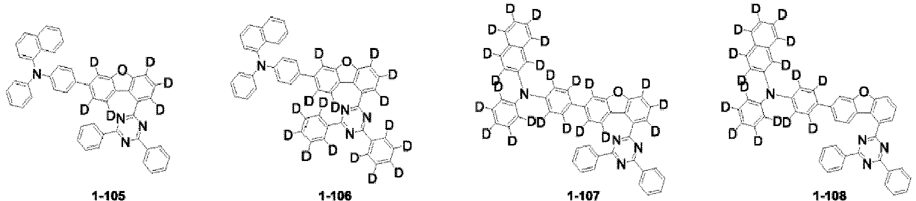

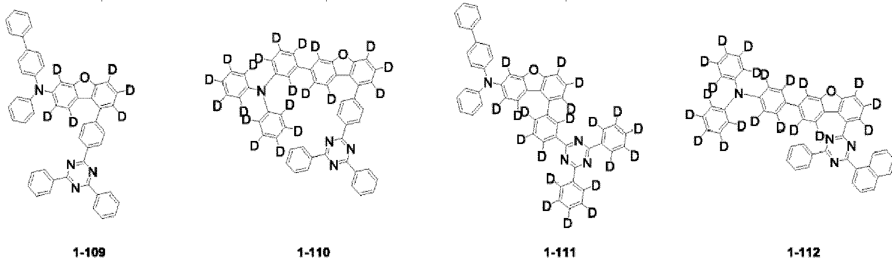

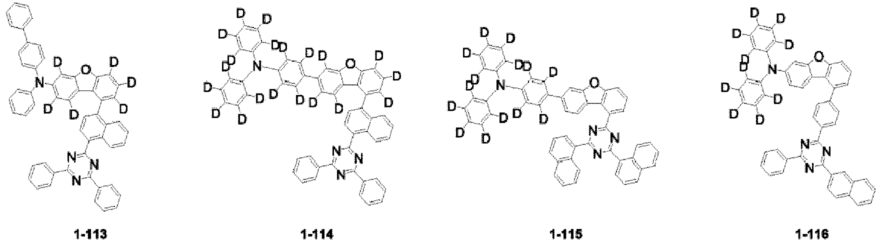

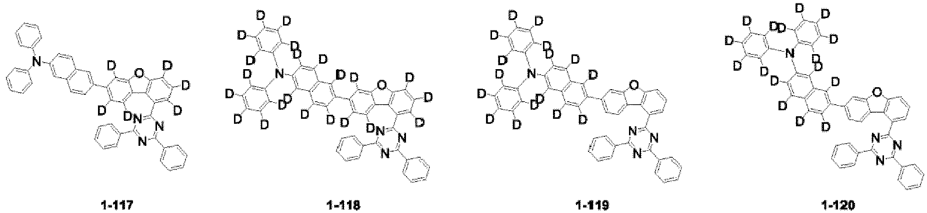

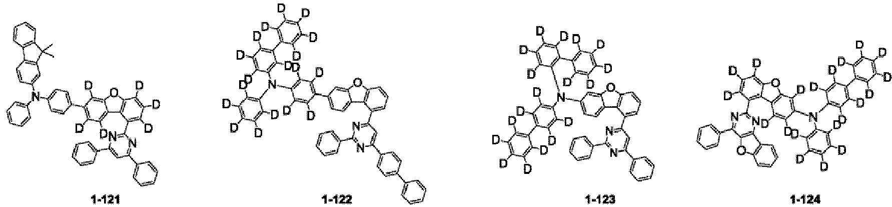

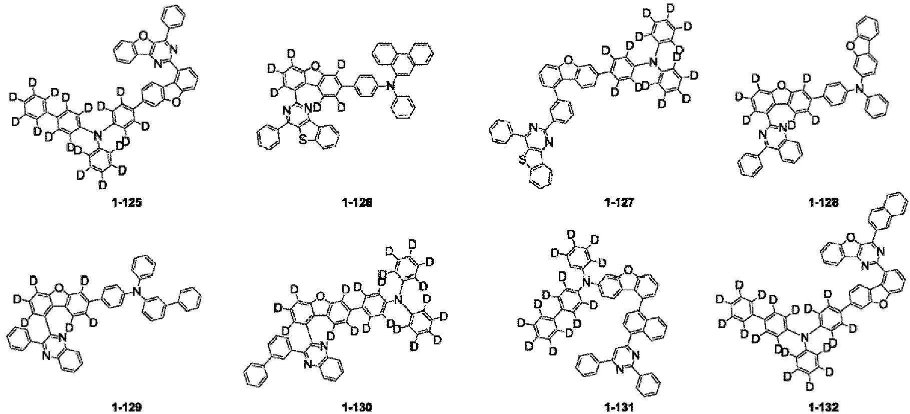

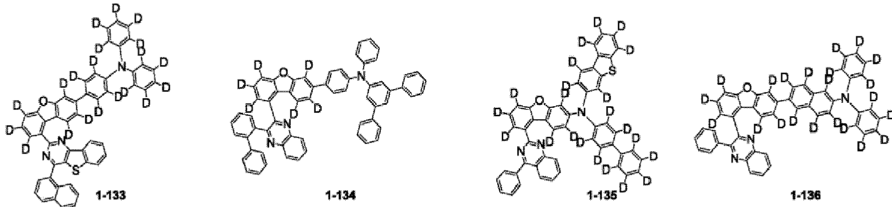

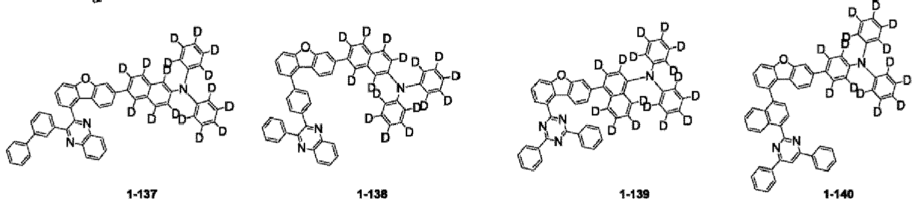

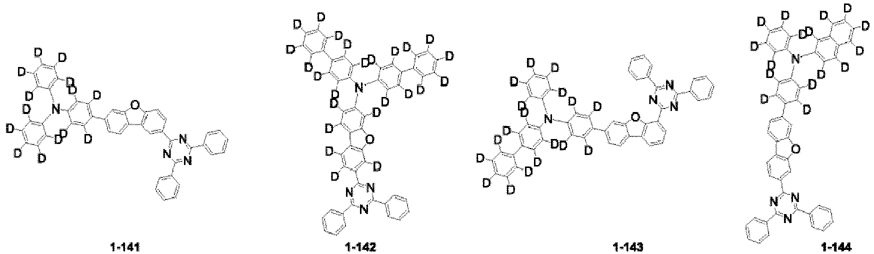

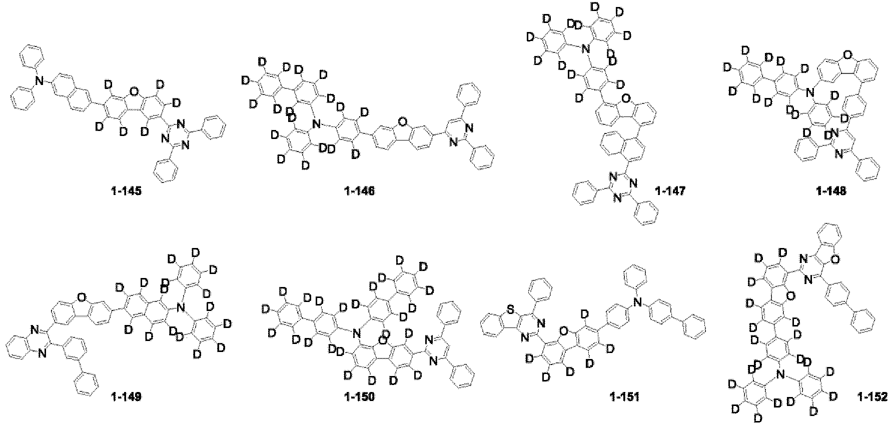

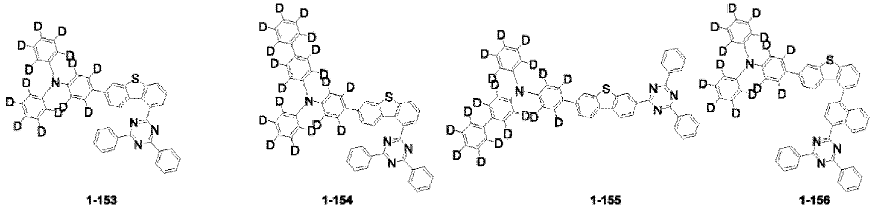

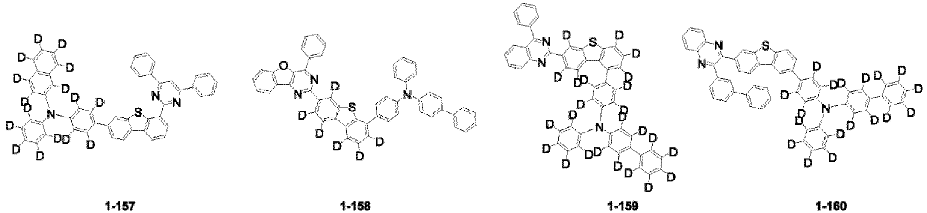

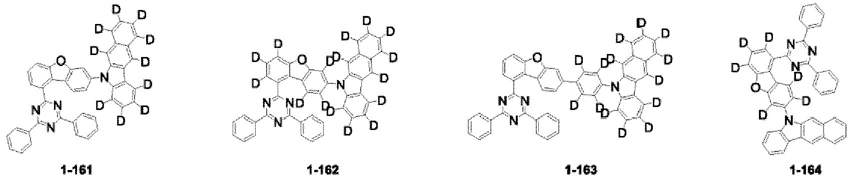

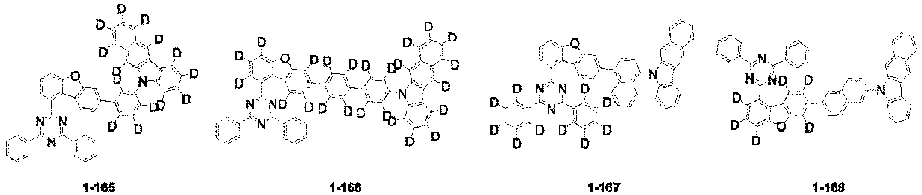

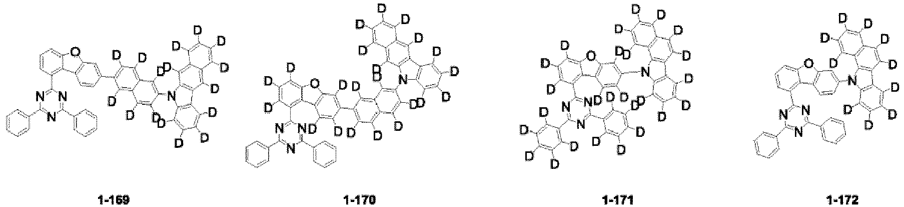

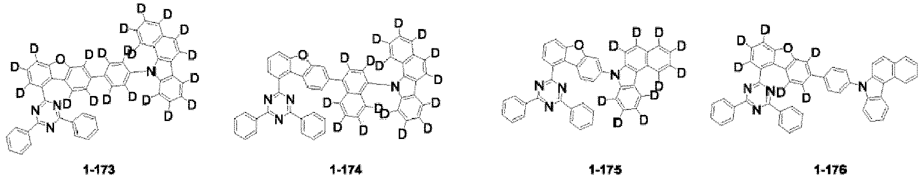

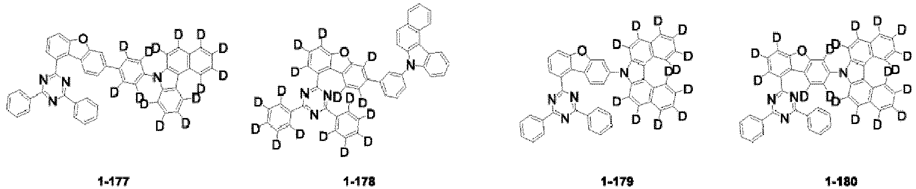

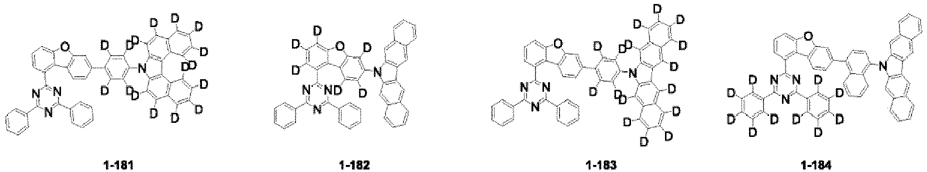

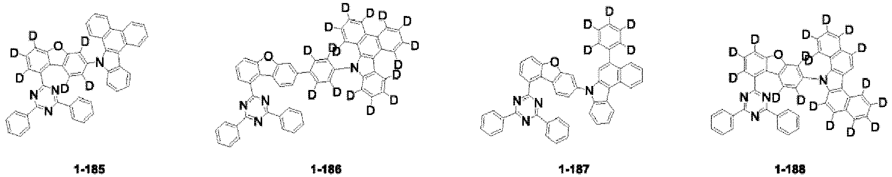

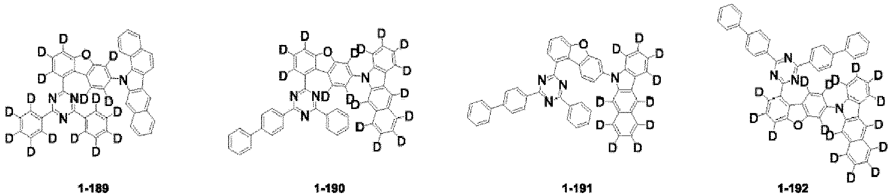

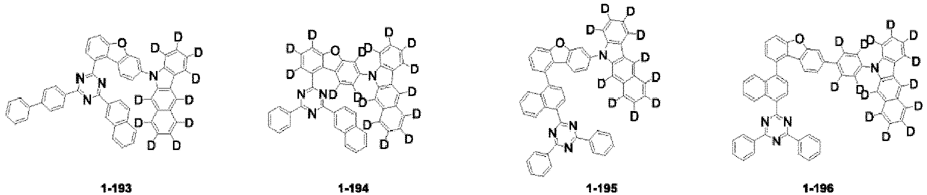

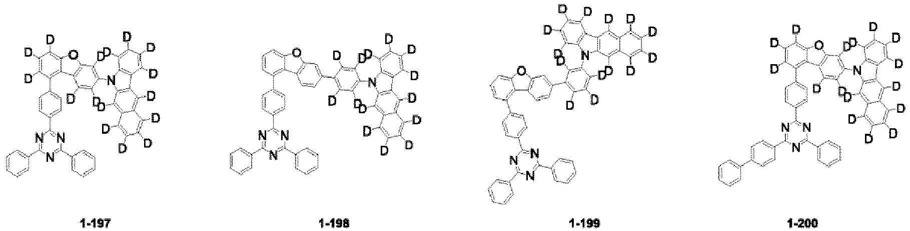

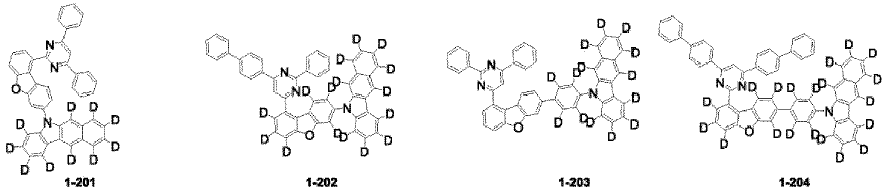

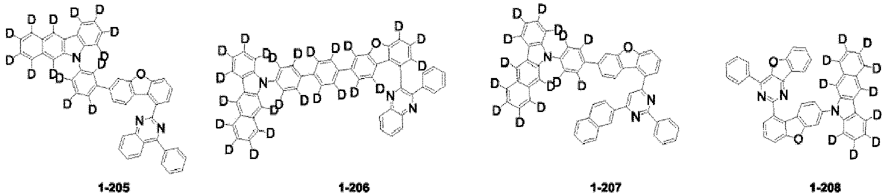

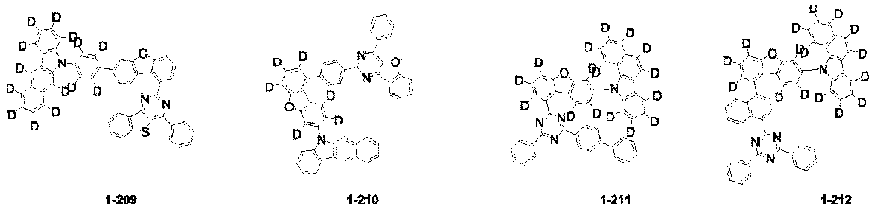

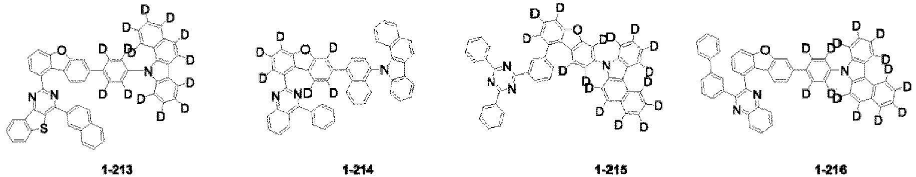

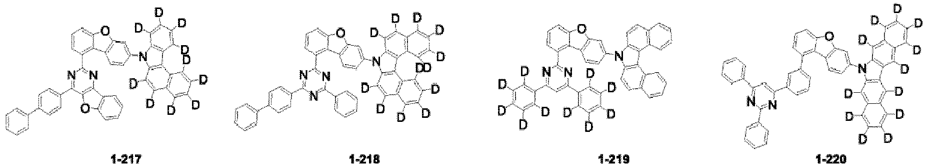

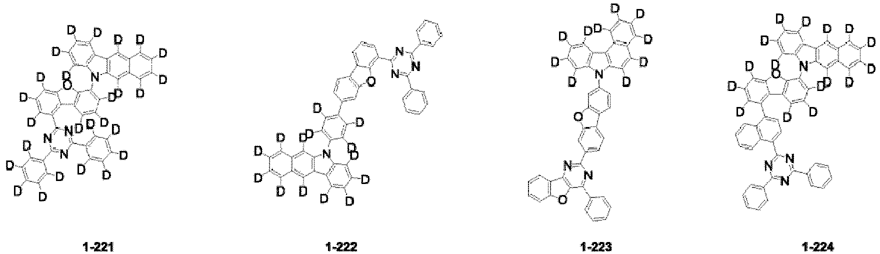

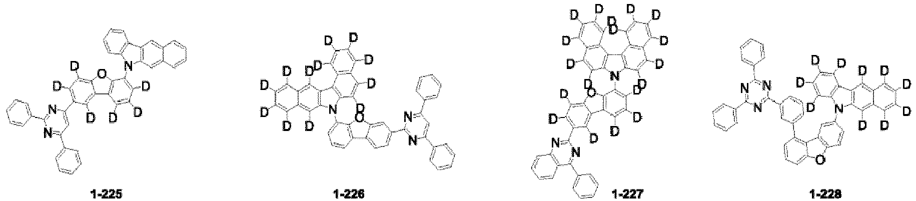

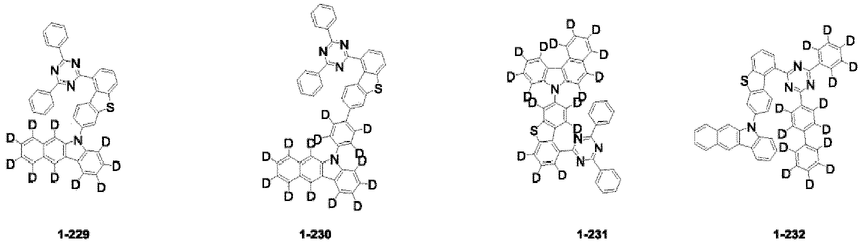

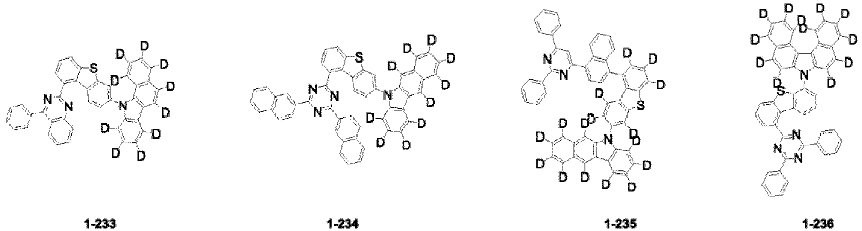

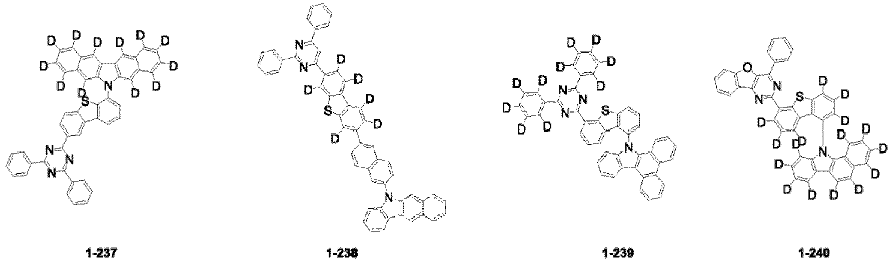

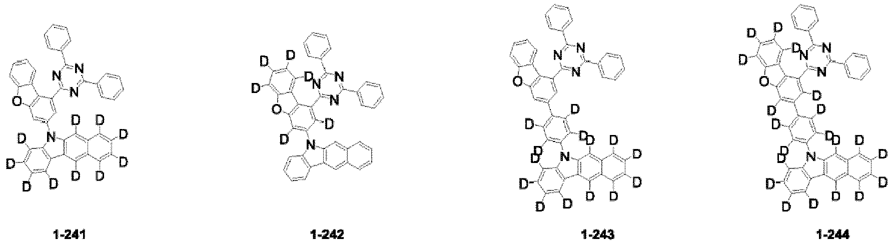

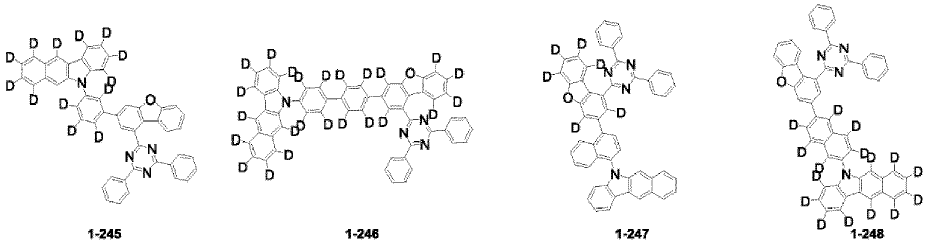

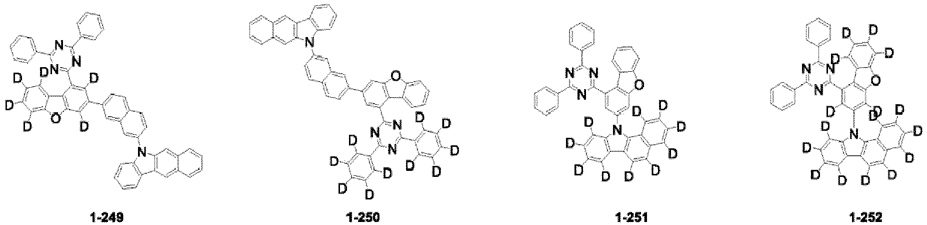

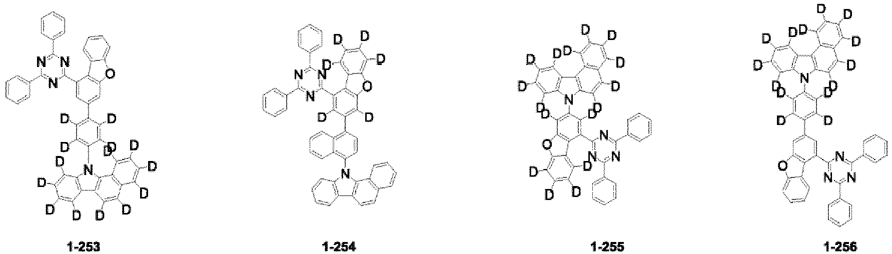

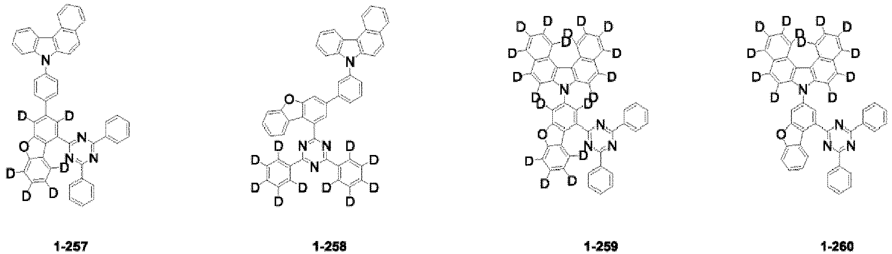

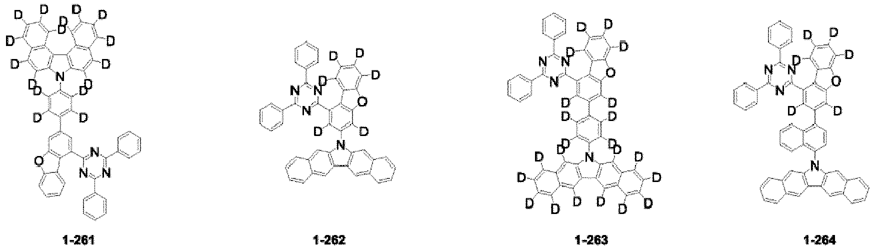

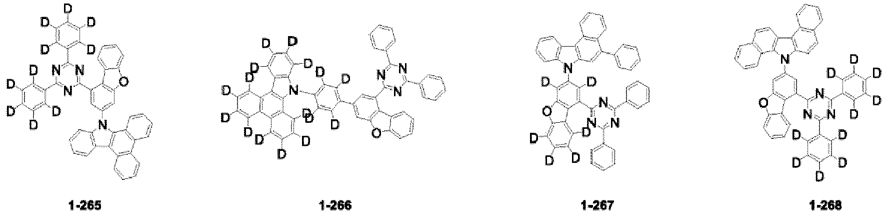

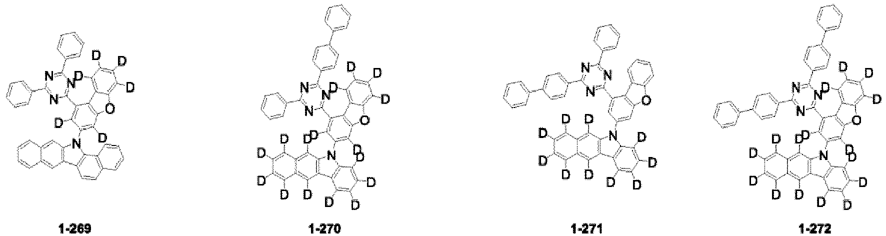

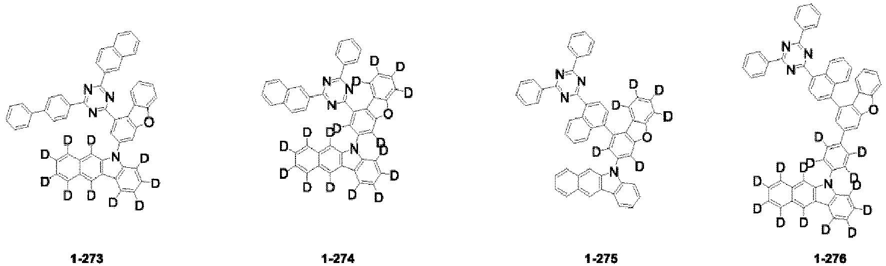

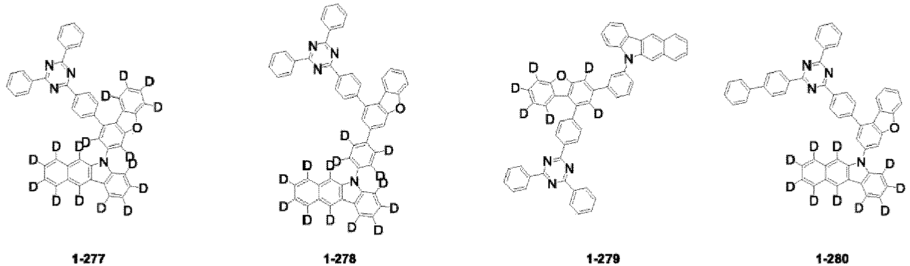

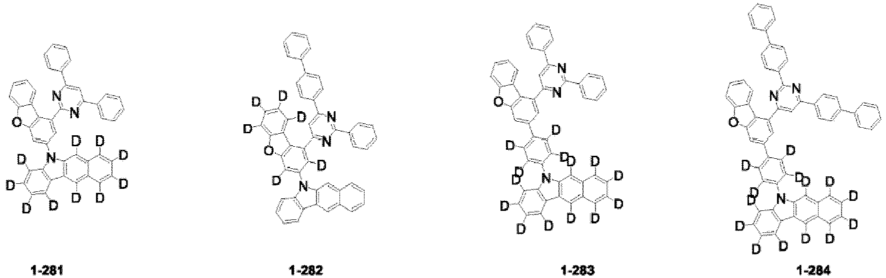

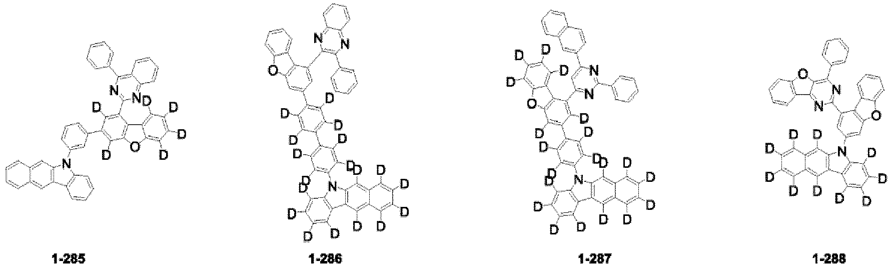

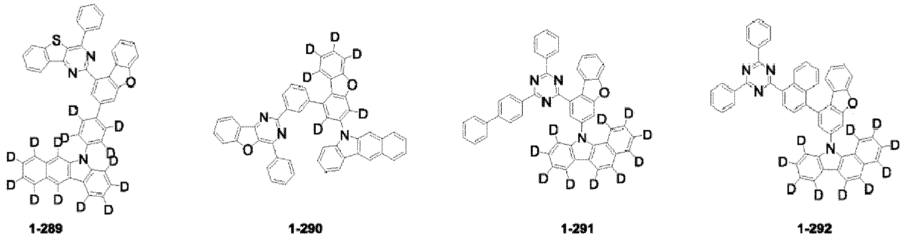

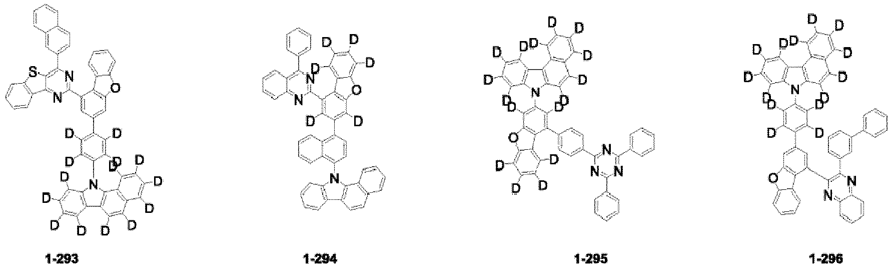

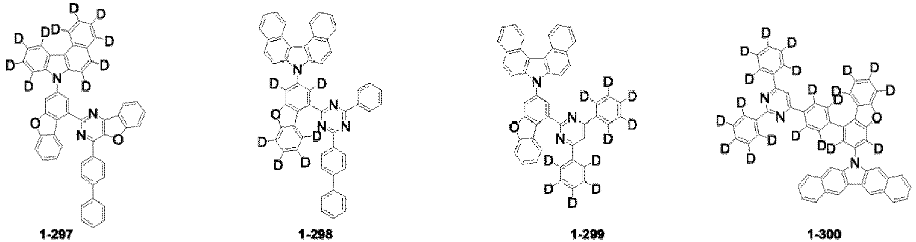

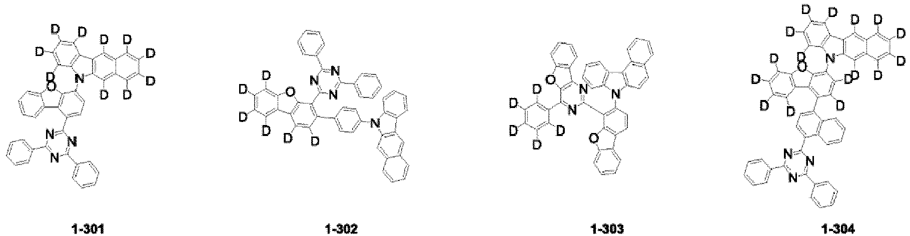

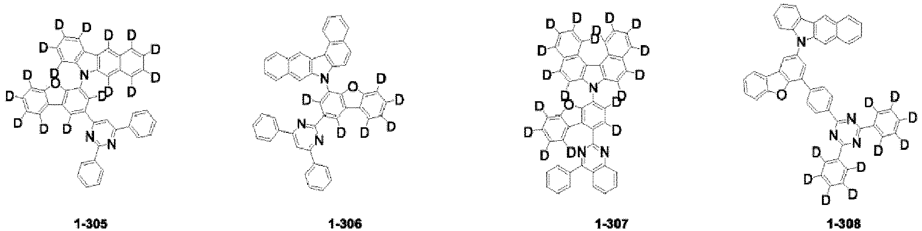

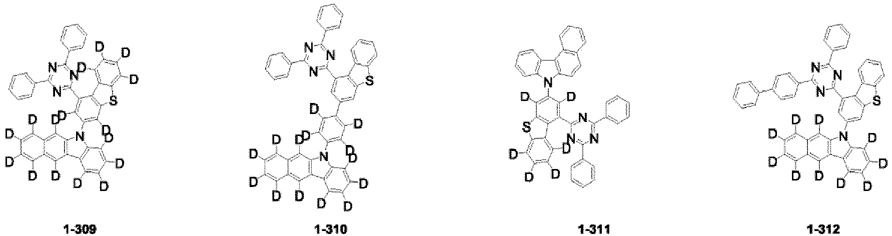

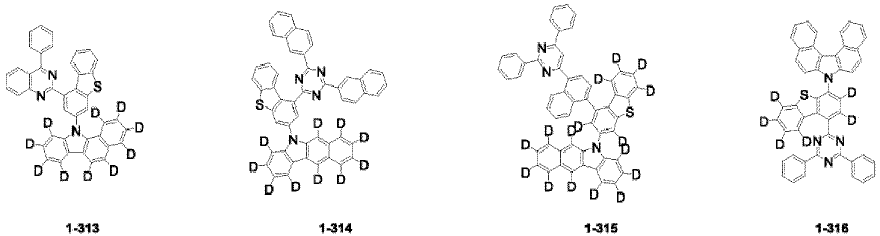

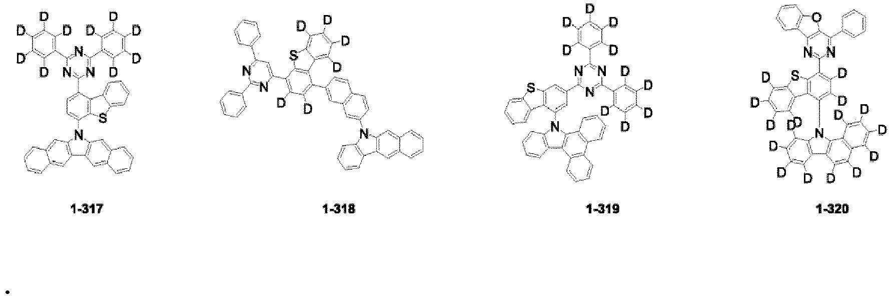

Further, various substituents may be introduced into the structure of Chemical Formula 1 to synthesize a compound having inherent characteristics of a substituent introduced. For example, it is possible to synthesize a material which satisfies conditions required for each organic material layer by introducing a substituent usually used for a hole injection layer material, a material for transporting holes, a light emitting layer material, an electron transport layer material, and a charge generation layer material, which are used for preparing an organic light emitting device, into the core structure.

In addition, it is possible to finely adjust an energy band-gap by introducing various substituents into the structure of Chemical Formula 1, and meanwhile, it is possible to improve characteristics at the interface between organic materials and diversify the use of the material.

Furthermore, in an exemplary embodiment of the present application, provided is an organic light emitting device including a first electrode; a second electrode provided to face the first electrode; and an organic material layer having one or more layers provided between the first electrode and the second electrode, in which one or more layers of the organic material layer include the heterocyclic compound according to Chemical Formula 1.

In another exemplary embodiment, provided is an organic light emitting device including: a first electrode; a second electrode provided to face the first electrode; and an organic material layer having one or more layers provided between the first electrode and the second electrode, in which one or more layers of the organic material layer include one heterocyclic compound according to Chemical Formula 1.

In still another exemplary embodiment, provided is an organic light emitting device including: a first electrode; a second electrode provided to face the first electrode; and an organic material layer having one or more layers provided between the first electrode and the second electrode, in which one or more layers of the organic material layer include two heterocyclic compounds according to Chemical Formula 1.

In the organic light emitting device, when two or more heterocyclic compounds are included, the types of heterocyclic compounds may be the same as or different from each other.

The specific content on the heterocyclic compound represented by Chemical Formula 1 is the same as that described above.

In an exemplary embodiment of the present application, the first electrode may be a positive electrode, and the second electrode may be a negative electrode.

In another exemplary embodiment, the first electrode may be a negative electrode, and the second electrode may be a positive electrode.

In an exemplary embodiment of the present application, the organic light emitting device may be a blue organic light emitting device, and the heterocyclic compound according to Chemical Formula 1 may be used as a material for the blue organic light emitting device. For example, the heterocyclic compound according to Chemical Formula 1 may be included in a host material of a blue light emitting layer of a blue organic light emitting device.

In an exemplary embodiment of the present application, the organic light emitting device may be a green organic light emitting device, and the heterocyclic compound according to Chemical Formula 1 may be used as a material for the green organic light emitting device. For example, the heterocyclic compound according to Chemical Formula 1 may be included in a host material of a green light emitting layer of a green organic light emitting device.

In an exemplary embodiment of the present application, the organic light emitting device may be a red organic light emitting device, and the heterocyclic compound according to Chemical Formula 1 may be used as a material for the red organic light emitting device. For example, the heterocyclic compound according to Chemical Formula 1 may be included in a host material of a red light emitting layer of a red organic light emitting device.

The organic light emitting device of the present invention may be manufactured using typical manufacturing methods and materials of an organic light emitting device, except that the above-described heterocyclic compound is used to form an organic material layer having one or more layers.

The heterocyclic compound may be formed as an organic material layer by not only a vacuum deposition method, but also a solution application method when an organic light emitting device is manufactured. Here, the solution application method means spin coating, dip coating, inkjet printing, screen printing, a spray method, roll coating, and the like, but is not limited thereto.

The organic material layer of the organic light emitting device of the present invention may be composed of a single-layered structure, but may be composed of a multilayered structure in which two or more organic material layers are stacked. For example, the organic light emitting device of the present invention may have a structure including a hole injection layer, a hole transport layer, a light emitting layer, an electron transport layer, an electron injection layer, and the like as organic material layers. However, the structure of the organic light emitting device is not limited thereto, and may include a fewer number of organic material layers.

In the organic light emitting device of the present invention, the organic material layer may include a light emitting layer, and the light emitting layer may include the heterocyclic compound.

In another organic light emitting device, the organic material layer includes a light emitting layer, the light emitting layer includes a host material, and the host material may include the heterocyclic compound.

As another example, the organic material layer including the heterocyclic compound includes the heterocyclic compound represented by Chemical Formula 1 as a host, and the heterocyclic compound may be used with an iridium-based dopant.

In the organic light emitting device of the present invention, the organic material layer includes an electron injection layer or an electron transport layer, and the electron injection layer or electron transport layer may include the heterocyclic compound.

In another organic light emitting device, the organic material layer includes an electron blocking layer or a hole blocking layer, and the electron blocking layer or the hole blocking layer may include the heterocyclic compound.

The organic light emitting device of the present invention may further include one or two or more layers selected from the group consisting of a light emitting layer, a hole injection layer, a hole transport layer, an electron injection layer, an electron transport layer, an electron blocking layer, and a hole blocking layer.

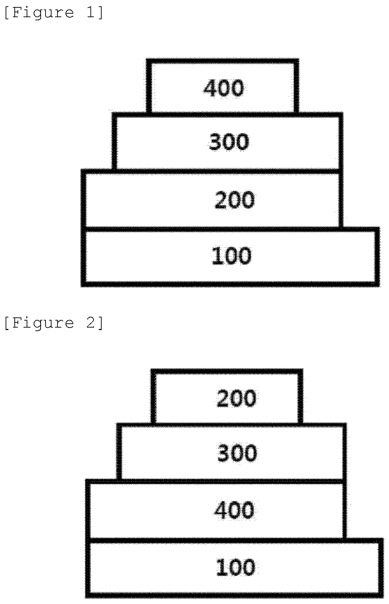

FIGS. 1 to 3 exemplify the stacking sequence of the electrodes and the organic material layer of the organic light emitting device according to an exemplary embodiment of the present application. However, the scope of the present application is not intended to be limited by these drawings, and the structure of the organic light emitting device known in the art may also be applied to the present application.

According to FIG. 1, an organic light emitting device in which a positive electrode 200, an organic material layer 300, and a negative electrode 400 are sequentially stacked on a substrate 100 is illustrated. However, the organic light emitting device is not limited only to such a structure, and as in FIG. 2, an organic light emitting device in which a negative electrode, an organic material layer, and a positive electrode are sequentially stacked on a substrate may also be implemented.

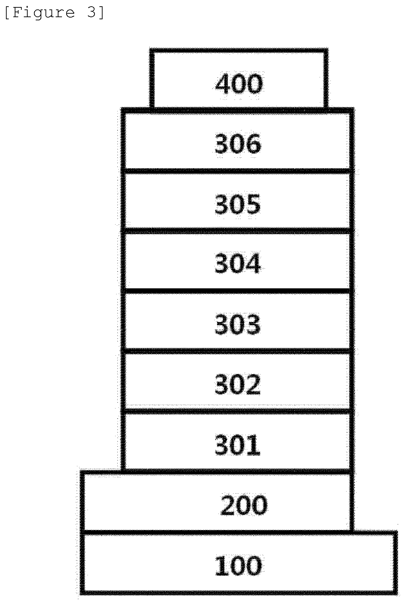

FIG. 3 exemplifies a case where an organic material layer is a multilayer. An organic light emitting device according to FIG. 3 includes a hole injection layer 301, a hole transport layer 302, a light emitting layer 303, a hole blocking layer 304, an electron transport layer 305, and an electron injection layer 306. However, the scope of the present application is not limited by the stacking structure as described above, and if necessary, the other layers except for the light emitting layer may be omitted, and another necessary functional layer may be further added.

An organic material layer including the compound of Chemical Formula 1 may additionally include other materials, if necessary.

Further, an exemplary embodiment of the present application provides an organic light emitting device in which an organic material layer including the heterocyclic compound of Chemical Formula 1 further includes: a compound represented by the following Chemical Formula A; or a compound represented by the following Chemical Formula B.

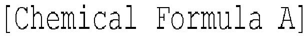

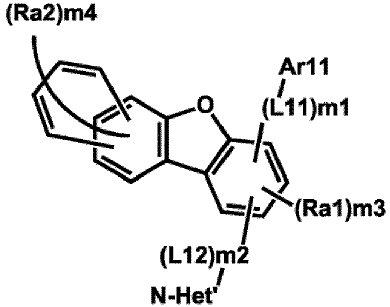

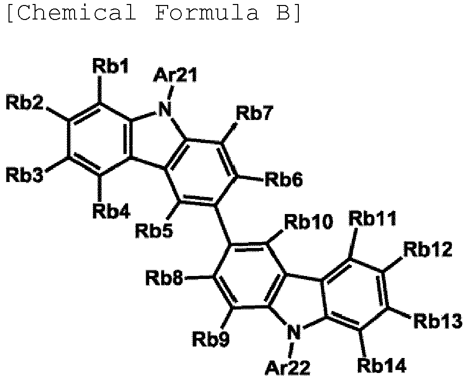

In Chemical Formulae A and B,
Ra1, Ra2 and Rb1 to Rb14 are the same as or different from each other, and are each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group, or two or more adjacent groups are bonded to each other to form a substituted or unsubstituted C6 to C60 aromatic hydrocarbon ring or a substituted or unsubstituted C2 to C60 hetero ring.
L11 and L12 are the same as or different from each other, and are each independently a direct bond; a substituted or unsubstituted C6 to C60 arylene group; or a substituted or unsubstituted C2 to C60 heteroarylene group,
N-Het' is a heteroaryl group which is substituted or unsubstituted and includes at least one N,
Ar11, Ar21 and Ar22 are the same as or different from each other, and each independently a substituted or unsubstituted C6 to C60 aryl group; or a substituted or unsubstituted C2 to C60 heteroaryl group,
m1 and m2 are an integer from 0 to 4,
m3 is an integer from 0 to 2,
m4 is an integer from 0 to 6, and
when m1, m2 and m4 are an integer or 2 or higher, or m3 is an integer of 2, substituents in the parenthesis are the same as or different from each other.

In an exemplary embodiment of the present application, N-Het' may be the same as the definition of N-Het of Chemical Formula 1.

In an exemplary embodiment of the present application, Ar21 and Ar22 of Chemical Formula B are the same as or different from each other, and are each independently a substituted or unsubstituted C6 to C60 aryl group; or a substituted or unsubstituted C2 to C60 heteroaryl group.

In another exemplary embodiment, Ar21 and Ar22 of Chemical Formula B are the same as or different from each other, and are each independently a substituted or unsubstituted C6 to C40 aryl group; or a substituted or unsubstituted C2 to C40 heteroaryl group.

In still another exemplary embodiment, Ar21 and Ar22 of Chemical Formula B are the same as or different from each other, and are each independently a substituted or unsubstituted C6 to C40 aryl group.

In yet another exemplary embodiment, Ar21 and Ar22 of Chemical Formula B are the same as or different from each other, and are each independently a substituted or unsubstituted C6 to C20 aryl group.

In yet another exemplary embodiment, Ar21 and Ar22 of Chemical Formula B are the same as or different from each other, and are each independently -CN; deuterium; or a C6 to C20 aryl group unsubstituted or substituted with a C6 to C20 aryl group.

In yet another exemplary embodiment, Ar21 and Ar22 of Chemical Formula B are the same as or different from each other, and are each independently -CN; deuterium; or a phenyl group unsubstituted or substituted with a naphthyl group; a naphthyl group unsubstituted or substituted with deuterium; or a biphenyl group unsubstituted or substituted with deuterium.

In an exemplary embodiment of the present application, Ra1, Ra2 and Rb1 to Rb14 are the same as or different from each other, and are each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group, or two or more adjacent groups are bonded to each other to form a substituted or unsubstituted C6 to C60 aromatic hydrocarbon ring or a substituted or unsubstituted C2 to C60 hetero ring.

In another exemplary embodiment, Ra1, Ra2 and Rb1 to Rb14 are the same as or different from each other, and each independently selected from the group consisting of hydrogen; deuterium; a substituted or unsubstituted C6 to C60 aryl group; and a substituted or unsubstituted C2 to C60 heteroaryl group, or two or more adjacent groups may be bonded to each other to form a substituted or unsubstituted C6 to C60 aromatic hydrocarbon ring or a substituted or unsubstituted C2 to C60 hetero ring.

In still another exemplary embodiment, Ra1, Ra2 and Rb1 to Rb14 are the same as or different from each other, and are each independently selected from the group consisting of hydrogen; and deuterium, or two or more adjacent groups may be bonded to each other to form a substituted or unsubstituted C6 to C60 aromatic hydrocarbon ring.

In yet another exemplary embodiment, Ra1, Ra2 and Rb1 to Rb14 are the same as or different from each other, and are each independently selected from the group consisting of hydrogen; and deuterium, and two or more adjacent groups may be bonded to each other to form a C6 to C60 aromatic hydrocarbon ring unsubstituted or substituted with deuterium.

In yet another exemplary embodiment, Ra1, Ra2 and Rb1 to Rb14 are the same as or different from each other, and are each independently selected from the group consisting of hydrogen; and deuterium, or two or more adjacent groups may be bonded to each other to form a benzene ring unsubstituted or substituted with deuterium.

In an exemplary embodiment of the present application, L11 and L12 are the same as or different from each other, and may be each independently a direct bond; a substituted or unsubstituted C6 to C60 arylene group; or a substituted or unsubstituted C2 to C60 heteroarylene group.

In another exemplary embodiment, L11 and L2 are the same as or different from each other, and may be each independently a direct bond; a substituted or unsubstituted C6 to C40 arylene group; or a substituted or unsubstituted C2 to C40 heteroarylene group.

In still another exemplary embodiment, L11 and L2 are the same as or different from each other, and may be each independently a direct bond; a C6 to C40 arylene group unsubstituted or substituted with deuterium; or a C2 to C40 heteroarylene group unsubstituted or substituted with deuterium.

In yet another exemplary embodiment, L11 and L12 are the same as or different from each other, and may be each independently a direct bond; or a C6 to C20 arylene group unsubstituted or substituted with deuterium.

In yet another exemplary embodiment, L11 and L12 are the same as or different from each other, and may be each independently a direct bond; a phenylene group unsubstituted or substituted with deuterium; a biphenylene group unsubstituted or substituted with deuterium; or a naphthalene group unsubstituted or substituted with deuterium.

In an exemplary embodiment of the present application, the deuterium content of the compound of Chemical Formula A may be 0% or more and 100% or less.

In another exemplary embodiment, the deuterium content of the compound of Chemical Formula A may be 5% or more, 10% or more, 15% or more, and 20% or more, and may satisfy a range of 100% or less.

In an exemplary embodiment of the present application, the deuterium content of the compound of Chemical Formula A includes all the contents of deuterium included in the specific example compound of Chemical Formula A to be described below, and may be calculated and represented by X% based on the compound of Chemical Formula A to be described below, and the range corresponds to one example of the range according to the present application.

That is, all the range of deuterium may be created based on the specific examples of Chemical Formula A to be described below, but only a part of the examples are described in the present specification, and the content of deuterium may be calculated and expressed in various ways based on the specific examples of Chemical Formula A to be described below, and the content of deuterium may be expressed as X% or more and X'% or less based on some specific examples.

In an exemplary embodiment of the present application, the deuterium content of the compound of Chemical Formula B may be 0% or more and 100% or less.

In another exemplary embodiment, the deuterium content of the compound of Chemical Formula B may be 5% or more, 10% or more, 15% or more, and 20% or more, and may satisfy a range of 100% or less.

In an exemplary embodiment of the present application, the deuterium content of the compound of Chemical Formula B includes all the contents of deuterium included in the specific example compound of Chemical Formula B to be described below, and may be calculated and represented by X% based on the compound of Chemical Formula B to be described below, and the range corresponds to one example of the range according to the present application.

That is, all the range of deuterium may be created based on the specific examples of Chemical Formula B to be described below, but only a part of the examples are described in the present specification, and the content of deuterium may be calculated and expressed in various ways based on the specific examples of Chemical Formula B to be described below, and the content of deuterium may be expressed as X% or more and X'% or less based on some specific examples.

The compound of Chemical Formula A according to an exemplary embodiment of the present application is a material used for n-type hosts, the compound of Chemical Formula B corresponds to a material used for p-type hosts, and the hetero compound represented by Chemical Formula 1 of the present application has a characteristic that the hetero compound can be used in combination with any material of Chemical Formula A or Chemical formula B as a bipolar host material.

In an exemplary embodiment of the present application, the compound of Chemical Formula A may be represented by any one of the following structural formulae.

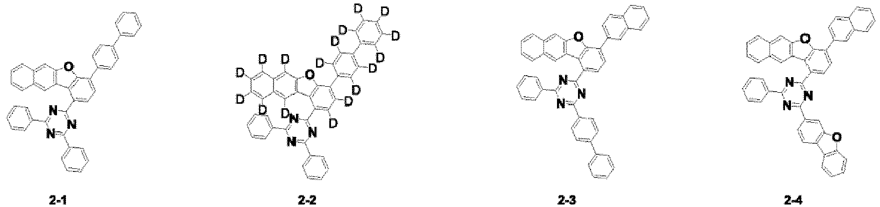

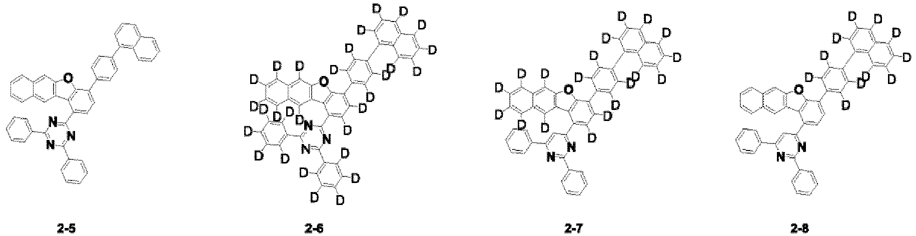

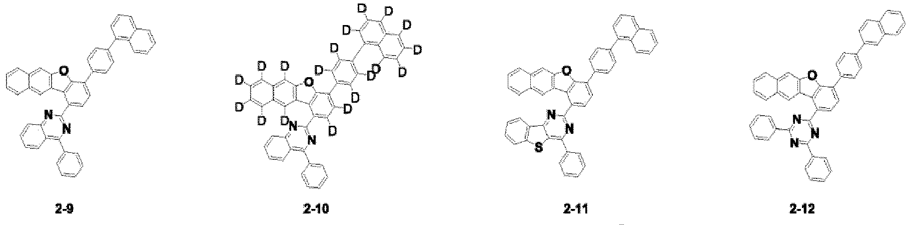

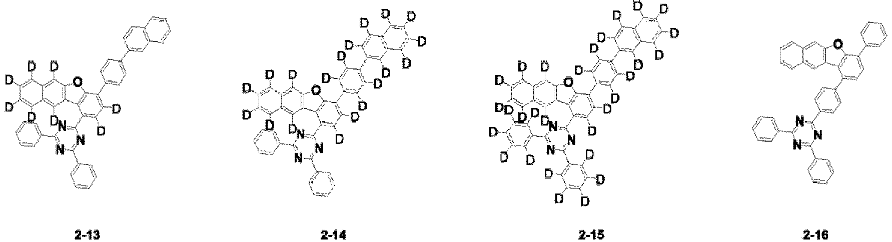

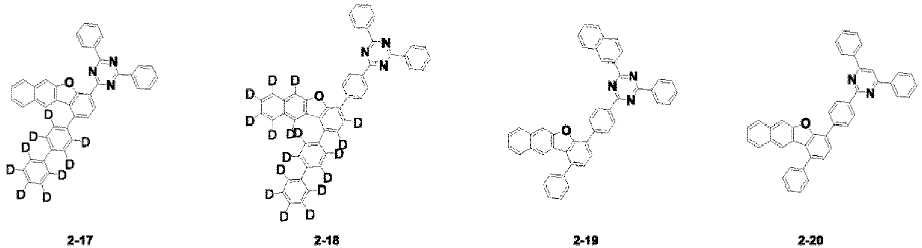

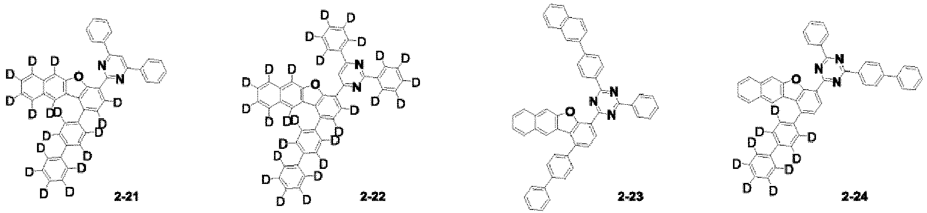

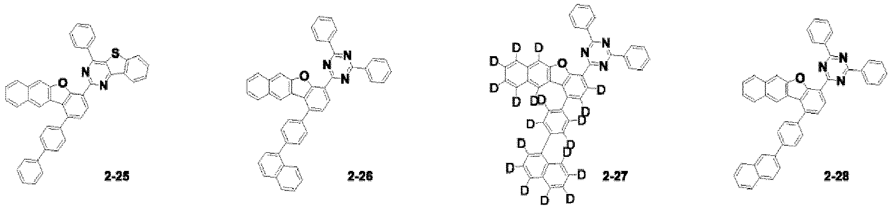

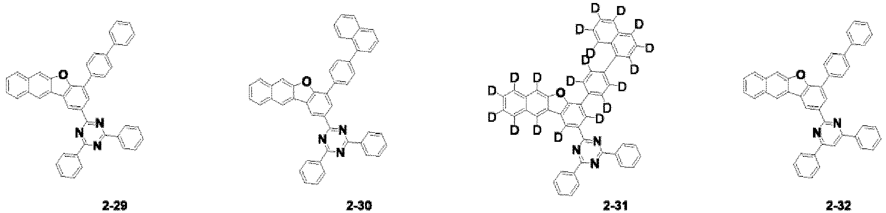

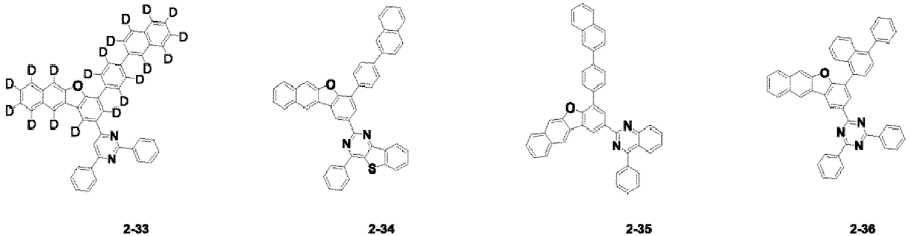

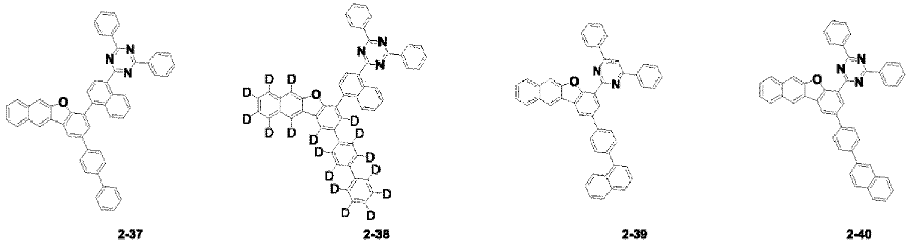

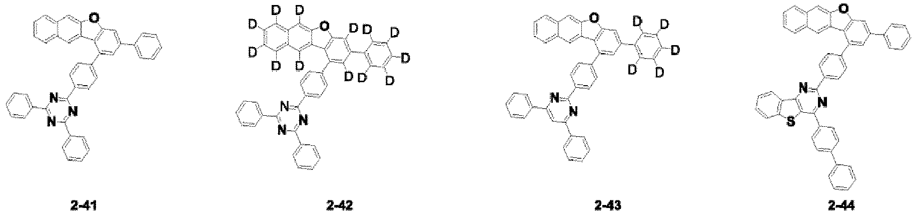

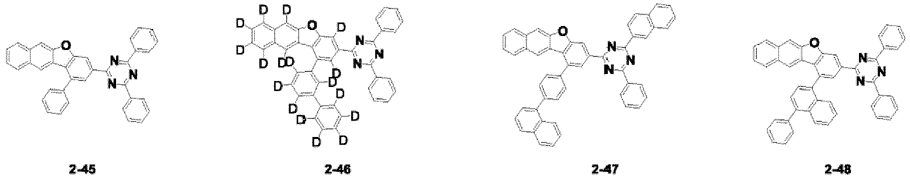

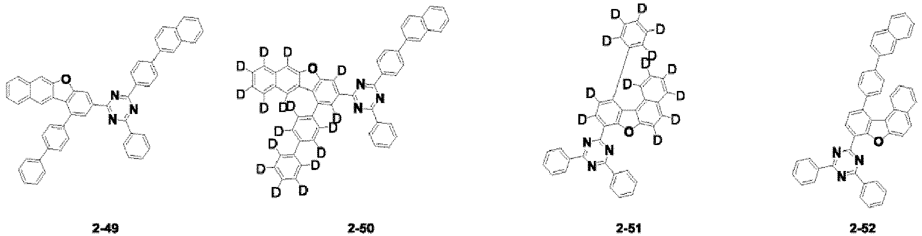

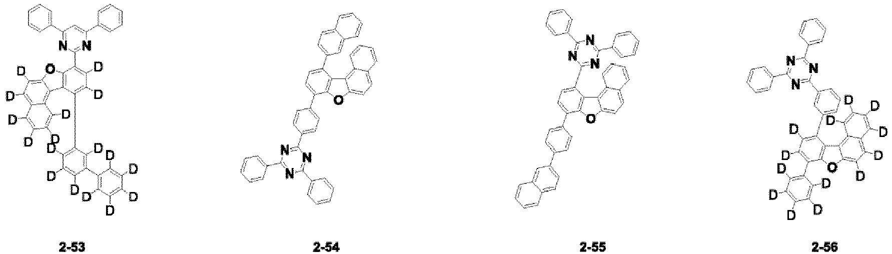

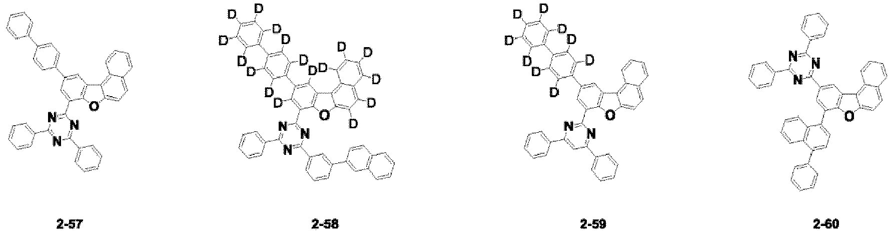

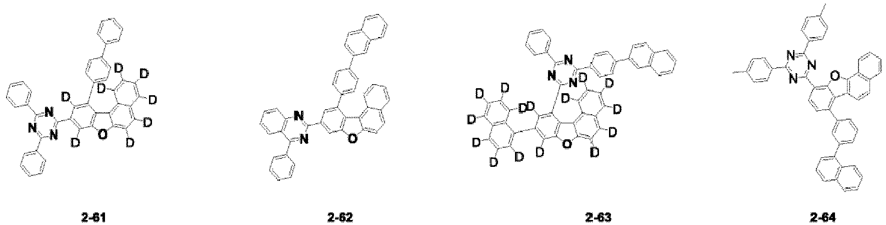

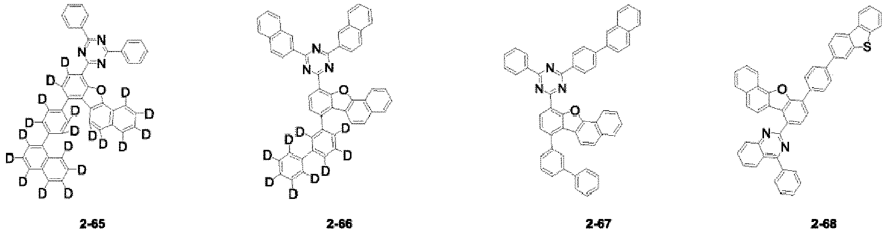

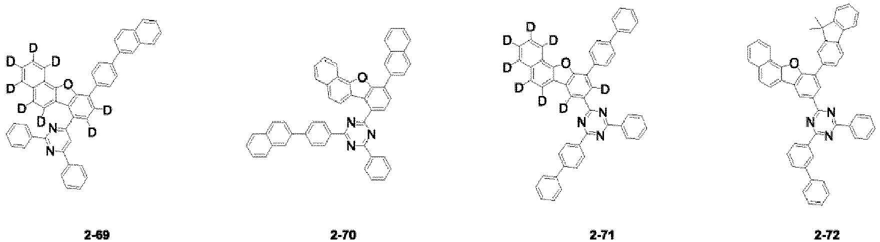

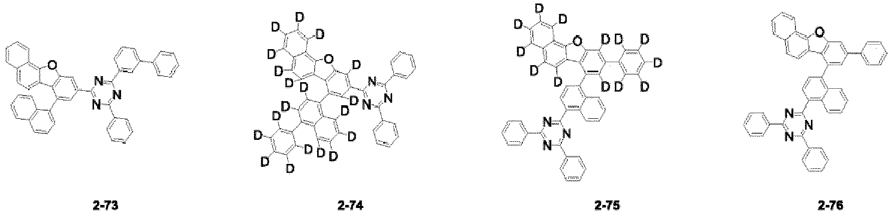

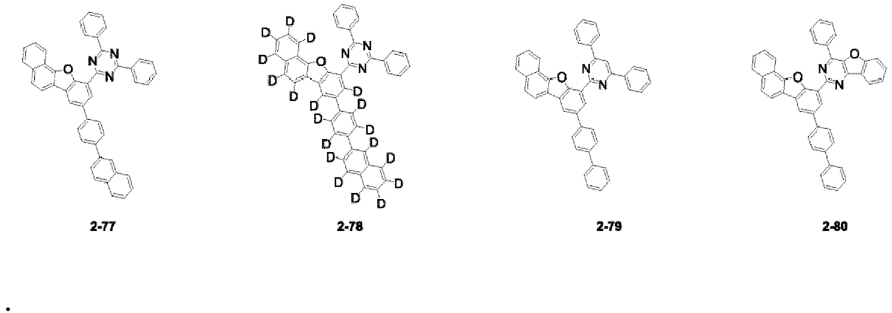

In an exemplary embodiment of the present specification, the compound of Chemical Formula B may be represented by any one of the following structural formulae.

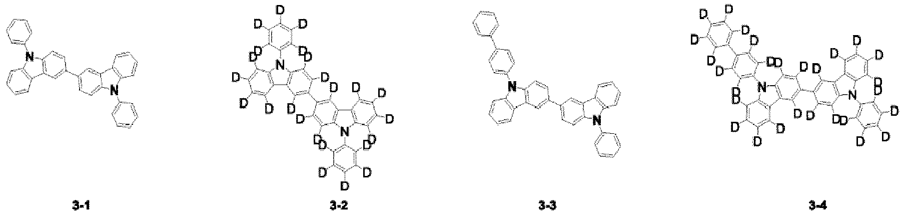

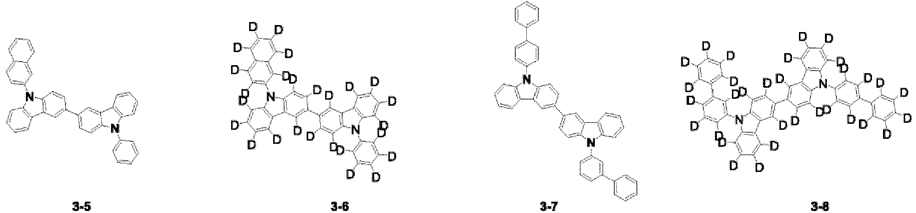

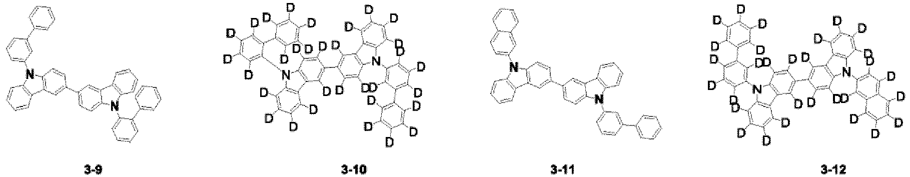

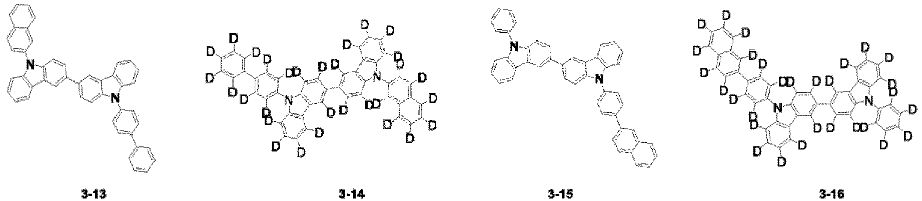

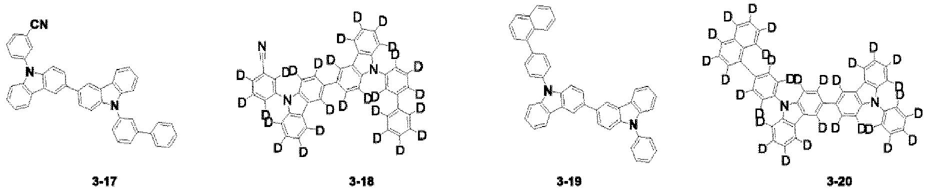

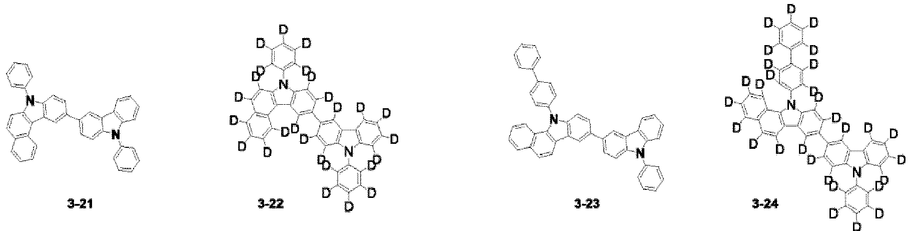

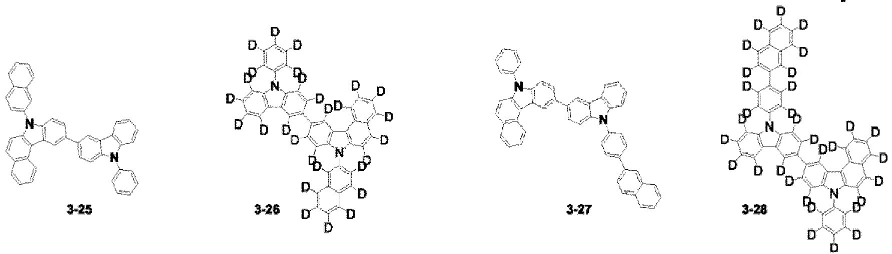

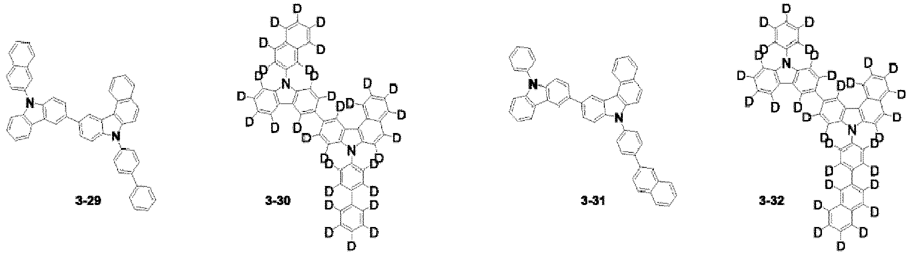

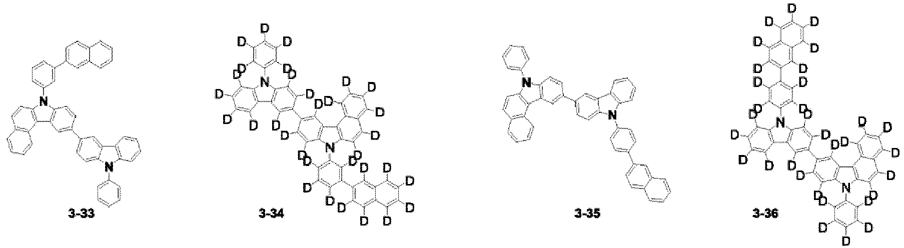

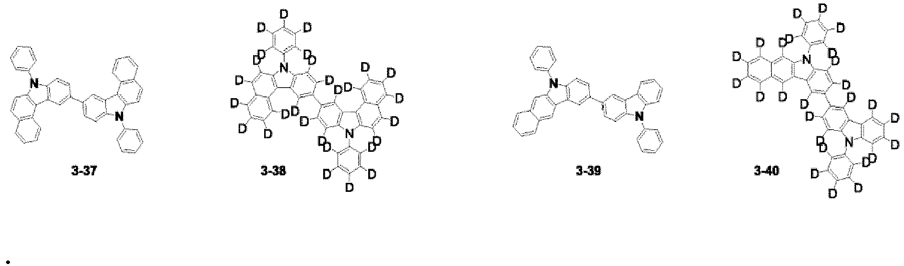

In the organic light emitting device according to an exemplary embodiment of the present application, the compound represented by Chemical Formula A or Chemical Formula B may be included in the light emitting layer among the organic material layers.

In the organic light emitting device according to an exemplary embodiment of the present application, the compound represented by Chemical Formula A or Chemical Formula B may be included in the light emitting layer among the organic material layers, and specifically, may be used as a host material for the light emitting layer.

In an exemplary embodiment of the present application, the host material for the light emitting layer of the organic light emitting device may simultaneously include: the heterocyclic compound of Chemical Formula 1; and the compound of Chemical Formula A or the compound of Chemical Formula B.

In an exemplary embodiment of the present application, in an exemplary embodiment of the present application, provided is a composition for an organic material layer of an organic light emitting device, including: the heterocyclic compound represented by Chemical Formula 1; and the compound of Chemical Formula A or the compound of Chemical Formula B.

The weight ratio of the heterocyclic compound represented by Chemical Formula 1 : the compound of Chemical Formula A or the compound of Chemical Formula B in the composition may be 1 : 10 to 10 : 1, 1 : 8 to 8 : 1, 1 : 5 to 5 : 1, and 1 : 2 to 2 : 1, but is not limited thereto.

In an exemplary embodiment of the present application, provided is a method for manufacturing an organic light emitting device, the method including: preparing a substrate; forming a first electrode on the substrate; forming an organic material layer having one or more layers on the first electrode; and forming a second electrode on the organic material layer, in which the forming of the organic material layer includes forming the organic material layer having one or more layers by using the composition for an organic material layer according to an exemplary embodiment of the present application.

In an exemplary embodiment of the present application, provided is a method for manufacturing an organic light emitting device, in which the forming of the organic material layer forms the organic material layer by pre-mixing the heterocyclic compound represented by Chemical Formula 1 and the compound represented by Chemical Formula 2, and using a thermal vacuum deposition method.

The pre-mixing means that before the heterocyclic compound represented by Chemical Formula 1 and the compound represented by Chemical Formula A are deposited onto an organic material layer, the materials are first mixed and the mixture is contained in one common container and pre-mixed.

The pre-mixing means that before the heterocyclic compound represented by Chemical Formula 1 and the compound represented by Chemical Formula B are deposited onto an organic material layer, the materials are first mixed and the mixture is contained in one common container and mixed.

The pre-mixed material may be referred to as a composition for an organic material layer according to an exemplary embodiment of the present application.

In the organic light emitting device according to an exemplary embodiment of the present application, materials other than the compound of Chemical Formula 1 will be exemplified below, but these materials are illustrative only and are not for limiting the scope of the present application, and may be replaced with materials publicly known in the art.

As a positive electrode material, materials having a relatively high work function may be used, and a transparent conductive oxide, a metal or a conductive polymer, and the like may be used. Specific examples of the positive electrode material include: a metal such as vanadium, chromium, copper, zinc, and gold, or an alloy thereof; a metal oxide such as zinc oxide, indium oxide, indium tin oxide (ITO), and indium zinc oxide (IZO); a combination of a metal and an oxide, such as ZnO:Al or SnO₂:Sb; a conductive polymer such as poly(3-methylthiophene), poly[3,4-(ethylene-1,2-dioxy)thiophene] (PEDOT), polypyrrole, and polyaniline; and the like, but are not limited thereto.

As a negative electrode material, materials having a relatively low work function may be used, and a metal, a metal oxide, or a conductive polymer, and the like may be used. Specific examples of the negative electrode material include: a metal such as magnesium, calcium, sodium, potassium, titanium, indium, yttrium, lithium, gadolinium, aluminum, silver, tin, and lead, or an alloy thereof; a multi-layer structured material, such as LiF/Al or LiO₂/Al; and the like, but are not limited thereto.

As a hole injection material, a publicly-known hole injection material may also be used, and it is possible to use, for example, a phthalocyanine compound such as copper phthalocyanine disclosed in US Patent No. 4,356,429 or starburst-type amine derivatives described in the document [Advanced Material, 6, p. 677 (1994)], for example, tris(4-carbazoyl-9-ylphenyl)amine (TCTA), 4,4',4"-tri[phenyl(m-tolyl)amino]triphenylamine (m-MTDATA), 1,3,5-tris[4-(3-methylphenylphenylamino)phenyl]benzene (m-MTDAPB), polyaniline/dodecylbenzenesulfonic acid or poly(3,4-ethylenedioxythiophene)/poly(4-styrenesulfonate), which is a soluble conductive polymer, polyaniline/camphor sulfonic acid or polyaniline/poly(4-styrene-sulfonate), and the like.

As a hole transport material, a pyrazoline derivative, an arylamine-based derivative, a stilbene derivative, a triphenyldiamine derivative, and the like may be used, and a low-molecular weight or polymer material may also be used.

As an electron transport material, it is possible to use an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyldicyanoethylene and a derivative thereof, a diphenoquinone derivative, a metal complex of 8-hydroxyquinoline and a derivative thereof, and the like, and a low-molecular weight material and a polymer material may also be used.

As an electron injection material, for example, LiF is representatively used in the art, but the present application is not limited thereto.

As a light emitting material, a red, green, or blue light emitting material may be used, and if necessary, two or more light emitting materials may be mixed and used. In this case, two or more light emitting materials are deposited or used as an individual supply source, or pre-mixed to be deposited and used as one supply source. Further, a fluorescent material may also be used as the light emitting material, but may also be used as a phosphorescent material. As the light emitting material, it is also possible to use alone a material which emits light by combining holes and electrons each injected from a positive electrode and a negative electrode, but materials in which a host material and a dopant material are involved in light emission together may also be used.

When hosts of the light emitting material are mixed and used, the same series of hosts may also be mixed and used, and different series of hosts may also be mixed and used. For example, two or more materials selected from n-type host materials or p-type host materials may be used as a host material for a light emitting layer.

The organic light emitting device according to an exemplary embodiment of the present application may be a top emission type, a bottom emission type, or a dual emission type according to the material to be used.

The heterocyclic compound according to an exemplary embodiment of the present application may act even in organic electronic devices including organic solar cells, organic photoconductors, organic transistors, and the like, based on the principle similar to those applied to organic light emitting devices.

Hereinafter, the present specification will be described in more detail through Examples, but these Examples are provided only for exemplifying the present application, and are not intended to limit the scope of the present application.

### <Preparation Examples>

### <Preparation Example 1> Preparation of Compound 1-5

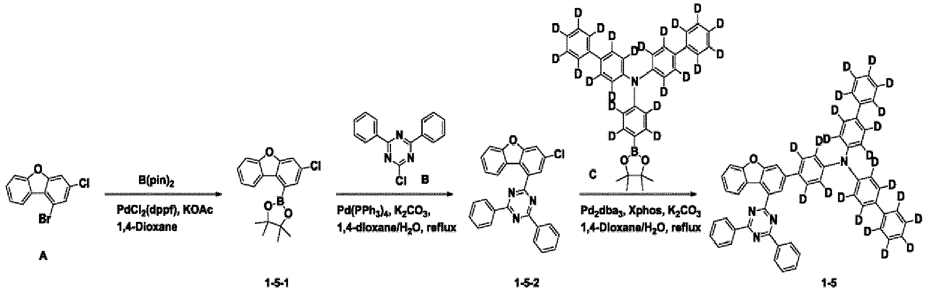

### 1) Preparation of Compound 1-5-1

After 10.0 g (35.5 mmol) of 1-bromo-3-chlorodibenzo[b,d]furan, 13.5 g (53.3 mmol) of 4,4,4',4',5,5,5',5'-octamethyl-2,2'-bi(1,3,2-dioxaborolane) , 1.3 g (1.8 mmol) of PdCl₂(dppf), and 10.5 g (106.5 mmol) of KOAc were dissolved in 100 mL of 1,4-dioxane, the resulting solution was refluxed for 2 hours. After the reaction was completed, the resulting product was filtered under reduced pressure at room temperature, and then the solvent was removed from the filtrate using a rotary evaporator. The reaction product was purified by column chromatography (DCM : Hex = 1 : 3) to obtain 9.8 g (81.4%) of Compound 1-5-1.

### 2) Preparation of Compound 1-5-2

After 9.8 g (28.9 mmol) of Compound 1-5-1, 7.7 g (28.9 mmol) of 2-chloro-4,6-diphenyl-1,3,5-triazine, 1.7 g (1.5 mmol) of Pd(PPh₃)₄, and 11.4 g (82.2 mmol) of K₂CO₃ were dissolved in 100 mL/20 mL of 1,4-dioxane/H₂O, the resulting solution was refluxed for 3 hours. After the reaction was completed, the resulting product was filtered under reduced pressure at room temperature. The residue was dissolved in DCB, purified by silica chromatography, and then recrystallized with DCM/MeOH. 12 g (95%) of Compound 1-5-2 was obtained.

### 3) Preparation of Compound 1-5

After 12.0 g (27.7 mmol) of Compound 1-5-2, 15.1 g (27.7 mmol) of N-([1,1'-biphenyl]-4-yl-d9)-N-(4-(4,4,5,5-tetramethyl-1,3,2-dioxaborolan-2-yl)phenyl-2,3,5,6-d4)-[1,1'-biphenyl]-4-amine-d9, 1.1 g (1.2 mmol) of Pd₂dba₃, 1.2 g(2.3 mmol) of Xphos, and 9.6 g (69.2 mmol) of K₂CO₃ were dissolved in 120 mL/25 mL of 1,4-dioxane/H₂O, the resulting solution was refluxed for 3 hours. After the reaction was completed, the resulting product was filtered under reduced pressure at room temperature. After the residue was dissolved in DCB and purified by silica chromatography, recrystallization was performed using DCB, and then 18 g (80 g) of Target Compound 1-5 was obtained.

The following target compound was synthesized in the same manner as the synthesis in Preparation Example 1, except that Intermediate A in the following Table 1 was used instead of 1-bromo-3-chlorodibenzo[b,d]furan (A) in Preparation Example 1, Intermediate B in the following Table 1 was used instead of 2-chloro-4,6-diphenyl-1,3,5-triazine (B) in Preparation Example 1, and Intermediate C in the following Table 1 was used instead of N-([1,1'-biphenyl]-4-yl-d9)-N-(4-(4,4,5,5-tetramethyl-1,3,2-dioxaborolan-2-yl)phenyl-2,3,5,6-d4)-[1,1'-biphenyl]-4-amine-d9 (C) in Preparation Example 1.

**[Table 1]**

| Compound No. | Intermediate A | Intermediate B | Intermediate C | Yield |
|---|---|---|---|---|
| 1-9 | 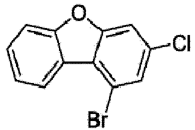 | 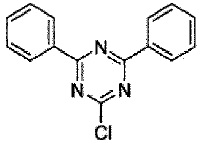 | 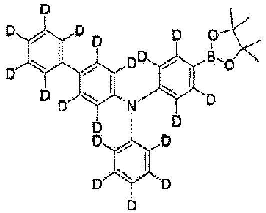 | 82% |
| 1-10 | 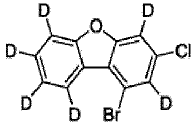 | 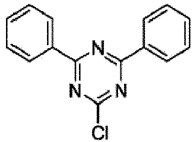 | 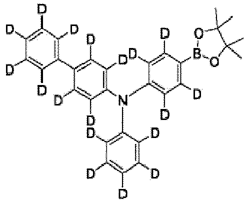 | 88% |
| 1-48 | 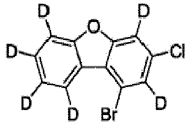 | 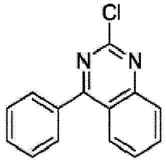 | 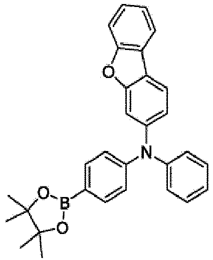 | 79% |
| 1-50 | 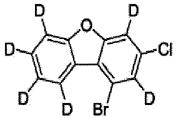 | 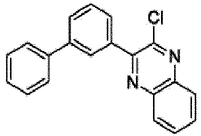 | 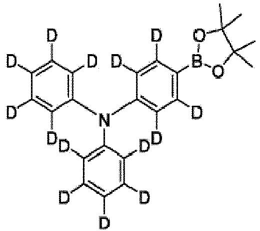 | 78% |
| 1-61 | 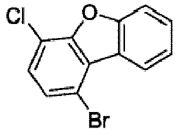 | 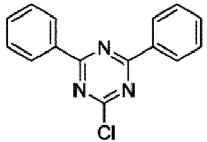 | 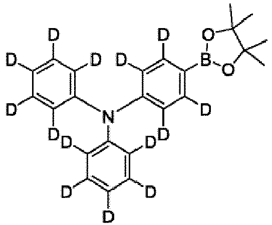 | 81% |
| 1-74 | 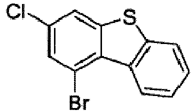 | 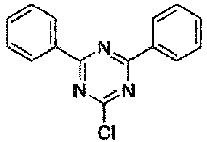 | 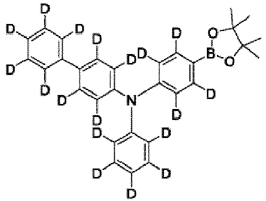 | 77% |
| 1-87 | 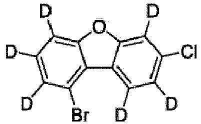 | 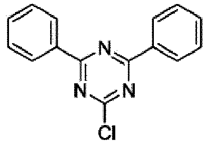 | 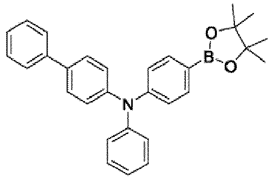 | 91% |
| 1-88 | 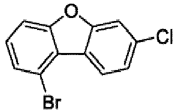 | 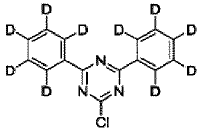 | 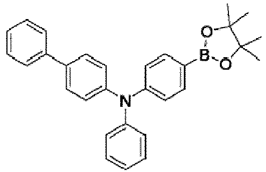 | 72% |
| 1-89 | 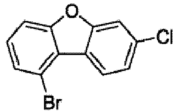 | 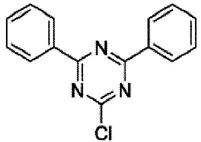 | 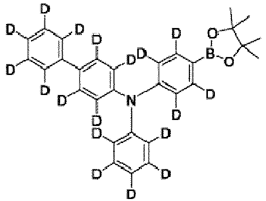 | 88% |
| 1-90 | 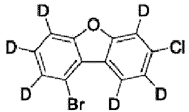 | 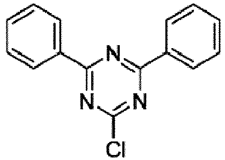 | 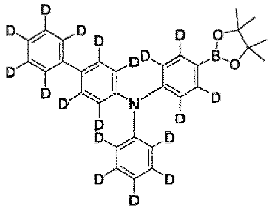 | 81% |
| 1-92 | 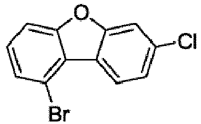 | 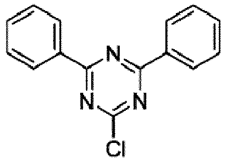 | 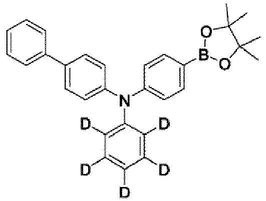 | 85% |
| 1-105 | 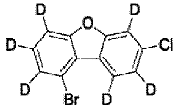 | 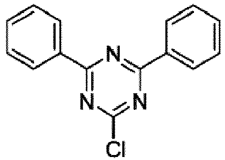 | 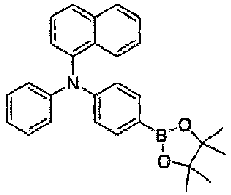 | 77% |
| 1-112 | 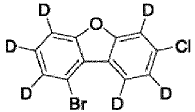 | 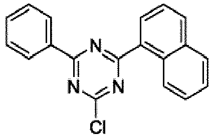 | 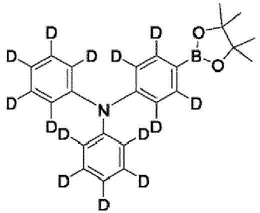 | 78% |
| 1-118 | 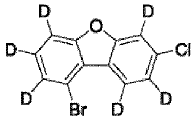 | 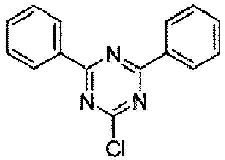 | 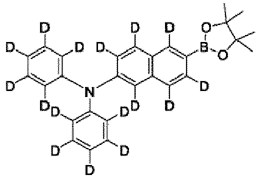 | 86% |
| 1-119 | 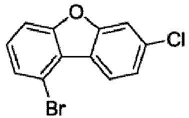 | 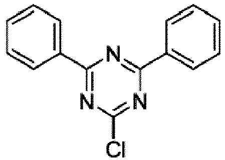 | 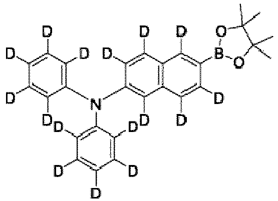 | 81% |
| 1-146 | 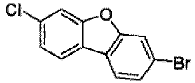 | 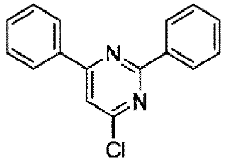 | 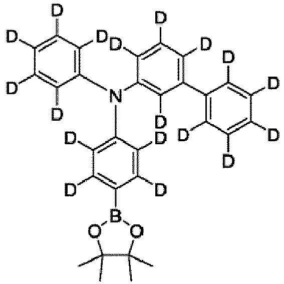 | 76% |
| 1-151 | 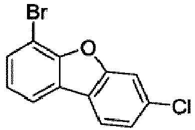 | 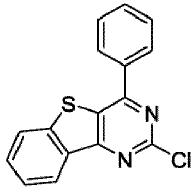 | 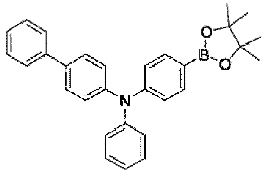 | 68% |
| 1-158 | 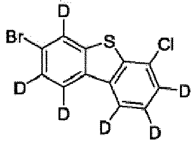 | 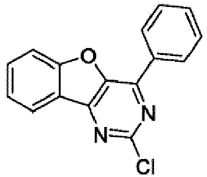 | 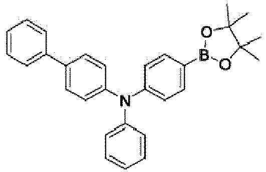 | 84% |
| 1-176 | 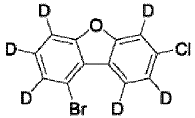 | 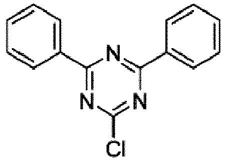 | 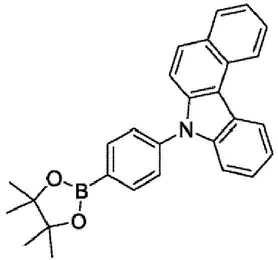 | 75% |
| 1-181 | 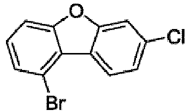 | 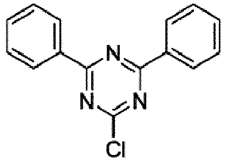 | 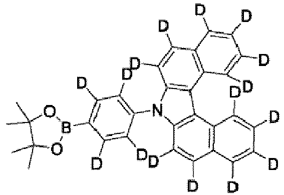 | 83% |
| 1-206 | 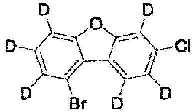 | 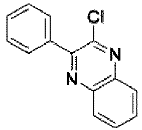 | 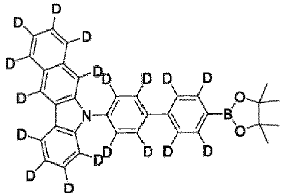 | 71% |
| 1-209 | 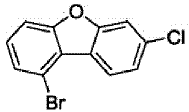 | 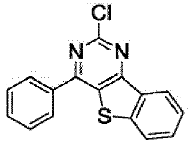 | 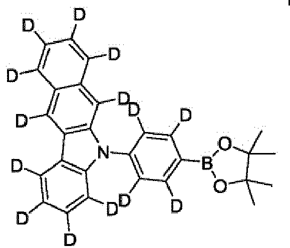 | 90% |
| 1-216 | 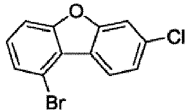 | 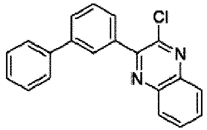 | 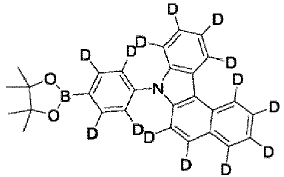 | 88% |

### <Preparation Example 2> Preparation of Compound 1-6

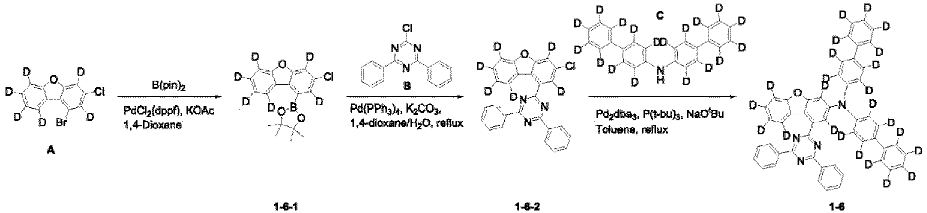

### 1) Preparation of Compound 1-6-1

9.9 g (83%) of Compound 1-6-1 was obtained in the same manner as in the preparation of Compound 1-5-1 in Preparation Example 1, except that 1-bromo-3-chlorodibenzo[b,d]furan-2,4,6,7,8,9-d6 was used instead of 1-bromo-3-chlorodibenzo[b,d]furan in the preparation of Compound 1-5-1 in Preparation Example 1.

### 2) Preparation of Compound 1-6-2

12.3 g (95%) of Compound 1-6-2 was obtained in the same manner as in the preparation of Compound 1-5-2 in Preparation Example 1, except that Compound 1-6-1 was used instead of Compound 1-5-1 in the preparation of Compound 1-5-2 in Preparation Example 1.

### 3) Preparation of Compound 1-6

After 12.3 g (28.0 mmol) of Compound 1-6-2, 9.5 g (28.0 mmol) of bis([1,1'-biphenyl]-4-yl-d8)amine, 1.1 g (1.2 mmol) of Pd₂dba₃, 1.0 ml (2.4 mmol) of P(t-bu)3, and 6.6 g (69.0 mmol) of NaOtBu were dissolved in 120 mL of toluene, the resulting solution was refluxed for 3 hours. After the reaction was completed, the resulting product was filtered under reduced pressure at room temperature. The residue was dissolved in DCB, purified by silica chromatography, and then recrystallized with DCB/MeOH to obtain 15.0 g (72%) of Target Compound 1-6.

The following target compound was synthesized in the same manner as in the synthesis in Preparation Example 2, except that Intermediate A in the following Table 2 was used instead of 1-bromo-3-chlorodibenzo[b,d]furan-2,4,6,7,8,9-d6 (A) in Preparation Example 2, Intermediate B in the following Table 2 was used instead of 2-chloro-4,6-diphenyl-1,3,5-triazine (B) in Preparation Example 2, and Intermediate C in the following Table 2 was used instead of bis([1,1'-biphenyl]-4-yl-d8)amine (C) in Preparation Example 2.

**[Table 2]**

| Compound No. | Intermediate A | Intermediate B | Intermediate C | Yield |
|---|---|---|---|---|
| 1-23 | 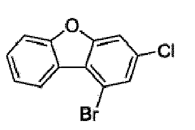 | 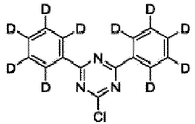 | 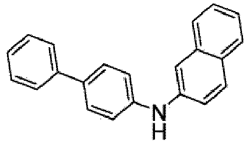 | 77% |
| 1-33 | 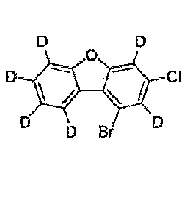 | 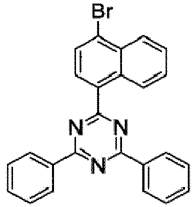 | 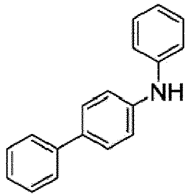 | 81% |
| 1-70 | 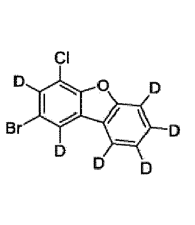 | 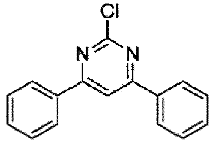 | 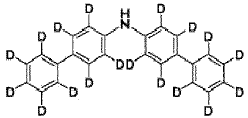 | 72%. |
| 1-95 | 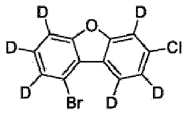 | 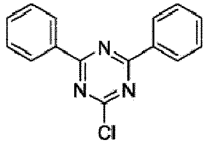 | 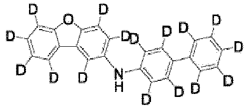 | 76% |
| 1-96 | 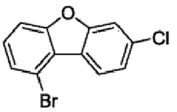 | 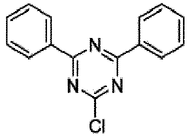 | 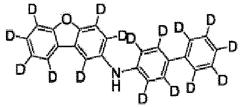 | 85% |
| 1-113 | 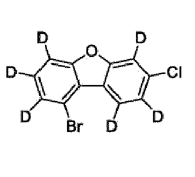 | 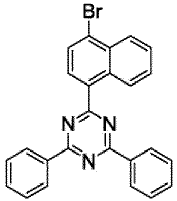 | 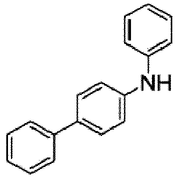 | 78% |
| 1-124 | 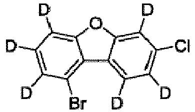 | 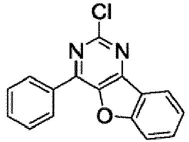 | 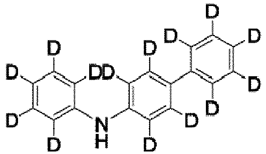 | 81% |
| 1-161 | 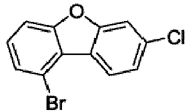 | 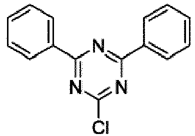 | 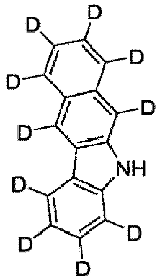 | 7 7% |
| 1-162 | 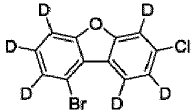 | 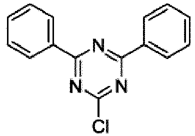 | 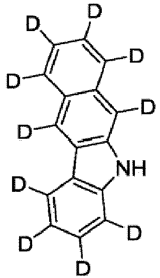 | 85% |
| 1-164 | 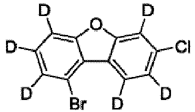 | 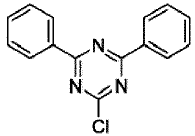 | 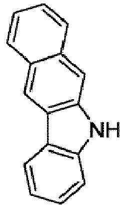 | 86% |
| 1-175 | 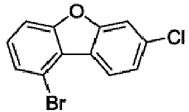 | 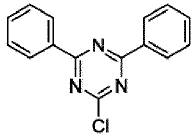 | 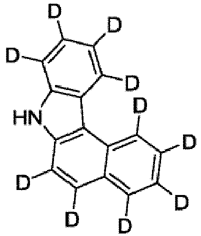 | 82% |
| 1-197 | 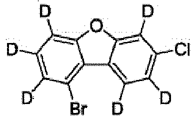 | 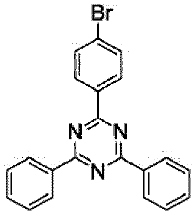 | 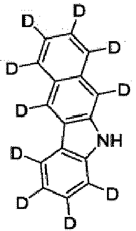 | 74% |
| 1-202 | 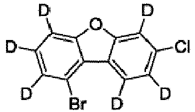 | 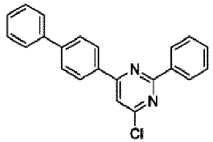 | 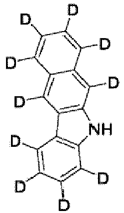 | 76% |
| 1-217 | 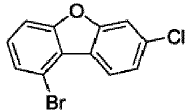 | 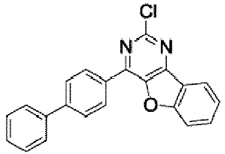 | 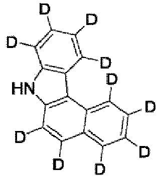 | 82% |
| 1-224 | 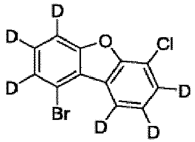 | 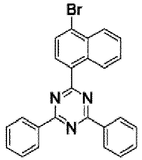 | 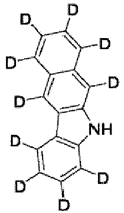 | 75% |
| 1-234 | 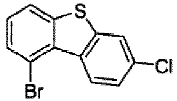 | 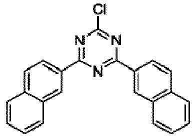 | 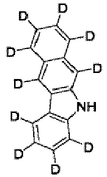 | 73% |
| 1-237 | 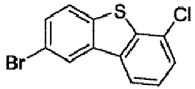 | 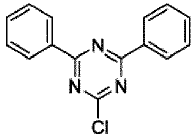 | 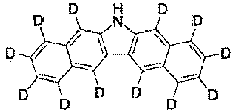 | 76% |

### <Preparation Example 3> Preparation of Compound 1-7

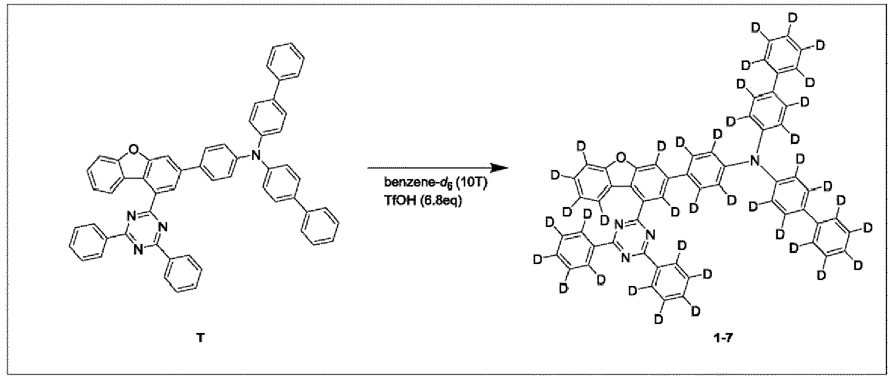

### 1) Preparation of Compound 1-7

Compound T may be synthesized by the methods of Preparation Examples 1 and 2 using a compound in which deuterium is not substituted. After Compound T (8 g, 10.06 mmol) was dissolved in benzene-d6 (80 mL), the temperature was adjusted to 0°C using an ice bath, and then trifluoromethanesulfonic acid (6.0 mL, 68.41 mmol) was slowly added dropwise thereto. Thereafter, the resulting mixture was stirred at 60°C for 1 hour. After a mixed solution in which the reaction was completed was cooled to room temperature, an ice bath is installed. Thereafter, the mixed solution was neutralized using a K₃PO₄ aqueous solution. A solid was precipitated using methanol and filtered. 8 g (95%) of Compound 1-7 was obtained.

The following target compound was synthesized in the same manner as in Preparation Example 3, except that Compound K in the following Table 3 was used instead of Compound T in Preparation Example 3.

**[Table 3]**

| Compound No. | Compound K | Target compound | Yield |
|---|---|---|---|
| 1-11 | 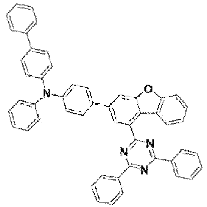 | 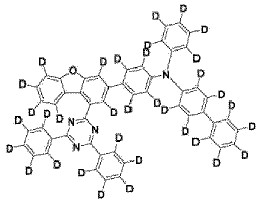 | 91% |
| 1-91 | 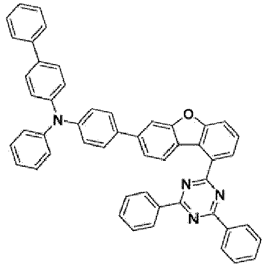 | 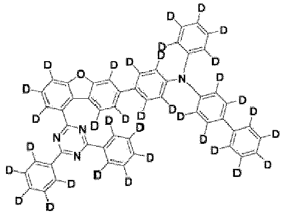 | 92% |
| 1-171 | 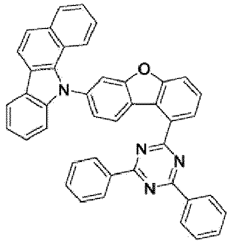 | 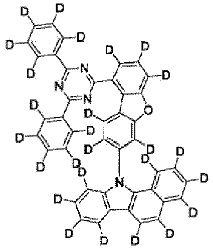 | 87% |
| 1-221 | 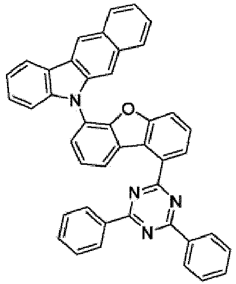 | 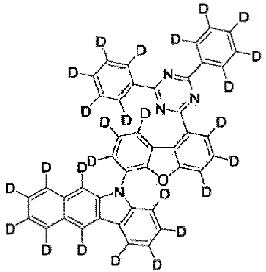 | 85% |

### <Preparation Example 4> Preparation of Compound 2-12

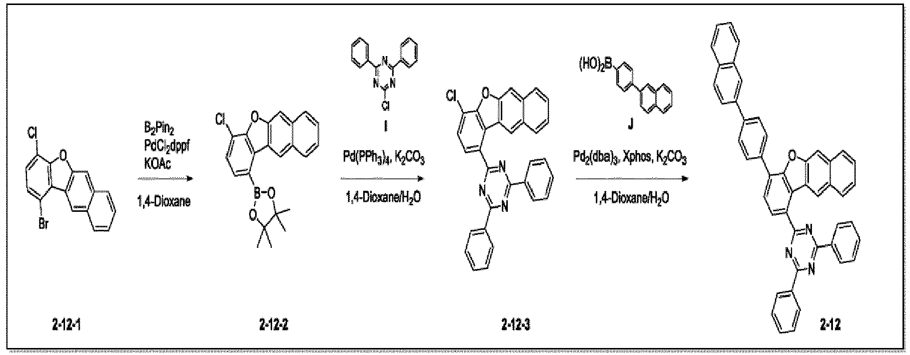

### 1) Preparation of Compound 2-12-2

After Compound 2-12-1 (20 g, 60.31 mmol), bis(pinacolato)diboron (30.6 g, 120.63 mmol), PdCl₂dppf (2.2 g, 3.02 mmol), and KOAc (18 g, 180.93 mmol) were dissolved in 1,4-dioxane (200 mL), the resulting solution was stirred under reflux at 120°C for 3 hours. A mixed solution in which the reaction was completed was extracted using MC and water, the organic layer was treated with anhydrous MgSO₄, a filtered solution was concentrated. The concentrate was dissolved in MC, and then silica gel-filtered. The solvent was removed from the filtered filtrate using a rotary evaporator, and the residue was recrystallized using MC and methanol. 20 g (87%) of Compound 2-12-2 was obtained.

### 2) Preparation of Compound 2-12-3

After Compound 2-12-2 (20 g, 52.83 mmol), Compound I (14 g, 52.83 mmol), Pd(PPh₃)₄ (3 g, 2.64 mmol), and K₂CO₃ (14.5 g, 105.66 mmol) were dissolved in 1,4-dioxane/H₂O (250 mL/50 mL), the resulting solution was stirred at 110°C for 6 hours. After the reaction was completed, a precipitated solid was filtered and washed with water (H₂O) and methanol (MeOH) to obtain 17 g (66%) of Compound 2-12-3.

### 3) Preparation of Compound 2-12

After Compound 2-12-3 (8 g, 16.53 mmol), Compound J (4.1 g, 16.53 mmol), Pd₂(dba)₃ (0.76 g, 0.83 mmol), Xphos (0.78 g, 1.65 mmol), and K₂CO₃ (5.7 g, 41.35 mmol) were dissolved in 1,4-dioxane/H₂O (150 mL/30 mL), the resulting solution was stirred at 110°C for 7 hours. After the reaction was completed, a precipitated solid was filtered and washed with water (H₂O) and acetone to obtain 8 g (74%) of Target Compound 2-12 which is a white solid.

### <Preparation Example 5> Preparation of Compound 2-15

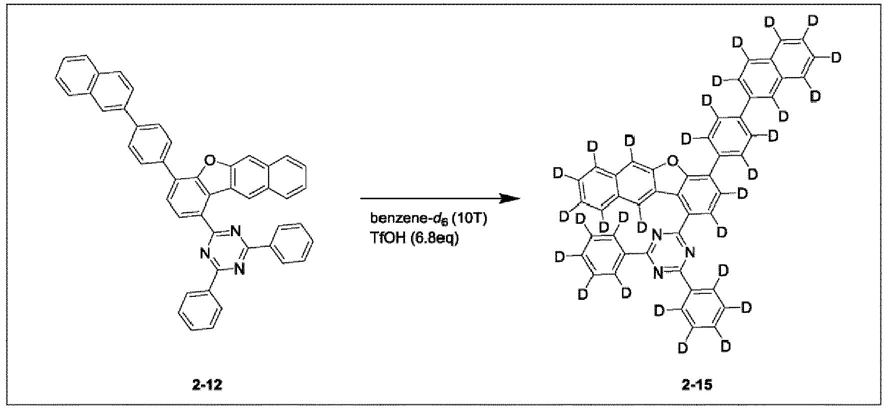

### 1) Preparation of Compound 2-15

After Compound 2-12 (10 g, 15.36 mmol) was dissolved in benzene-d6 (100 mL), the temperature was adjusted to 0°C using an ice bath, and then trifluoromethanesulfonic acid (9.2 mL, 104.44 mmol) was slowly added dropwise thereto. Thereafter, the resulting mixture was stirred at 60°C for 1 hour. After a mixed solution in which the reaction was completed was cooled to room temperature, an ice bath is installed. Thereafter, the mixed solution was neutralized using a K₃PO₄ aqueous solution. A solid was precipitated using methanol and filtered. 8.5 g (82%) of Compound 2-15 was obtained.

### <Preparation Example 6> Preparation of Compound 3-21

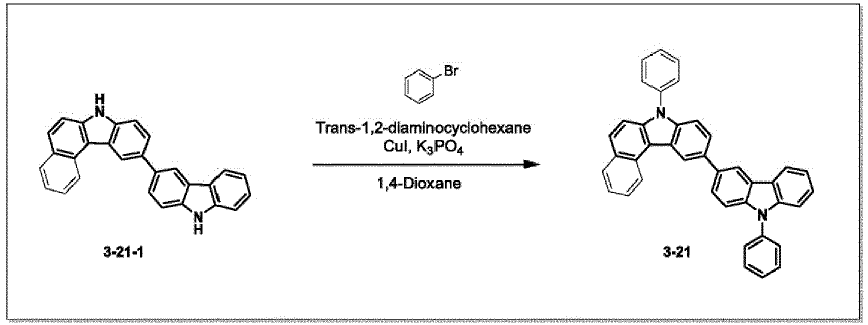

### 1) Preparation of Compound 3-21

After Compound 3-21-1 (5.7 g, 15 mmol), bromobenzene (4.9 g, 31.5 mmol), CuI (0.57 g, 30 mmol), trans-1,2-diaminocyclohexane (0.34 g, 30 mol), and K₃PO₄ (12.74 g, 60 mmol) were dissolved in 50 mL of 1,4-dioxane, the resulting solution was stirred under reflux at 125°C for 14 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hexane = 1:3) and sonicated with methanol to obtain Target Compound 3-21 (6.4 g, 12 mmol) which is a white solid.

### <Preparation Example 7> Preparation of Compound 3-22

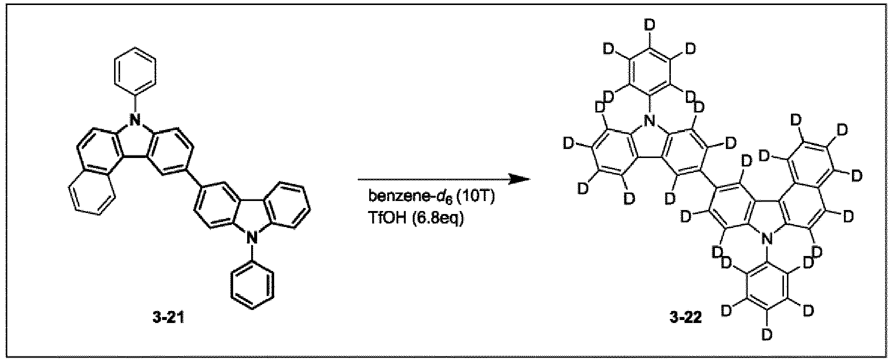

### 1) Preparation of Compound 3-22

After Compound 3-21 (10 g, 18.7mmol) was dissolved in benzene-d6 (100 mL), the temperature was adjusted to 0°C using an ice bath, and then trifluoromethanesulfonic acid (11.2 mL, 127.16 mmol) was slowly added dropwise thereto. Thereafter, the resulting mixture was stirred at 60°C for 1 hour. After a mixed solution in which the reaction was completed was cooled to room temperature, an ice bath is installed. Thereafter, the mixed solution was neutralized using a K₃PO₄ aqueous solution. A solid was precipitated using methanol and filtered. 9 g (87%) of Compound 3-22 was obtained.

The following Tables 4 and 5 are the 1H NMR data and FD-MS data of the synthesized compounds, and it can be confirmed through the following data that the desired compound was synthesized.

**[Table 4]**

| Compound No. | ¹H NMR (CDCl₃, 400Mz) |
|---|---|
| 1-5 | δ = 8.36(d, 4H), 7.98(d, 1H), 7.86(s, 1H), 7.81(s, 1H), 7.54~7.50(m, 7H), 7.39(t, 1H), 7.31(t, 1H), |
| 1-6 | δ = 8.36(d, 4H), 7.50(m, 6H) |
| 1-9 | δ = 8.36(d, 4H), 7.98(d, 1H), 7.86(s, 1H), 7.81(s, 1H), 7.54-7.50(m, 7H), 7.39(t, 1H), 7.31(t, 1H), |
| 1-10 | δ = 8.36(d, 4H), 7.50(m, 6H) |
| 1-23 | δ = 8.20(s, 1H), 8.13(s, 1H), 7.98(d, 1H), 7.78~7.71(m, 4H), 7.55~7.31(m, 14H), 7.11(s, 1H) |
| 1-33 | δ = 9.02(d, 1H), 8.95(d, 1H), 8.36(d, 4H), 8.06(d, 1H), 7.84(d, 1H), 7.75(d, 2H), 7.55~7.37(m, 15H), 7.24(t, 2H), 7.08~7.00(m, 3H) |
| 1-48 | δ = 8.13(d, 1H), 8.03(s, 1H), 7.98(d, 1H), 7.84~7.80(m, 5H), 7.65~7.24(m, 13H), 7.08~7.00(m, 3H), 6.91(d, 1H) |
| 1-50 | δ = 8.50(m, 2H), 7.80~7.67(m, 8H), 7.49~7.41(m, 3H) |
| 1-61 | δ = 8.36(m, 4H), 8.07~7.98(m, 3H), 7.54~7.50(m, 7H), 7.39~7.31(m, 2H) |
| 1-70 | δ = 8.23(s, 1H), 7.94(d, 4H), 7.55~7.49(m, 6H) |
| 1-74 | δ = 8.45(d, 1H), 8.36~8.30(m, 5H), 8.01(s, 1H), 7.93(d, 1H), 7.56~7.49(m, 8H) |
| 1-87 | δ = 8.36(d, 4H), 7.75(d, 2H), 7.55~7.37(m, 17H), 7.24(t, 2H), 7.08~7.00(m, 3H) |
| 1-88 | δ = 8.03(d, 1H), 7.82~7.69(m, 6H), 7.57~7.37(m, 12H), 7.24(t, 2H), 7.08~7.00(m, 3H) |
| 1-89 | δ = 8.36(d, 4H), 8.03(d, 1H), 7.82~7.76(m, 3H), 7.69(d, 1H), 7.57~7.50(m, 7H) |
| 1-90 | δ = 8.36(d, 4H), 7.50(d, 6H) |
| 1-92 | δ = 8.36(d, 4H), 8.03(d, 1H), 7.82~7.69(m, 6H), 7.57~7.37(m, 18H) |
| 1-95 | δ = 8.36(d, 4H), 7.50(d, 6H) |
| 1-96 | δ = 8.36(d, 4H), 8.03(s, 1H), 7.82~7.80(m, 2H), 7.69(d, 1H), 7.57~7.50(m, 7H), 6.91(s, 1H) |
| 1-105 | δ = 8.36(d, 4H), 8.22(d, 1H), 8.15(d, 1H), 7.81(d, 1H), 7.63~7.49(m, 12H), 7.37(d, 2H), 7.24(t, 2H), 7.08~7.00(m, 3H) |
| 1-112 | δ = 8.97(d, 1H), 8.36(d, 2H), 8.25(d, 2H), 8.15~8.10(m, 2H), 8.00(t, 1H), 7.59~7.50(m, 5H) |
| 1-113 | δ = 9.02(d, 1H), 8.95(d, 1H), 8.36(d, 4H), 8.06(d, 1H), 7.84(d, 1H), 7.75(d, 2H), 7.55~7.37(m, 15H), 7.24(t, 2H), 7.08~7.00(m, 3H) |
| 1-118 | δ = 8.36(d, 4H), 7.50(d, 6H) |
| 1-119 | δ = 8.36(d, 4H), 8.03(d, 1H), 7.82~7.76(m, 3H), 7.69(d, 1H), 7.57~7.50(m, 7H) |
| 1-124 | δ = 7.84(d, 2H), 7.70(d, 1H), 7.65(d, 1H), 7.53~7.49(m, 3H), 7.36(t, 1H), 7.22(t, 1H) |
| 1-146 | δ = 8.35(d, 2H), 8.23(s, 1H), 8.03(d, 2H), 7.94(d, 2H), 7.82~7.76(m, 4H), 7.55~7.49(m, 6H) |
| 1-151 | δ = 8.05(d, 1H), 7.93(d, 1H), 7.84(d, 2H), 7.75(d, 2H), 7.55~7.37(m, 16H), 7.24(t, 2H), 7.08(d, 2H), 7.00(t, 1H) |
| 1-158 | δ = 7.84(d, 2H), 7.75~7.65(m, 4H), 7.55~7.36(m, 15H), 7.24(t, 2H), 7.22(t, 1H), 7.08(d, 2H), 7.00(t, 1H) |
| 1-161 | δ = 8.36(d, 4H), 7.98(d, 1H), 7.82(d, 1H), 7.69(d, 1H), 7.57~7.50(m, 8H), 7.25(d, 1H) |
| 1-162 | δ = 8.36(d, 4H), 7.50(d, 6H) |
| 1-164 | δ = 8.55(d, 1H), 8.36~8.28(m, 5H), 8.11(d, 1H), 7.94(d, 1H), 7.75~7.69(m, 2H), 7.55~7.50(m, 7H), 7.40~7.35(m, 2H), 7.16(t, 1H) |
| 1-175 | δ = 8.36(d, 4H), 7.98(d, 1H), 7.82(d, 1H), 7.69(d, 1H), 7.57~7.50(m, 8H), 7.25(d, 1H) |
| 1-176 | δ = 8.55(d, 1H), 8.54(d, 1H), 8.36(d, 4H), 7.99~7.91(m, 6H), 7.65~7.50(m, 10H), 7.35(t, 1H), 7.16(t, 1H) |
| 1-181 | δ = 8.36(d, 4H), 8.03(d, 1H), 7.82~7.76(m, 3H), 7.69(d, 1H), 7.57~7.50(m, 7H) |
| 1-197 | δ = 8.36(d, 4H), 7.96(d, 2H), 7.50(d, 6H), 7.25(d, 2H) |
| 1-202 | δ = 8.35~8.30(m, 4H), 8.23(s, 1H), 7.85(d, 2H), 7.75(d, 2H), 7.50~7.41(m, 6H) |
| 1-206 | δ = 8.03(d, 2H), 7.80(d, 2H), 7.67~7.59(m, 3H), 7.32(t, 2H) |
| 1-209 | δ = 8.05~8.03(m, 2H), 7.93(d, 1H), 7.84~7.69(m, 6H), 7.57~7.42(m, 6H) |
| 1-216 | δ = 8.50(m, 2H), 8.38(d, 1H), 8.03(d, 1H), 7.82~7.67(m, 12H), 7.49~7.41(m, 3H) |
| 1-217 | δ = 8.30(d, 2H), 7.98(d, 1H), 7.85~7.36(m, 14H), 7.25(d, 1H), 7.22(t, 1H) |
| 1-224 | δ = 8.95(d, 2H), 8.36(d, 4H), 8.06(d, 1H), 7.84(d, 1H), 7.52~7.46(m, 8H) |
| 1-234 | δ = 9.09(s, 2H), 8.49(d, 2H), 8.19~7.94(m, 10H), 7.68~7.59(m, 5H), 7.45(d, 1H) |
| 1-237 | δ = 8.41~8.36(m, 5H), 8.12(s, 1H), 7.99(d, 1H), 7.92(d, 1H), 7.65(t, 1H), 7.50~7.43(m, 7H) |
| 2-12 | δ = 8.36~8.28(m, 5H), 8.11~7.99(m, 6H), 7.75~7.49(m, 12H), 7.42(s, 1H), 7.38(d, 1H), 7.25(d, 4H) |
| 3-21 | δ = 8.54(d, 1H), 8.30(d, 2H), 8.19~8.13(m, 3H), 7.99(d, 1H), 7.89(s, 2H), 7.65~7.50(m, 16H), 7.20(t, 1H) |

**[Table 5]**

| Compound | FD-Mass | Compound | FD-Mass |
|---|---|---|---|
| 1-5 | m/z= 817.09 (C57H16D22N4O, 816.44) | 1-124 | m/z= 675.88 (C46H9D20N3O2, 675.35) |
| 1-6 | m/z= 743.01 (C51H10D24N4O, 742.42) | 1-146 | m/z= 735.98 (C52H17D18N3O, 735.39) |
| 1-7 | m/z= 833.19 (C57D38N4O, 832.54) | 1-151 | m/z= 753.95 (C52H27D6N3OS, 753.27) |
| 1-9 | m/z= 736.97 (C51H16D18N4O, 736.39) | 1-158 | m/z= 753.95 (C52H27D6N3OS, 753.27) |
| 1-10 | m/z= 743.01 (C51H10D24N4O, 742.42) | 1-161 | m/z= 624.77 (C43H16D10N4O, 624.27) |
| 1-11 | m/z= 753.07 (C51D34N4O, 752.49) | 1-162 | m/z= 630.81 (C43H10D16N4O, 630.31) |
| 1-23 | m/z= 702.88 (C49H22D10N4O, 702.32) | 1-164 | m/z= 620.74 (C43H20D6N4O, 620.25) |
| 1-33 | m/z= 774.96 (C55H30D6N4O, 774.33) | 1-171 | m/z= 640.87 (C43D26N4O, 640.37) |
| 1-48 | m/z= 711.85 (C50H25D6N3O2, 711.28) | 1-175 | m/z= 624.77 (C43H16D10N4O, 624.27) |
| 1-50 | m/z= 711.96 (C50H13D20N3O, 711.39) | 1-176 | m/z= 696.84 (C49H24D6N4O, 696.28) |
| 1-61 | m/z= 656.85 (C45H16D14N4O, 656.33) | 1-181 | m/z= 756.96 (C53H16D16N4O, 756.36) |
| 1-70 | m/z= 742.02 (C52H11D24N3O, 741.43) | 1-197 | m/z= 706.90 (C49H14D16N4O, 706.34) |
| 1-74 | m/z= 753.03 (C51H16D18N4S, 752.36) | 1-202 | m/z= 705.92 (C50H15D16N3O, 705.35) |
| 1-87 | m/z= 724.90 (C51H28D6N4O, 724.31) | 1-206 | m/z= 764.02 (C54H9D24N3O, 763.41) |
| 1-88 | m/z= 728.92 (C51H24D10N4O, 728.34) | 1-209 | m/z= 733.95 (C50H15D14N3OS, 733.29) |
| 1-89 | m/z= 736.97 (C51H16D18N4O, 736.39) | 1-216 | m/z= 753.96 (C54H19D14N3O, 753.35) |
| 1-90 | m/z= 743.01 (C51H10D24N4O, 742.42) | 1-217 | m/z= 713.86 (C50H19D10N3O2, 713.29) |
| 1-91 | m/z= 752.07 (C51D34N4O, 752.49) | 1-221 | m/z= 640.87 (C43D26N4O, 640.37) |
| 1-92 | m/z= 723.89 (C51H29D5N4O, 723.30) | 1-224 | m/z= 756.96 (C53H16D16N4O, 756.36) |
| 1-95 | m/z= 754.98 (C51H10D22N4O2, 754.39) | 1-234 | m/z= 740.95 (C51H20D10N4S, 740.28) |
| 1-96 | m/z= 748.94 (C51H16D16N4O2, 748.35) | 1-237 | m/z= 692.90 (C47H16D12N4S, 692.28) |
| 1-105 | m/z= 698.86 (C49H26D6N4O, 698.30) | 2-12 | m/z= 651.77 (C47H29N3O, 651.23) |
| 1-112 | m/z= 712.94 (C49H12D20N4O, 712.38) | 2-15 | m/z= 680.95 (C47D29N3O, 680.41) |
| 1-113 | m/z= 774.96 (C55H30D6N4O, 774.33) | 3-21 | m/z= 534.66 (C40H26N2, 534.21) |
| 1-118 | m/z= 714.96 (C49H10D22N4O, 714.40) | 3-22 | m/z= 560.82 (C40D26N2, 560.37) |
| 1-119 | m/z= 708.92 (C49H16D16N4O, 708.36) | | |

### [Experimental Examples]

### <Experimental Example 1-1>-Manufacture of organic light emitting device

A glass substrate, in which indium tin oxide (ITO) was thinly coated to have a thickness of 1,500 Å, was ultrasonically washed with distilled water. When the washing with distilled water was finished, the glass substrate was ultrasonically washed with a solvent such as acetone, methanol, and isopropyl alcohol, was dried and then was subjected to ultraviolet ozone (UVO) treatment for 5 minutes by using UV in an ultraviolet (UV) washing machine. Thereafter, the substrate was transferred to a plasma washing machine (PT), and then was subjected to plasma treatment in a vacuum state for an ITO work function and in order to remove a residual film, and was transferred to a thermal deposition apparatus for organic deposition.

The hole injection layer 4,4',4"-tris[2-naphthyl(phenyl)amino] triphenylamine (2-TNATA) and the hole transport layer N,N'-diphenyl-(1,1'-biphenyl)-4,4'-diamine (NPB), which are common layers, were formed on the ITO transparent electrode (positive electrode).

A light emitting layer was thermally vacuum deposited thereon as follows. The light emitting layer was deposited to have a thickness of 500 Å by using a compound described in the following Table 6 as a red host (or a green host) and (piq)2(Ir)(acac) as a red phosphorescent dopant (or doped with 7% of a green phosphorescent dopant Ir(ppy)3) to dope the host with (piq)2(Ir) (acac) in an amount of 3%. Thereafter, BCP as a hole blocking layer was deposited to have a thickness of 60 Å, and Alq3 as an electron transport layer was deposited to have a thickness of 200 Å thereon. Thereafter, BCP as a hole blocking layer was deposited to have a thickness of 60 Å, and Alq3 as an electron transport layer was deposited to have a thickness of 200 Å thereon. Finally, lithium fluoride (LiF) was deposited to have a thickness of 10 Å on the electron transport layer to form an electron injection layer, and then aluminum (Al) negative electrode was deposited to have a thickness of 1,200 Å on the electron injection layer to form a negative electrode, thereby manufacturing an organic electroluminescence device.

Meanwhile, all the organic compounds required for manufacturing an OLED device were subjected to vacuum sublimed purification under 10⁻⁶ to 10⁻⁸ torr for each material, and used for the manufacture of OLED.

For the organic electroluminescence device manufactured as described above, electroluminescence (EL) characteristics were measured by M7000 manufactured by McScience Inc., and based on the measurement result thereof, T₉₀ was measured by a service life measurement equipment (M6000) manufactured by McScience Inc., when the reference luminance was 6,000 cd/m². Characteristics of the organic electroluminescence device of the present invention are as described in the following Table 6.

**[Table 6]**

| | Compound | Life Time (T₉₀) | | Compound | Life Time (T₉₀) |
|---|---|---|---|---|---|
| Example 1 | 1-5 | 320 | Comparative 1 Example | T | 221 |
| Example 2 | 1-7 | 328 | | | |
| Example 3 | 1-9 | 318 | Comparative 2 Example | U1 | 226 |
| Example 4 | 1-10 | 325 | | | |
| Example 5 | 1-11 | 332 | | | |
| Example 6 | 1-23 | 235 | Comparative 3 Example | U2 | 224 |
| Example 7 | 1-33 | 232 | Comparative 4 Example | U3 | 211 |
| Example 8 | 1-48 | 241 | Comparative 5 Example | U4 | 215 |
| Example 9 | 1-50 | 327 | Comparative 6 Example | U5 | 218 |
| Example 10 | 1-61 | 280 | Comparative 7 Example | U6 | 198 |
| Example 11 | 1-70 | 312 | Comparative 8 Example | U7 | 215 |
| Example 12 | 1-74 | 252 | Comparative 9 Example | U8 | 180 |
| Example 13 | 1-87 | 253 | Comparative 10 Example | V | 228 |
| Example 14 | 1-88 | 235 | | | |
| Example 15 | 1-89 | 330 | | | |
| Example 16 | 1-90 | 346 | | | |
| Example 17 | 1-91 | 360 | | | |
| Example 18 | 1-92 | 234 | | | |
| Example 19 | 1-95 | 340 | Comparative 11 Example | U9 | 222 |
| Example 20 | 1-96 | 315 | | | |
| Example 21 | 1-105 | 224 | Comparative 12 Example | U10 | 203 |
| Example 22 | 1-112 | 332 | Comparative 13 Example | U11 | 217 |
| Example 23 | 1-113 | 249 | Comparative14 Example | U12 | 221 |
| Example 24 | 1-118 | 351 | Comparative 15 Example | U13 | 225 |
| Example 25 | 1-119 | 317 | | | |
| Example 26 | 1-124 | 339 | Comparative 16 Example | U14 | 219 |
| Example 27 | 1-146 | 290 | Comparative 17 Example | U15 | 203 |
| Example 28 | 1-151 | 205 | Comparative 18 Example | U16 | 185 |
| Example 29 | 1-158 | 228 | Comparative 19 Example | U17 | 200 |
| Example 30 | 1-161 | 299 | Comparative 20 Example | W | 205 |
| Example 31 | 1-162 | 321 | | | |
| Example 32 | 1-164 | 232 | | | |
| Example 33 | 1-171 | 331 | Comparative 21 Example | W2 | 210 |
| Example 34 | 1-175 | 290 | Comparative22 Example | U18 | 200 |
| Example 35 | 1-176 | 229 | Comparative23 Example | U19 | 203 |
| Example 36 | 1-181 | 288 | Comparative 24 Example | U20 | 201 |
| Example 37 | 1-197 | 325 | Comparative 25 Example | U21 | 206 |
| Example 38 | 1-202 | 313 | Comparative 26 Example | U22 | 202 |
| Example 39 | 1-206 | 309 | Comparative 27 Example | U23 | 198 |
| Example 40 | 1-209 | 286 | Comparative 28 Example | U24 | 200 |
| Example 41 | 1-216 | 297 | Comparative 29 Example | U25 | 207 |
| Example 42 | 1-217 | 293 | Comparative 30 Example | U26 | 204 |
| Example 43 | 1-221 | 302 | Comparative 31 Example | W3 | 200 |
| Example 44 | 1-224 | 300 | Comparative 32 Example | U27 | 196 |
| Example 45 | 1-234 | 288 | Comparative 33 Example | U28 | 203 |
| Example 46 | 1-237 | 266 | Comparative 34 Example | U29 | 186 |
| | | | Comparative35 Example | Y | 211 |
| | | | Comparative 36 Example | Z | 174 |
| 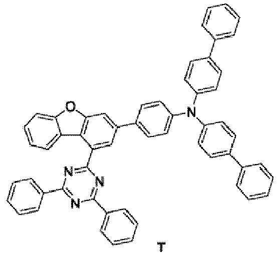 | 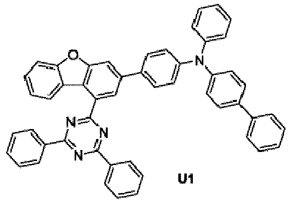 | 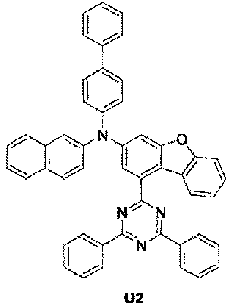 | | | |
| 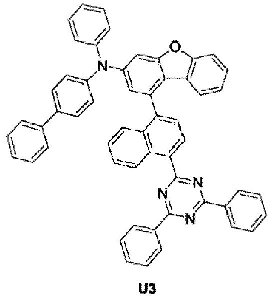 | 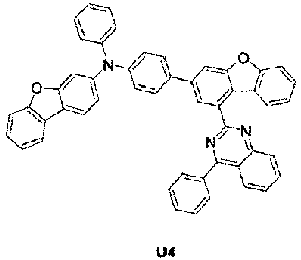 | 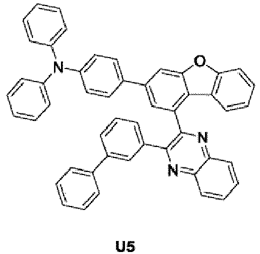 | | | |
| 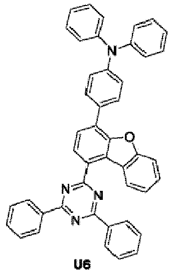 | 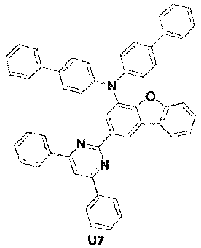 | 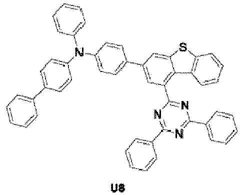 | | | |
| 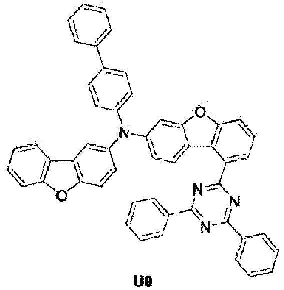 | 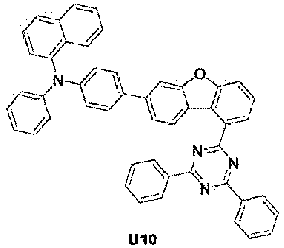 | 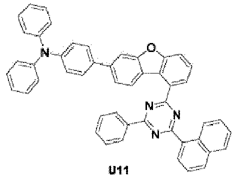 | | | |
| 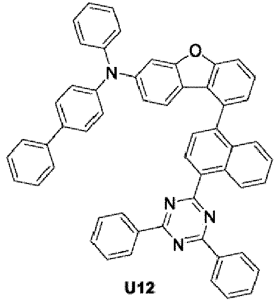 | 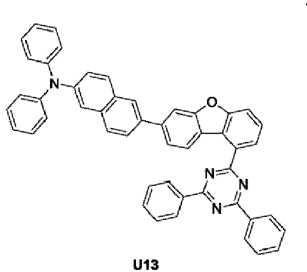 | 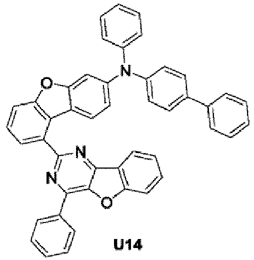 | | | |
| 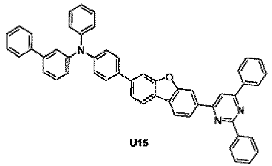 | 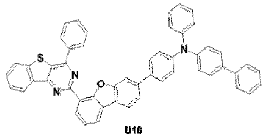 | 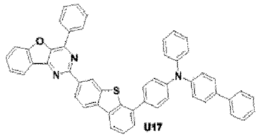 | | | |
| 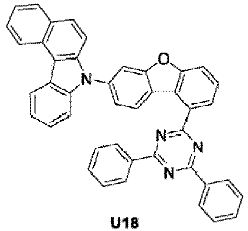 | 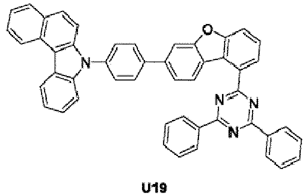 | 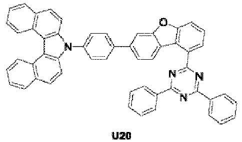 | | | |
| 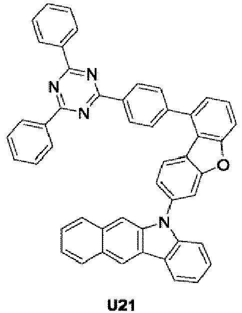 | 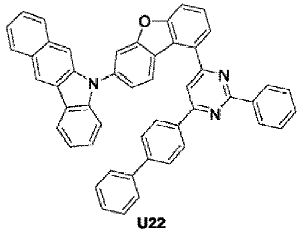 | 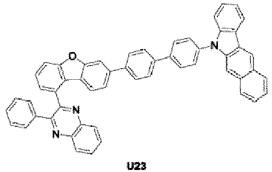 | | | |
| 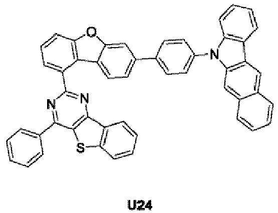 | 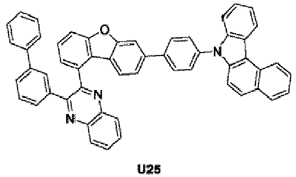 | 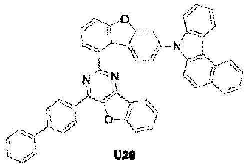 | | | |
| 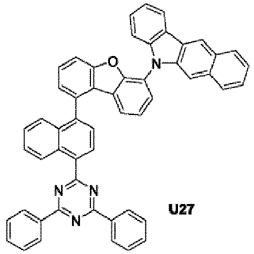 | 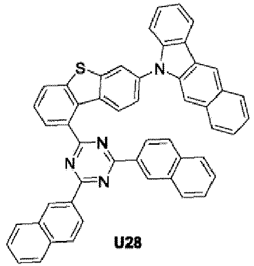 | 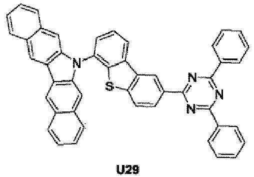 | | | |
| 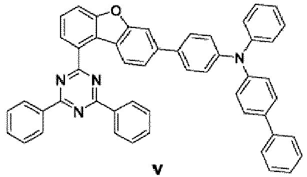 | 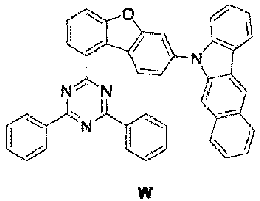 | 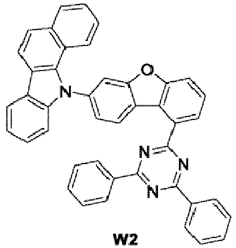 | | | |

As can be seen from the above device evaluation, it could be confirmed that when the heterocyclic compound of Chemical Formula 1 of the present application is used as the light emitting layer of the organic light-emitting device, an excellent effect of increase by 40% or more was exhibited in service life characteristic compared to a compound not substituted with deuterium.

When hydrogen is substituted with deuterium, the mass increases, but in this case, the compound has a lower vibrational energy than hydrogen to lower and stabilize the energy of the molecule. Therefore, since the bond dissociation energy is greater for carbon-deuterium bonds than for carbon-hydrogen bonds, it is determined that the structure of the molecule is more stable when substituted with deuterium, and thus the service life is affected.

The service lives of 1-89 and 1-90 in which the amine substituent is substituted with deuterium are better than those of 1-87 in dibenzofuran, which is the central skeleton of the compound and is substituted with deuterium and 1-88 in which a zine substituent is substituted with deuterium. This caused holes in the device to be formed while an oxidation-reduction reaction occurs between compounds, and due to the reaction that takes place in this case, the compounds are more damaged and the stability is lowered, which is one of the factors that shorten the service life of the device. The reason why the service life results of 1-89 and 1-90 were particularly excellent is that the kinetic isotope effect was observed by substituting the amine compound, which is an HT group involved in hole formation in the compound, with deuterium. (Chem. Commun., 2014,50, 14870-14872). Therefore, it is possible to confirm a better service life effect than when dibenzofuran and the azine substituent are substituted with deuterium.

### <Experimental Example 1-2>-Manufacture of organic light emitting device

A glass substrate, in which indium tin oxide (ITO) was thinly coated to have a thickness of 1,500 Å, was ultrasonically washed with distilled water. When the washing with distilled water was finished, the glass substrate was ultrasonically washed with a solvent such as acetone, methanol, and isopropyl alcohol, was dried and then was subjected to ultraviolet ozone (UVO) treatment for 5 minutes by using UV in an ultraviolet (UV) washing machine. Thereafter, the substrate was transferred to a plasma washing machine (PT), and then was subjected to plasma treatment in a vacuum state for an ITO work function and in order to remove a residual film, and was transferred to a thermal deposition apparatus for organic deposition.

The hole injection layer 4,4',4"-tris[2-naphthyl(phenyl)amino] triphenylamine (2-TNATA) and the hole transport layer N,N'N,N"-diphenyl-(1,1'-biphenyl)-4,4'-diamine (NPB), which are common layers, were formed on the ITO transparent electrode (positive electrode).

A light emitting layer was thermally vacuum deposited thereon as follows. The light emitting layer was deposited to have a thickness of 400 Å by using a compound described in the following Table 2 as a red host and (piq)2(Ir)(acac) as a red phosphorescent dopant to dope the host with (piq)2(Ir)(acac) in an amount of 2%.

Thereafter, Alq3 was deposited to have a thickness of 120 Å as an electron transport layer, Bphen was deposited to have a thickness of 120 Å as a charge generation layer thereon, and MoO3 was also deposited to have a thickness of 100 Å as a charge generation layer thereon. A hole transport layer N,N'-diphenyl-(1,1'-biphenyl)-4,4'-diamine (NPB) was formed.

A light emitting layer was thermally vacuum deposited thereon as follows. The light emitting layer was deposited to have a thickness of 400 Å by using a compound described in the following Table 7 as a red host and (piq)2(Ir)(acac) as a red phosphorescent dopant to dope the host with (piq)2(Ir)(acac) in an amount of 2%.

Thereafter, Alq3 was deposited to have a thickness of 300 Å as an electron transport layer. Finally, lithium fluoride (LiF) was deposited to have a thickness of 20 Å on the electron transport layer to form an electron injection layer, and then aluminum (Al) negative electrode was deposited to have a thickness of 1,200 Å on the electron injection layer to form a negative electrode, thereby manufacturing an organic electroluminescence device.

Meanwhile, all the organic compounds required for manufacturing an OLED device were subjected to vacuum sublimed purification under 10⁻⁶ to 10⁻⁸ torr for each material, and used for the manufacture of OLED. In order to measure the change in device performance depending on the deposition ratio of the compounds, deposition was performed up to 20 times by using the same deposition source. The deposition ratio of the compound according to the number of times of deposition was measured by using a high performance liquid chromatography (HPLC1260) manufactured by Agilent Technologies, Inc. for the compound deposited on the substrate.

For the organic electroluminescence device manufactured as described above, electroluminescence (EL) characteristics were measured by M7000 manufactured by McScience Inc., and based on the measurement result thereof, T90 was measured by a service life measurement equipment (M6000) manufactured by McScience Inc., when the reference luminance was 6,000 cd/m². Characteristics of the organic electroluminescence device of the present invention are as described in the following Table 7.

**[Table 7]**

| | Compound (P:N) | Deposition Method (P:N ratio) | Deposition Number | Driving voltage (Vₒₚ) | Efficiency (cd/A) | Color coordinate (x, y) | Service life (T₉₀) | Deposition Ratio (%) |
|---|---|---|---|---|---|---|---|---|
| Example 1 | 1-124 : 2-12 | Premixed (2:1) | 1 | 6.28 | 94.18 | (0.681, 0.315) | 1137 | 2.2 |
| Example 2 | | Premixed | 1 | 6.23 | 98.86 | (0.681, | 1005 | 1.9 |
| | | (1:2) | | | | 0.316) | | |
| Example 3 | | premixed (1:1) | 1 | 6.23 | 97.45 | (0.681, 0.316) | 1073 | 1.7 |
| Example 4 | | | 2 | 6.23 | 97.09 | (0.681, 0.316) | 1072 | 1.7 |
| Example 5 | | | 3 | 6.24 | 96.87 | (0.681, 0.315) | 1070 | 1.8 |
| Example 6 | | | 4 | 6.24 | 96.84 | (0.680, 0.316) | 1068 | 1.9 |
| Example 7 | | | 9 | 6.24 | 96.51 | (0.681, 0.316) | 1057 | 2.2 |
| Example 8 | | | 10 | 6.25 | 96.47 | (0.681, 0.315) | 1052 | 2.3 |
| Example 9 | | | 20 | 6.27 | 95.14 | (0.681, 0.316) | 1008 | 2.8 |
| Example 10 | 1-124 : 2-15 | premixed (1:1) | 1 | 6.25 | 96.58 | (0.681, 0.316) | 1180 | 3.5 |
| Example 11 | | | 2 | 6.25 | 96.54 | (0.681, 0.315) | 1177 | 3.6 |
| Example 12 | | | 3 | 6.26 | 96.11 | (0.680, 0.316) | 1174 | 3.6 |
| Example 13 | | | 4 | 6.26 | 95.99 | (0.680, 0.316) | 1169 | 3.7 |
| Example 14 | | | 10 | 6.28 | 95.01 | (0.681, | 1095 | 4.1 |
| | | | | | | 0.316) | | |
| Example 15 | | | 20 | 6.36 | 92.76 | (0.680, 0.316) | 997 | 4.9 |
| Example 16 | 3-21 : 1-161 | Premixed (2:1) | 1 | 6.35 | 92.18 | (0.680, 0.317) | 946 | 3.2 |
| Example 17 | | Premixed (1:2) | 1 | 6.33 | 94.86 | (0.681, 0.316) | 885 | 2.9 |
| Example 18 | | premixed (1:1) | 1 | 6.33 | 94.21 | (0.680, 0.316) | 911 | 2.8 |
| Example 19 | | | 2 | 6.33 | 94.09 | (0.680, 0.317) | 910 | 2.8 |
| Example 20 | | | 3 | 6.33 | 93.87 | (0.681, 0.316) | 908 | 2.9 |
| Example 21 | | | 4 | 6.34 | 93.84 | (0.680, 0.316) | 908 | 2.9 |
| Example 22 | | | 9 | 6.35 | 93.51 | (0.680, 0.317) | 891 | 3.2 |
| Example 23 | | | 10 | 6.35 | 93.47 | (0.681, 0.316) | 891 | 3.2 |
| Example 24 | | | 20 | 6.37 | 92.64 | (0.680, 0.316) | 887 | 3.6 |
| Example 25 | 3-22 : 1-161 | premixed (1:1) | 1 | 6.34 | 93.02 | (0.681, 0.315) | 1086 | 3.5 |
| Example 26 | | | 2 | 6.34 | 93.00 | (0.680, | 1084 | 3.5 |
| | | | | | | 0.316) | | |
| Example 27 | | | 3 | 6.35 | 92.98 | (0.680, 0.316) | 1078 | 3.6 |
| Example 28 | | | 4 | 6.36 | 92.85 | (0.681, 0.316) | 1072 | 3.7 |
| Example 29 | | | 10 | 6.43 | 91.00 | (0.680, 0.316) | 995 | 4.4 |
| Example 30 | | | 20 | 6.56 | 89.56 | (0.680, 0.316) | 891 | 5.6 |
| Comparative Example 1 | U14 : 2-12 | Co-dep (1:1) | 1 | 6.28 | 93.02 | (0.681, 0.315) | 698 | 5.4 |
| Comparative Example 2 | | premixed (1:1) | 1 | 6.26 | 95.33 | (0.681, 0.316) | 711 | 4.1 |
| Comparative Example 3 | | | 2 | 6.26 | 94.98 | (0.681, 0.315) | 709 | 4.5 |
| Comparative Example 4 | | | 3 | 6.27 | 94.02 | (0.680, 0.316) | 703 | 4.9 |
| Comparative Example 5 | | | 4 | 6.28 | 93.87 | (0.680, 0.316) | 697 | 5.3 |
| Comparative Example 6 | | | 9 | 6.34 | 86.78 | (0.681, 0.316) | 672 | 7.0 |
| Comparative Example 7 | | | 10 | 6.36 | 85.61 | (0.681, 0.316) | 669 | 7.5 |
| Comparative | | | 20 | 6.72 | 76.05 | (0.680, | 612 | 12.7 |
| Example 8 | | | | | | 0.316) | | |
| Comparative Example 9 | 3-21 : W | Co-dep (1:1) | 1 | 6.36 | 87.02 | (0.680, 0.317) | 685 | 6.3 |
| Comparative Example 10 | | premixed (1:1) | 1 | 6.35 | 92.32 | (0.680, 0.316) | 701 | 5.2 |
| Comparative Example 11 | | | 2 | 6.35 | 91.91 | (0.681, 0.315) | 701 | 5.4 |
| Comparative Example 12 | | | 3 | 6.36 | 89.64 | (0.681, 0.316) | 697 | 5.8 |
| Comparative Example 13 | | | 4 | 6.36 | 88.87 | (0.681, 0.315) | 690 | 6.2 |
| Comparative Example 14 | | | 9 | 6.43 | 83.78 | (0.680, 0.316) | 665 | 7.9 |
| Comparative Example 15 | | | 10 | 6.44 | 82.41 | (0.681, 0.315) | 654 | 8.5 |
| Comparative Example 16 | | | 20 | 6.81 | 73.04 | (0.680, 0.317) | 592 | 13.4 |

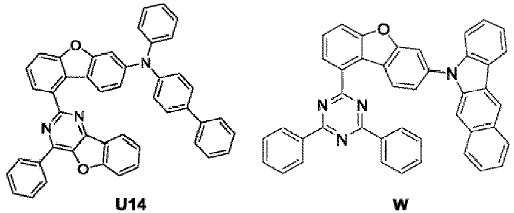

When the compound corresponding to Chemical Formula 1 of the present invention and the compound corresponding to Chemical Formula A or B of the present invention are combined and deposited on the device, in the co-host deposition (hereinafter referred to as co-dep) method of depositing the compound through each deposition source, the compound is generally formed in a non-uniform manner in the light emitting layer compared to the method in which the compound is premixed and then deposited through one evaporation source (hereafter, premixed).

When Comparative Examples 1 and 2 and Comparative Examples 9 and 10 in Table 6 are compared, it can be confirmed that premixed deposition is better than non-premixed deposition in all aspects of driving, efficiency, and service life. In the case of the premixed method, when one deposition source is used, the deposition ratio of the compounds may vary depending on the deposition temperature difference of the compounds. The difference in the deposition rate of each compound does not pose a big problem when the deposition is performed only once, but acts as a factor that may cause a problem in the mass production process in which devices are continuously produced. A smaller deposition ratio means that each compound is uniformly deposited on the device. Referring to Comparative Examples 2 to 8 and Comparative Examples 10 to 16, which are compounds which are not substituted with deuterium, it can be seen that the difference in deposition ratio increases as the number of times of deposition increases. In contrast, from Examples 3 to 9 and Examples 12 to 18 in which the compound is substituted with deuterium, it can be confirmed that the difference is not as large as in the case of substituting the compound with deuterium. This indicates that it is more advantageous to substitute the compound with deuterium during repeated manufacture of the device.

Through the phenomenon as described above, it was confirmed that materials using deuterium substitution help not only to improve service life due to increased stability, but also to improve mass productivity by using the control of the deposition rate of materials.

### [Explanation of Reference Numerals and Symbols]

- 100:: Substrate
- 200:: Positive electrode
- 300:: Organic material layer
- 301:: Hole injection layer
- 302:: Hole transport layer
- 303:: Light emitting layer
- 304:: Hole blocking layer
- 305:: Electron transport layer
- 306:: Electron injection layer
- 400:: Negative electrode

## Claims

1. A heterocyclic compound represented by the following Chemical Formula 1: 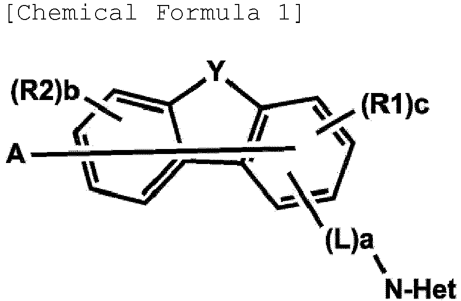 wherein, in Chemical Formula 1,
Y is O; or S,
N-Het is a heteroaryl group which is substituted or unsubstituted and includes at least one N,
L is a direct bond; a substituted or unsubstituted C6 to C60 arylene group; or a substituted or unsubstituted C2 to C60 heteroarylene group,
R1 and R2 are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R"; and a substituted or unsubstituted amine group, or two or more adjacent groups are bonded to each other to form a substituted or unsubstituted C6 to C60 aliphatic or aromatic hydrocarbon ring, or a substituted or unsubstituted C2 to C60 aliphatic or aromatic hetero ring,
b is an integer from 0 to 4, c is an integer from 0 to 3, and b and c satisfy 0≤b+c ≤6,
a is an integer from 0 to 4,
when a, b and c are 2 or higher, substituents in the parenthesis are the same as or different from each other,
A is represented by the following Chemical Formula 1-1 or 1-2, 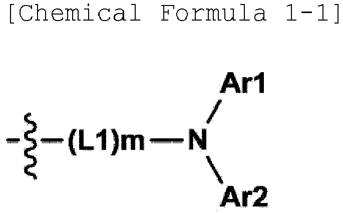 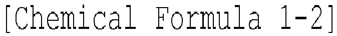 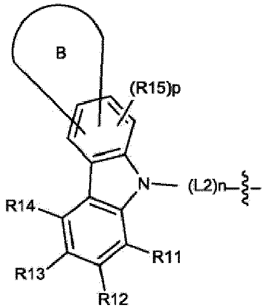
in Chemical Formulae 1-1 and 1-2,
Ar1 and Ar2 are the same as or different from each other, and are each independently selected from the group consisting of a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R"; and a substituted or unsubstituted amine group,
L1 and L2 are the same as or different from each other, and are each independently a direct bond; a substituted or unsubstituted C6 to C60 arylene group; or a substituted or unsubstituted C2 to C60 heteroarylene group,
R11 to R15 are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R"; and a substituted or unsubstituted amine group, or two or more adjacent groups are bonded to each other to form a substituted or unsubstituted C6 to C60 aliphatic or aromatic hydrocarbon ring, or a substituted or unsubstituted C2 to C60 aliphatic or aromatic hetero ring,
B is a substituted or unsubstituted C6 to C60 aliphatic or aromatic hydrocarbon ring; or a substituted or unsubstituted C2 to C60 aliphatic or aromatic hetero ring,
p is an integer from 0 to 2,
m and n are an integer from 0 to 4,
when p is 2 and when m and n are 2 or higher, substituents in the parenthesis are the same as or different from each other,
R, R' and R" are the same as or different from each other, and are each independently a substituted or unsubstituted C1 to C60 alkyl group; a substituted or unsubstituted C6 to C60 aryl group; or a substituted or unsubstituted C2 to C60 heteroaryl group, and
the deuterium content of Chemical Formula 1 is more than 0% and 100% or less.

2. The heterocyclic compound of claim 1, wherein Chemical Formula 1 is represented by the following Chemical Formula 2 or 3: 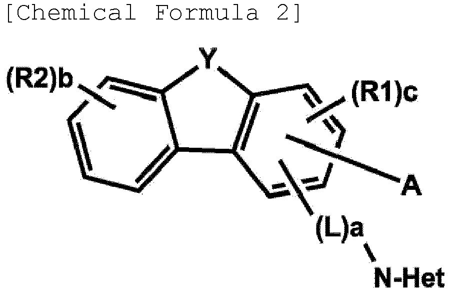 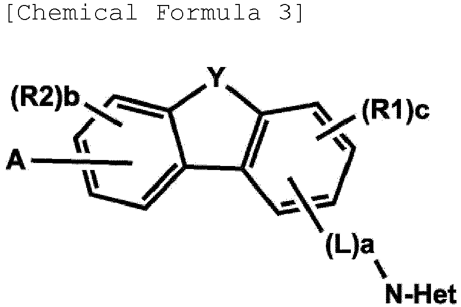 in Chemical Formulae 2 and 3,
the definitions of R1, R2, Y, L, N-Het, A, a, b and c are the same as the definitions in Chemical Formula 1.

3. The heterocyclic compound of claim 1, wherein Chemical Formula 1 is divided into and represented by the following Structural Formula A, Structural Formula B and Structural Formula C, and the deuterium content of the following Structural Formula A; the following Structural Formula B; the following Structural Formula C; the following Structural Formula A and the following Structural Formula B; the following Structural Formula A and the following Structural Formula C; the following Structural Formula B and the following Structural Formula C; and the following Structural Formulae A to C is 100%. 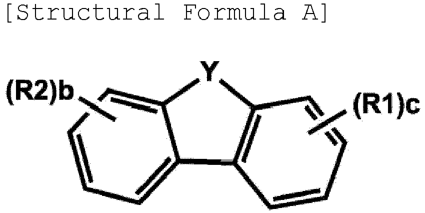 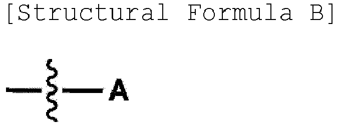 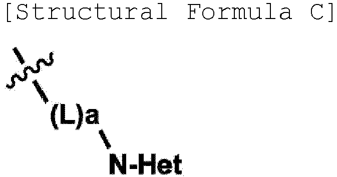 in Structural Formulae A to C,
the definition of each substituent is the same as the definition in Chemical Formula 1, and 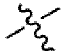 of Structural Formulae B and C each means a position bonded to Structural Formula A.

4. The heterocyclic compound of claim 1, wherein the deuterium content of the heterocyclic compound of Chemical Formula 1 is 20% to 100%.

5. The heterocyclic compound of claim 1, wherein Chemical Formula 1 is represented by any one of the following compounds:

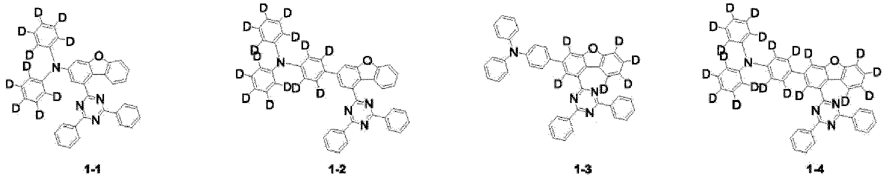

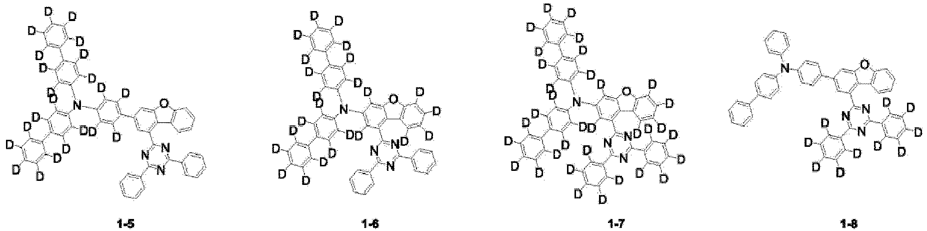

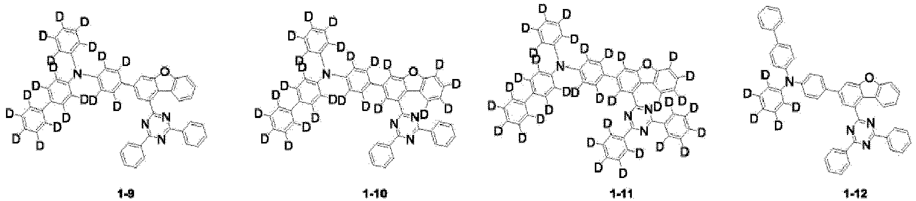

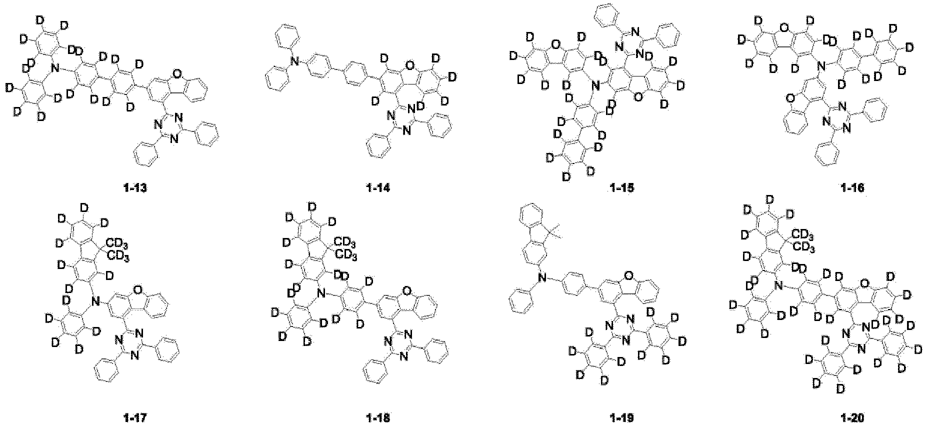

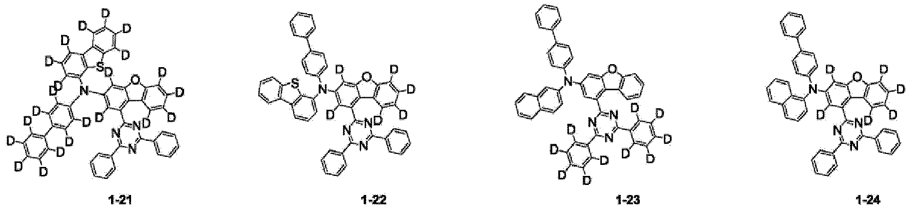

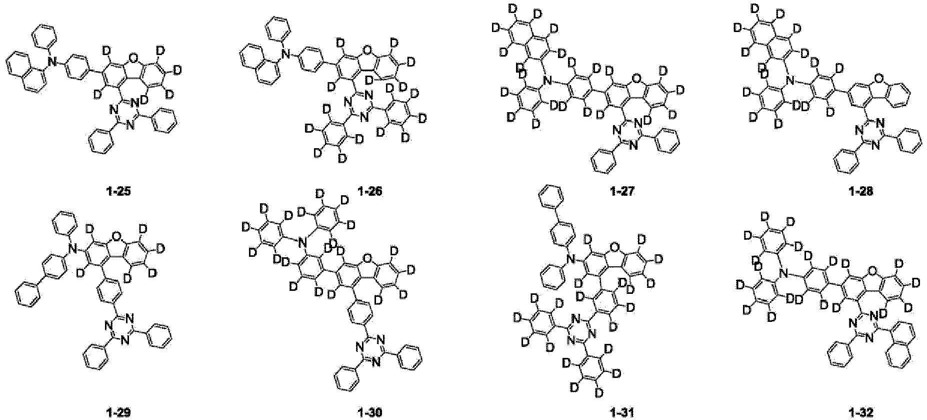

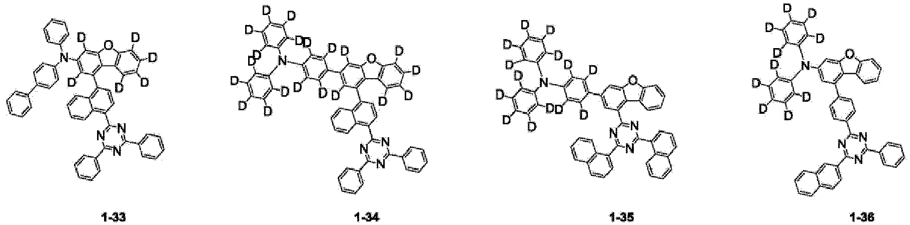

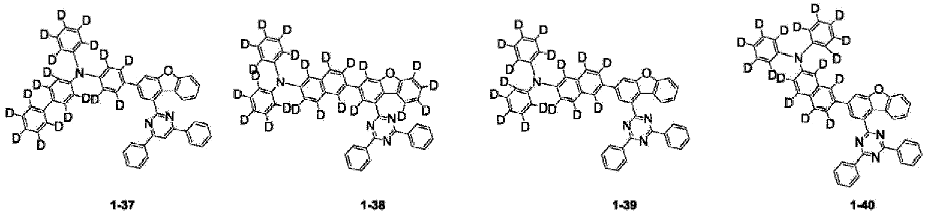

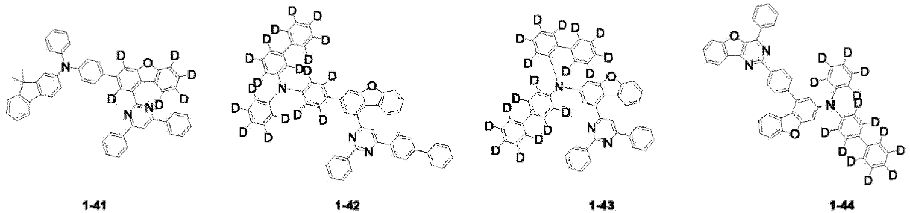

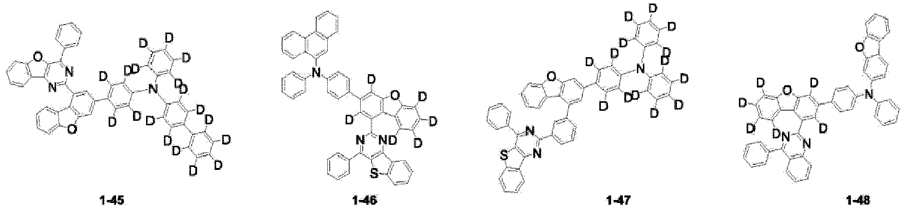

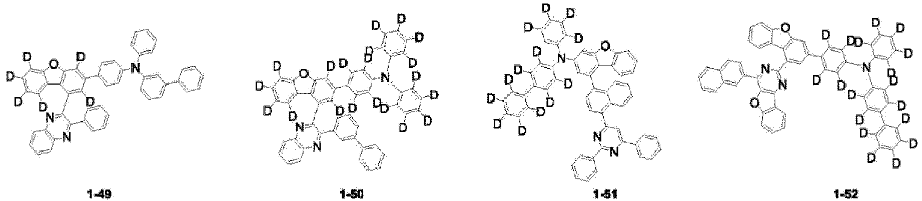

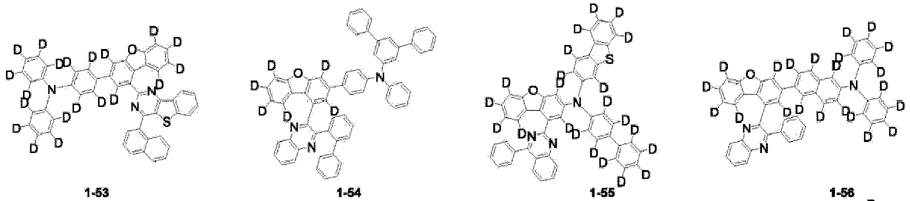

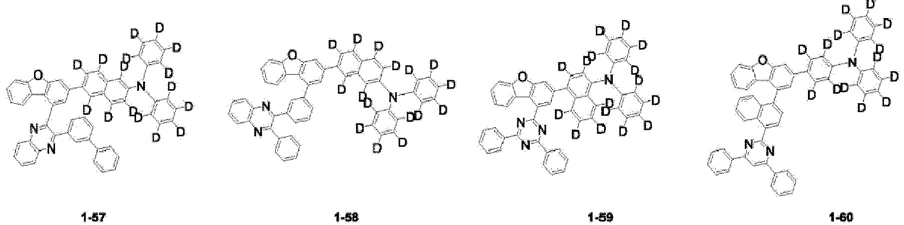

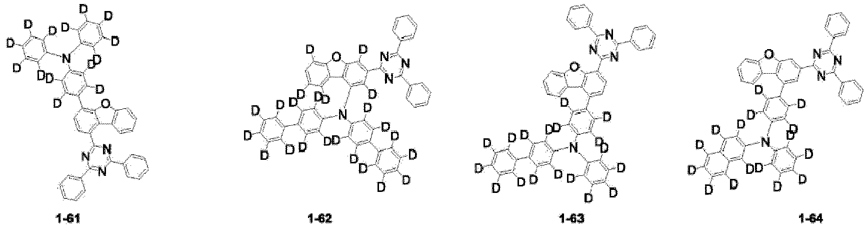

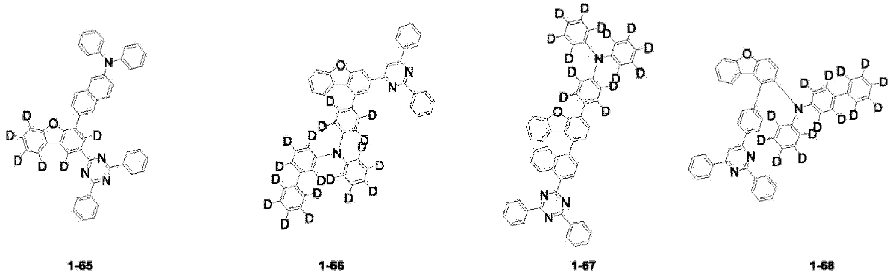

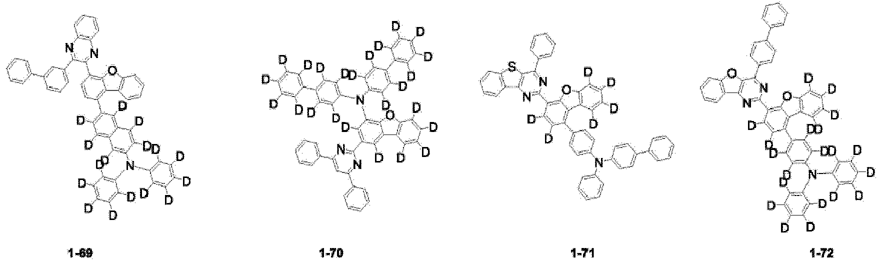

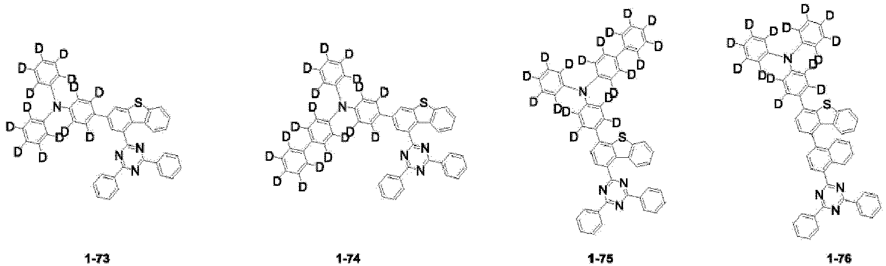

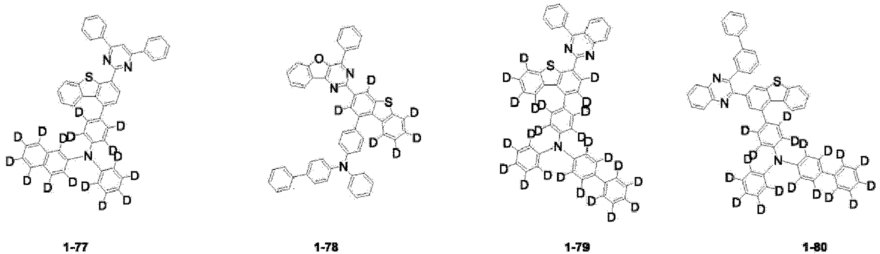

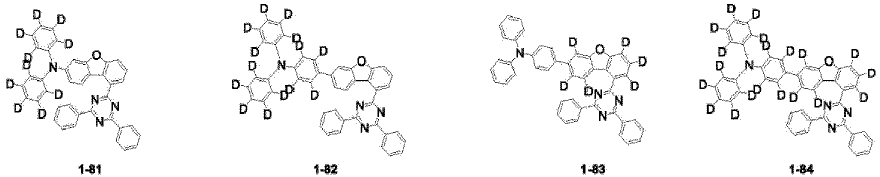

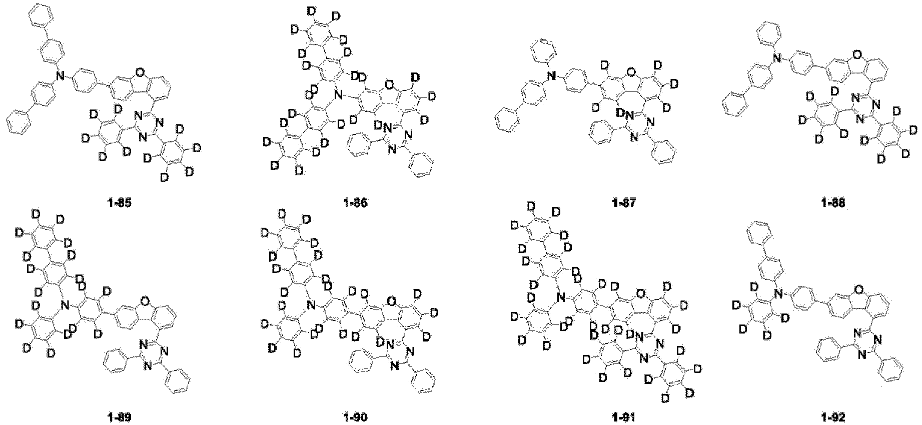

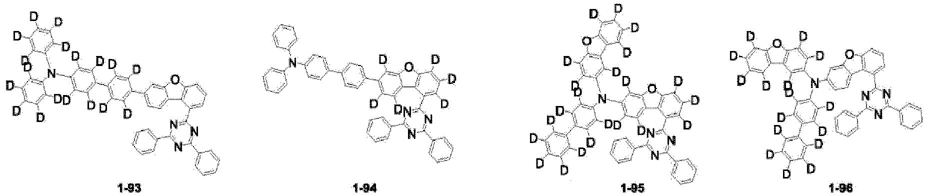

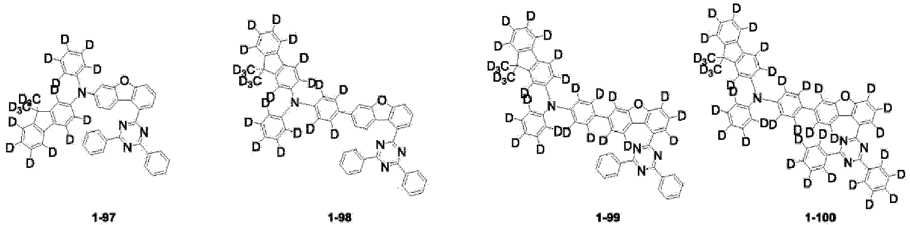

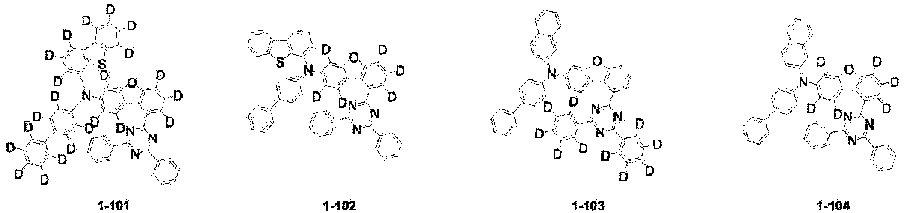

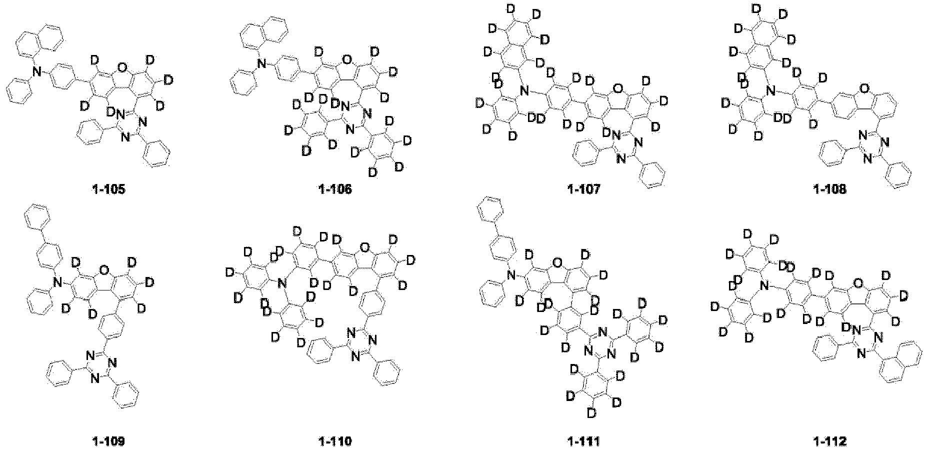

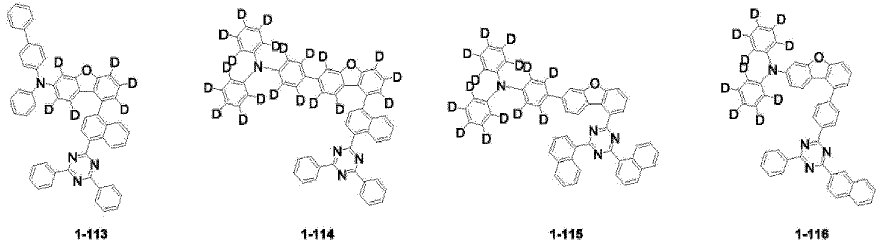

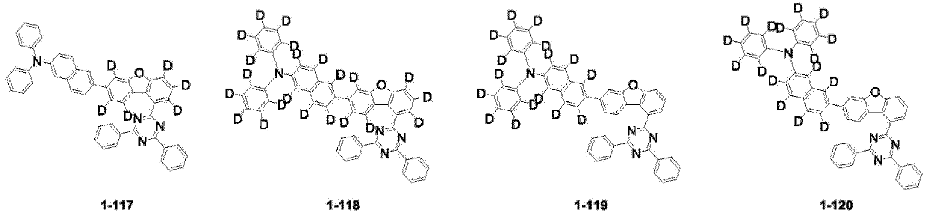

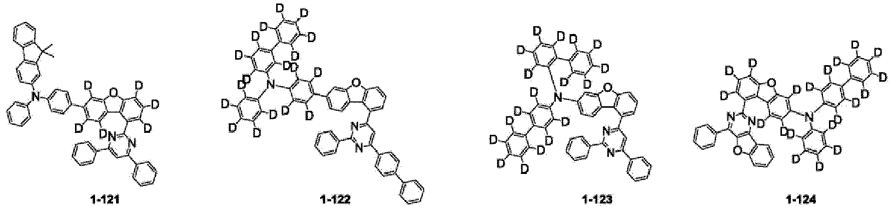

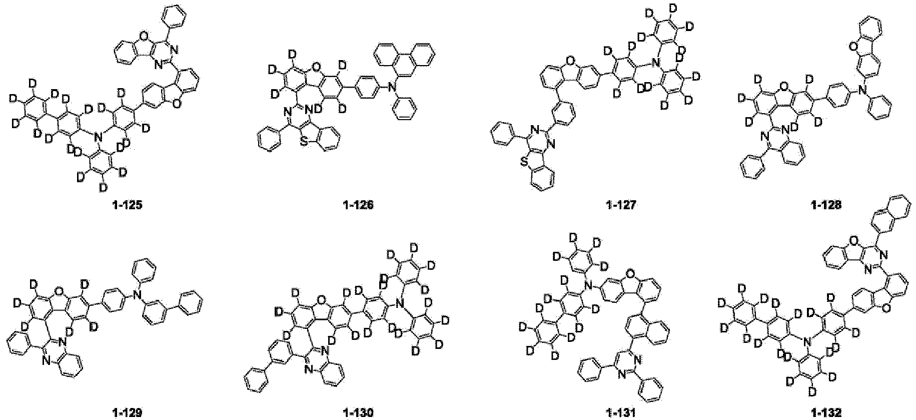

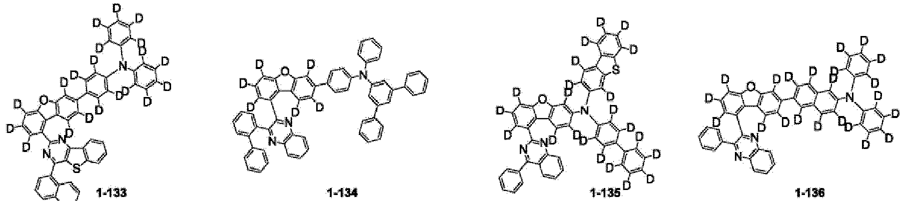

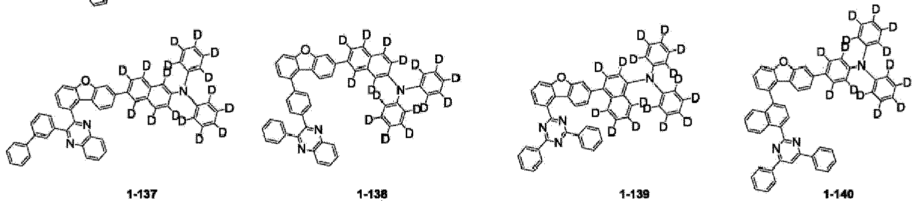

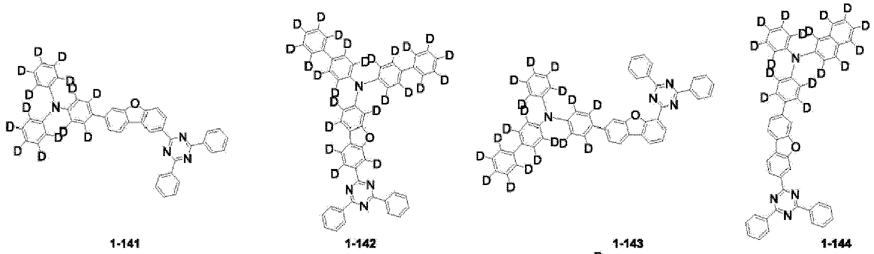

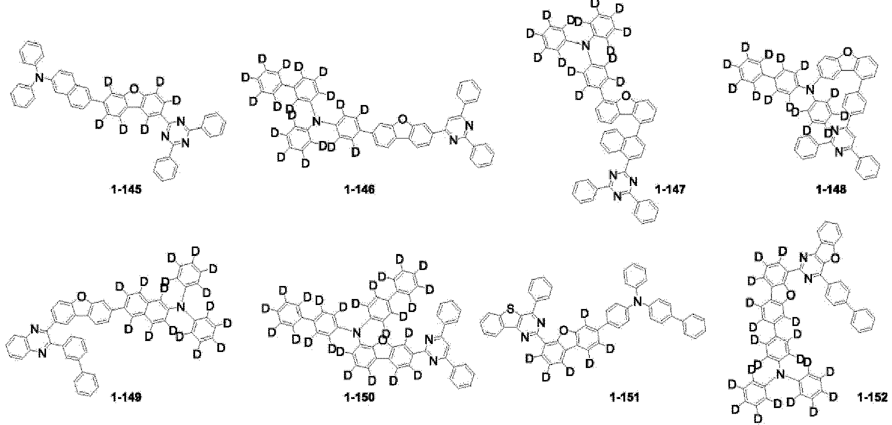

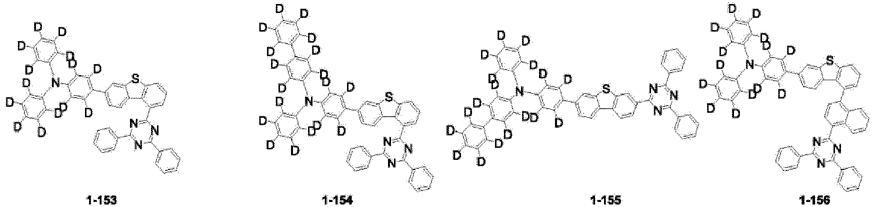

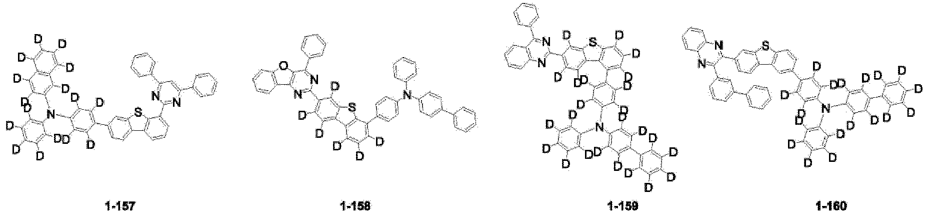

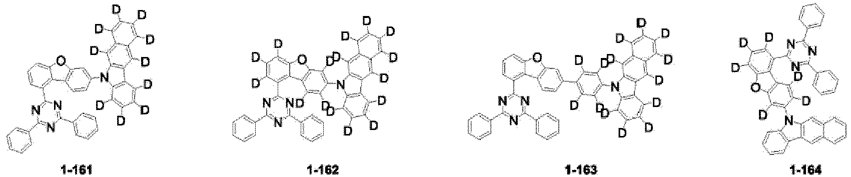

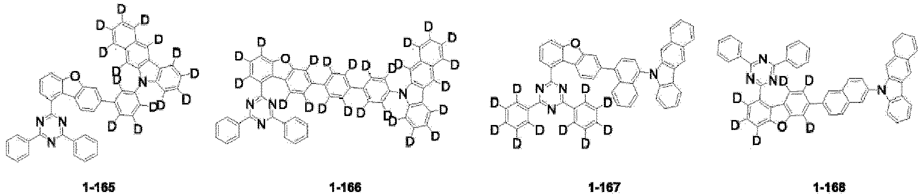

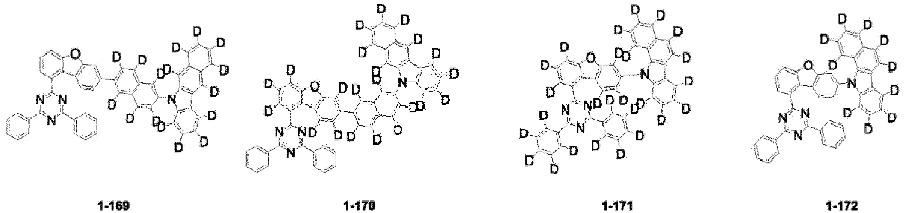

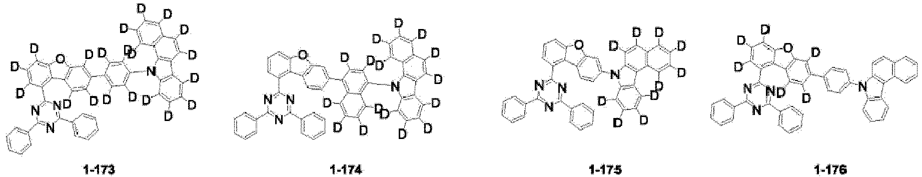

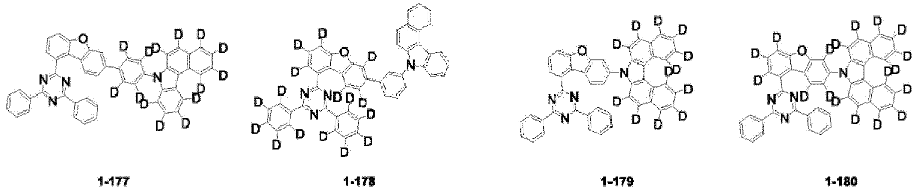

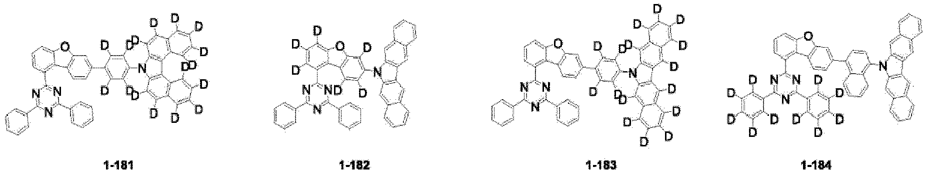

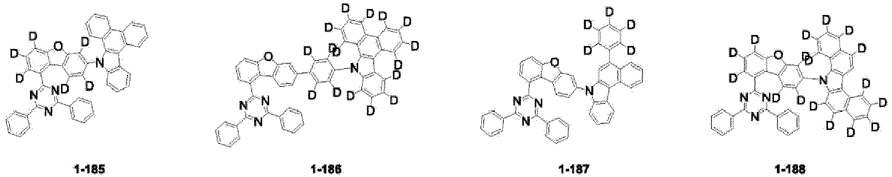

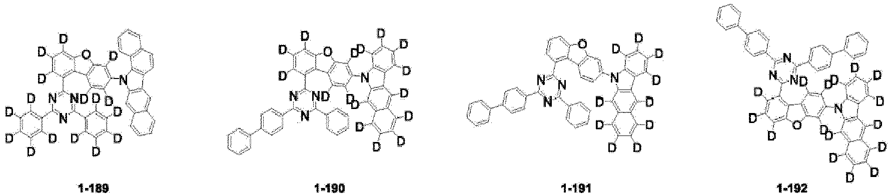

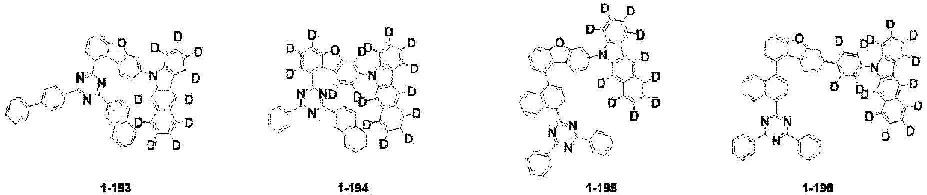

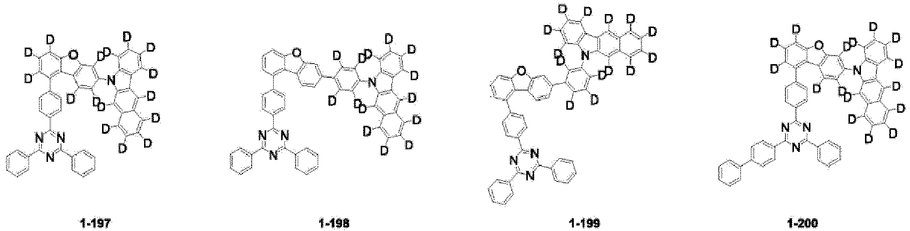

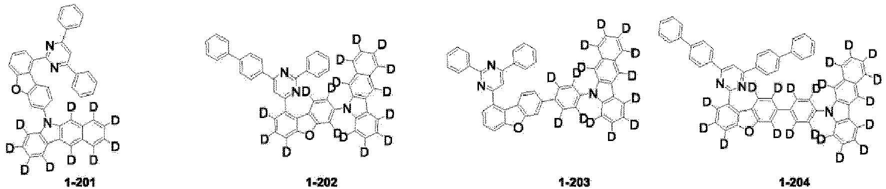

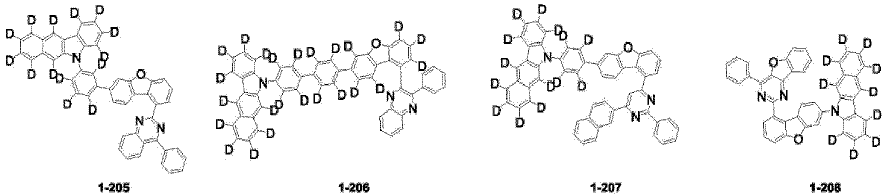

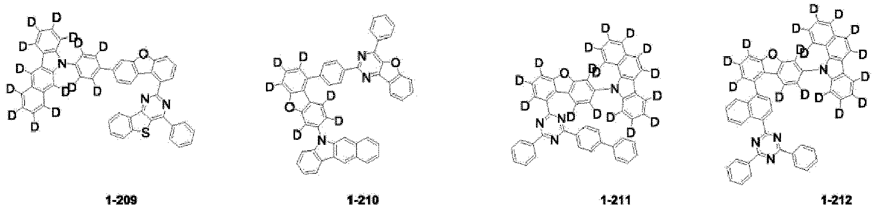

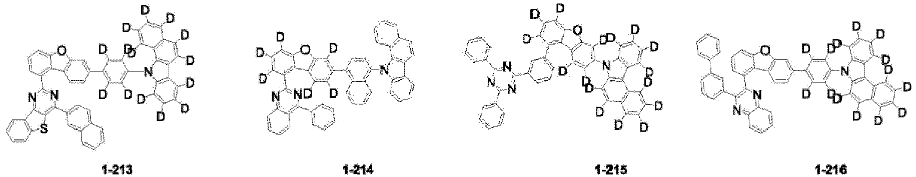

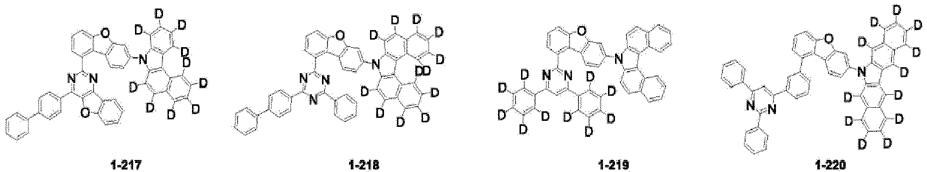

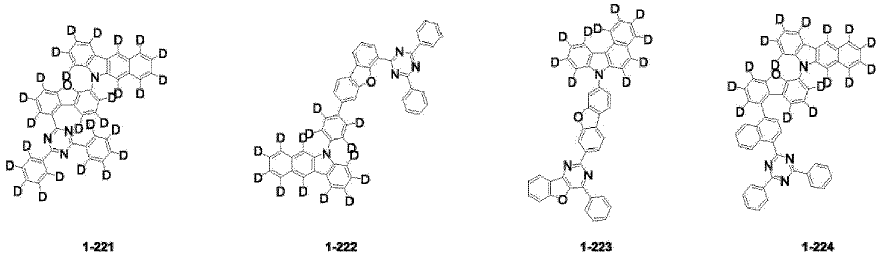

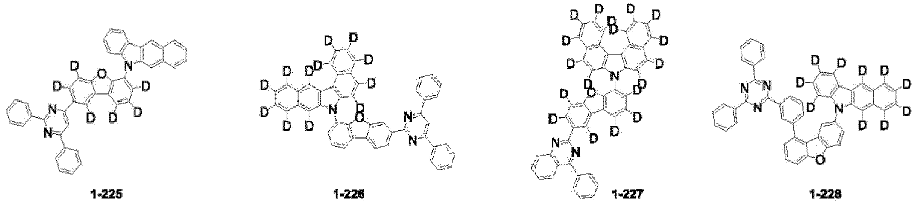

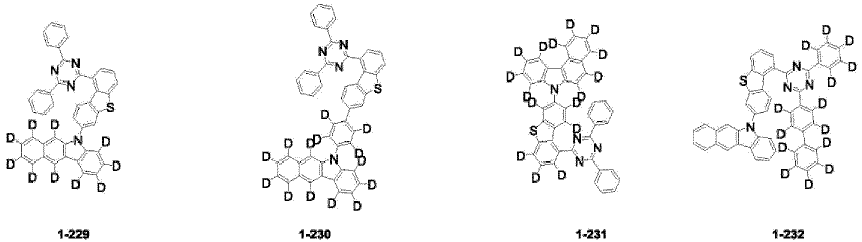

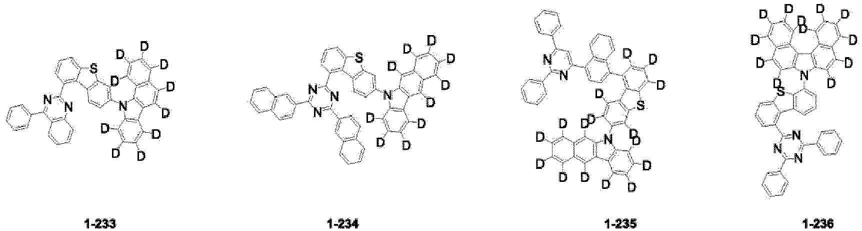

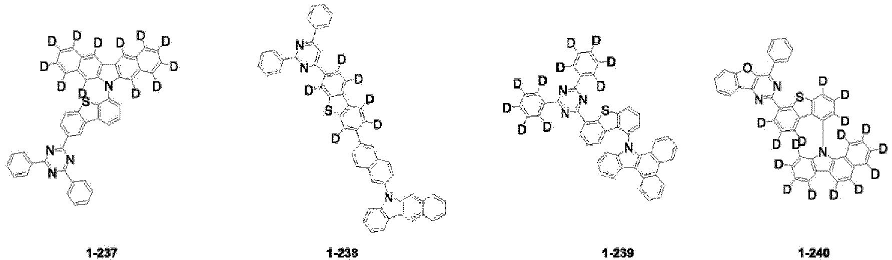

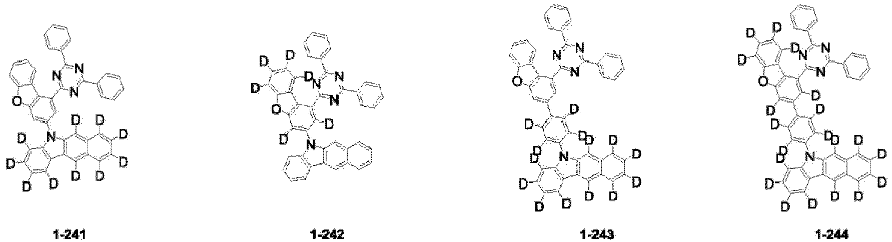

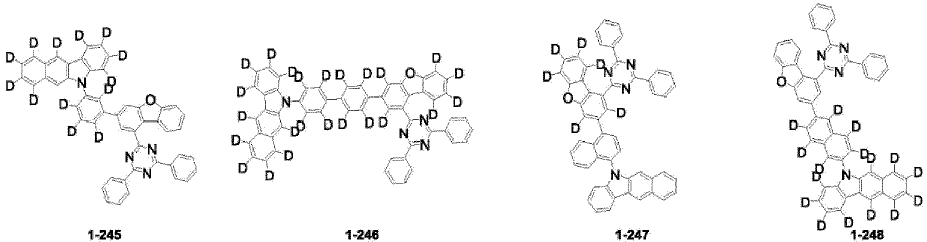

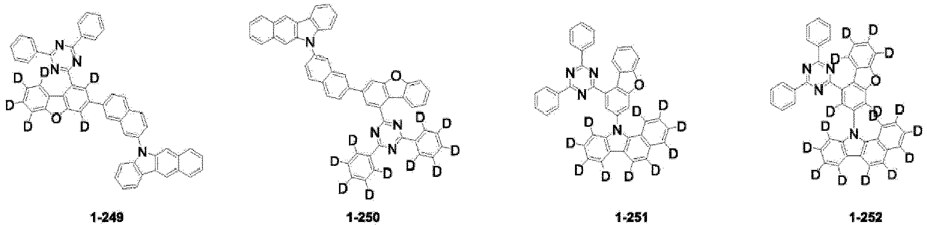

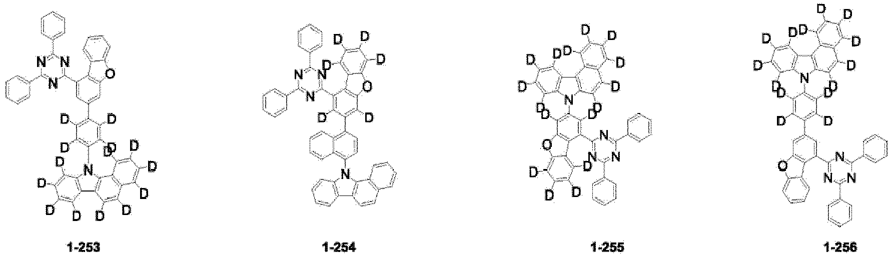

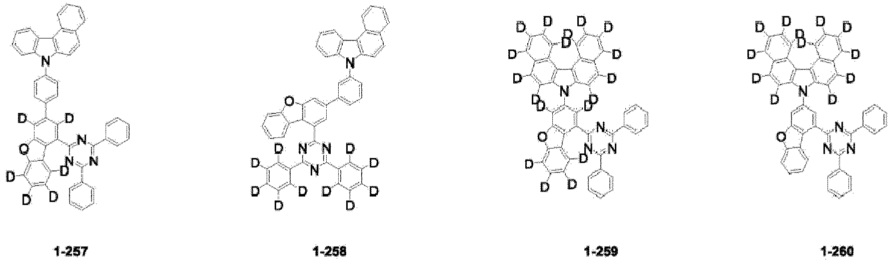

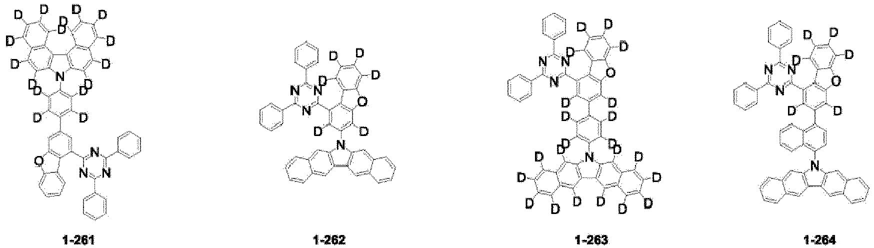

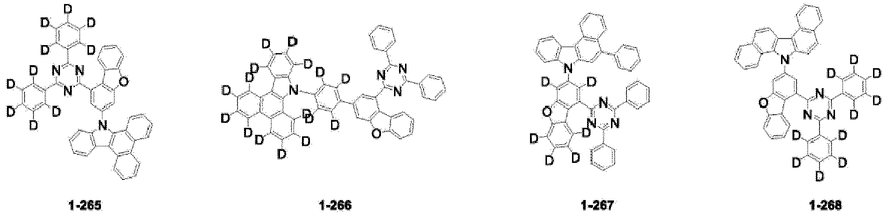

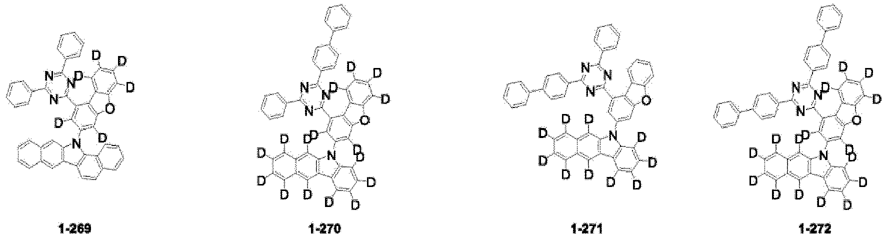

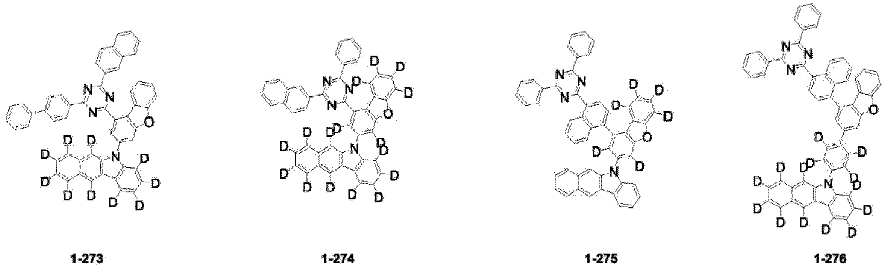

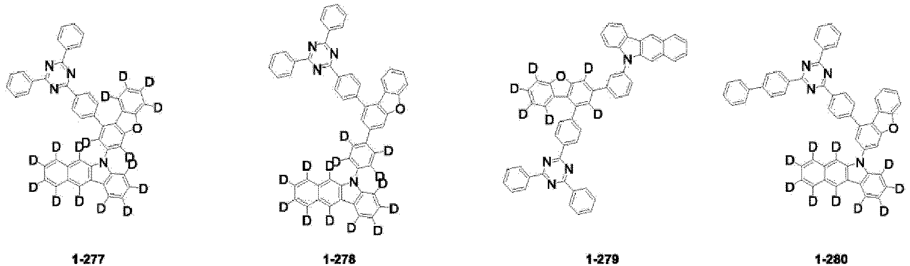

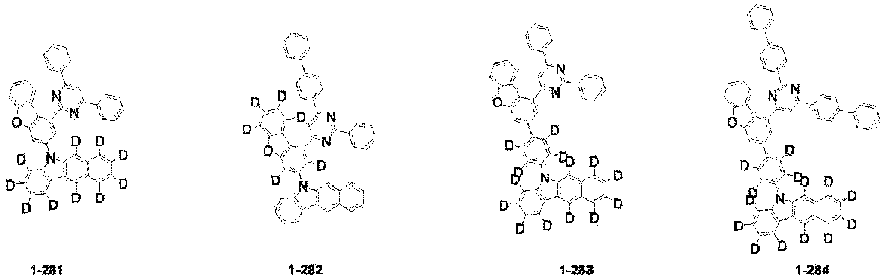

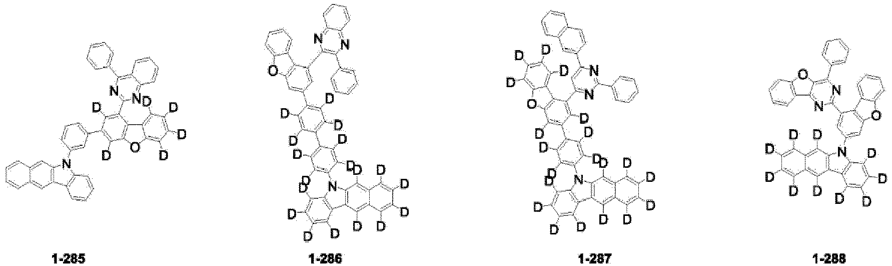

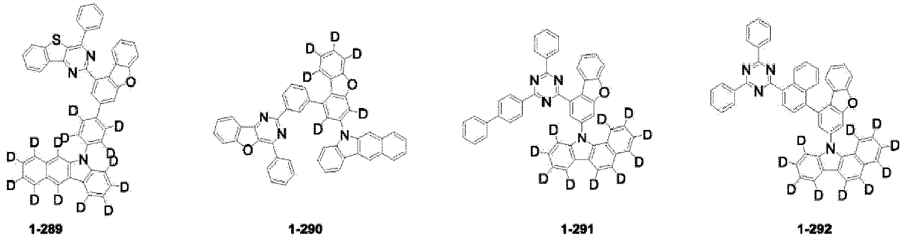

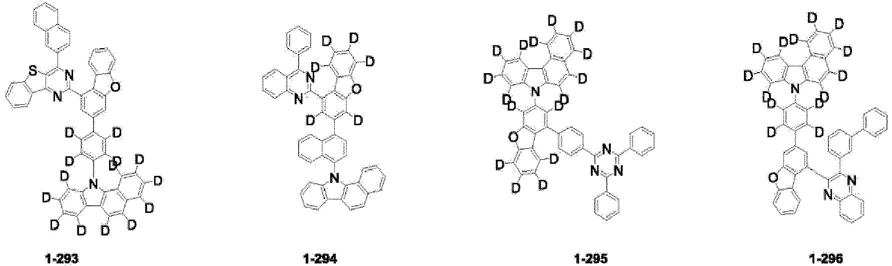

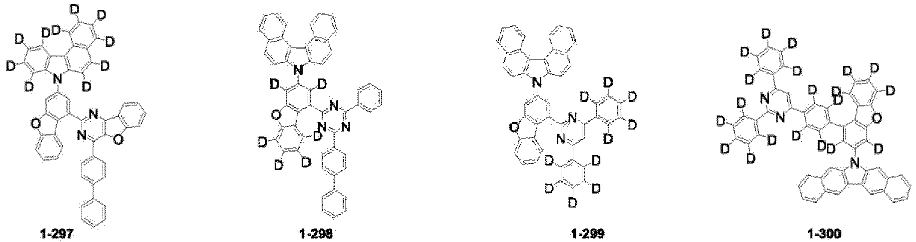

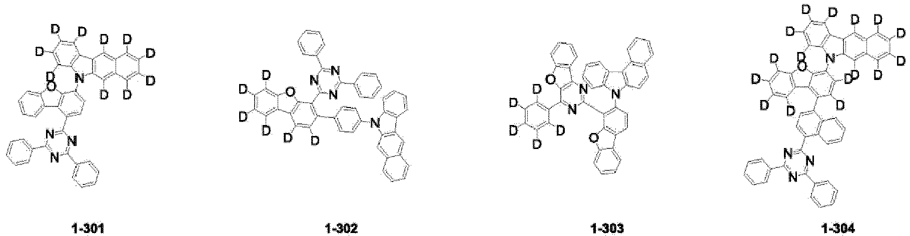

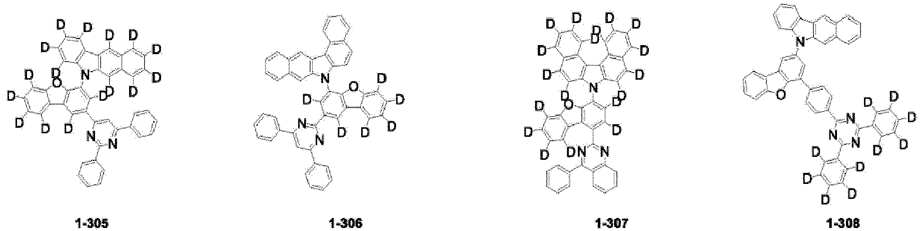

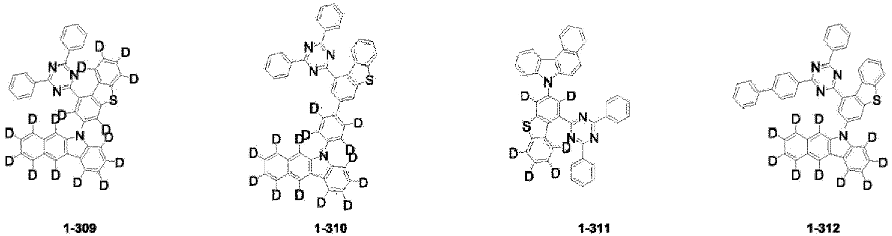

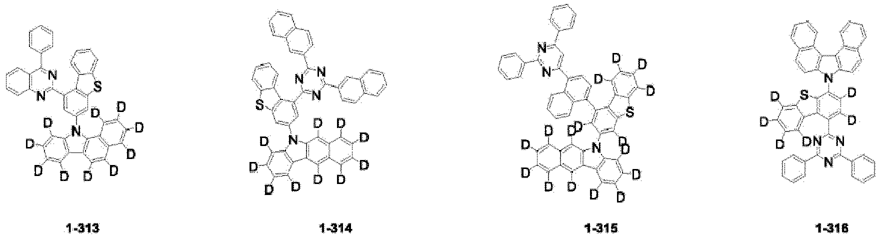

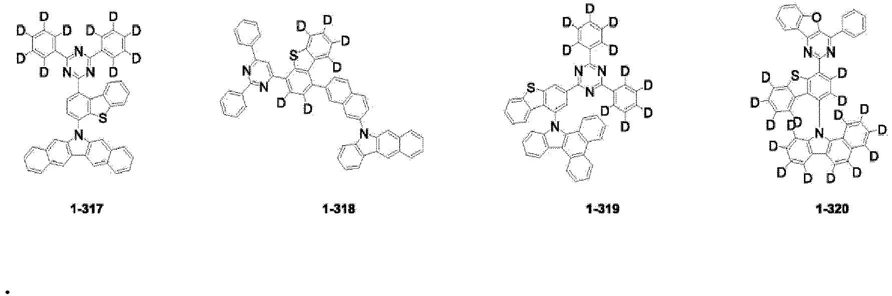

6. An organic light emitting device comprising:
a first electrode;
a second electrode provided to face the first electrode; and
an organic material layer having one or more layers provided between the first electrode and the second electrode,
wherein one or more layers of the organic material layer comprise one or more of the heterocyclic compound according to any one of claims 1 to 5.

7. The organic light emitting device of claim 6, wherein the organic material layer comprises a light emitting layer, and the light emitting layer comprises the heterocyclic compound.

8. The organic light emitting device of claim 6, wherein the organic material layer comprises a light emitting layer, the light emitting layer comprises a host material, and the host material comprises the heterocyclic compound.

9. The organic light emitting device of claim 6, further comprising one or two or more layers selected from the group consisting of a light emitting layer, a hole injection layer, a hole transport layer, an electron injection layer, an electron transport layer, an electron blocking layer and a hole blocking layer.

10. The organic light emitting device of claim 6, wherein the organic material layer comprising the heterocyclic compound comprises: a compound represented by the following Chemical Formula A; or a compound represented by the following Chemical Formula B: 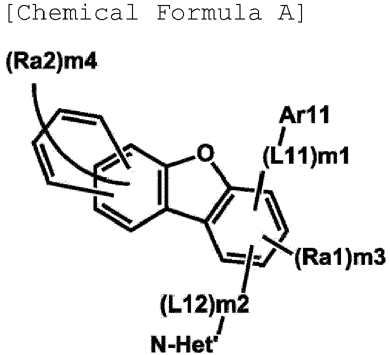 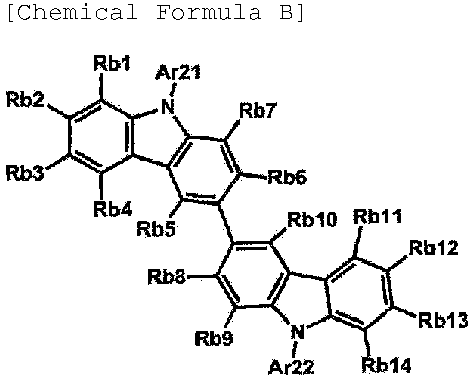 in Chemical Formulae A and B,
Ra1, Ra2 and Rb1 to Rb14 are the same as or different from each other, and are each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group, or two or more adjacent groups are bonded to each other to form a substituted or unsubstituted C6 to C60 aromatic hydrocarbon ring or a substituted or unsubstituted C2 to C60 hetero ring.
L11 and L12 are the same as or different from each other, and are each independently a direct bond; a substituted or unsubstituted C6 to C60 arylene group; or a substituted or unsubstituted C2 to C60 heteroarylene group,
N-Het' is a heteroaryl group which is substituted or unsubstituted and includes at least one N,
Ar11, Ar21 and Ar22 are the same as or different from each other, and each independently a substituted or unsubstituted C6 to C60 aryl group; or a substituted or unsubstituted C2 to C60 heteroaryl group,
m1 and m2 are an integer from 0 to 4,
m3 is an integer from 0 to 2,
m4 is an integer from 0 to 6, and
when m1, m2 and m4 are an integer or 2 or higher, or m3 is an integer of 2, substituents in the parenthesis are the same as or different from each other.

11. The organic light emitting device of claim 10, wherein the deuterium content of Chemical Formula A and Chemical Formula B is 0% or more and 100% or less.

12. The organic light emitting device of claim 10, wherein Chemical Formula A is represented by any one of the following compounds:

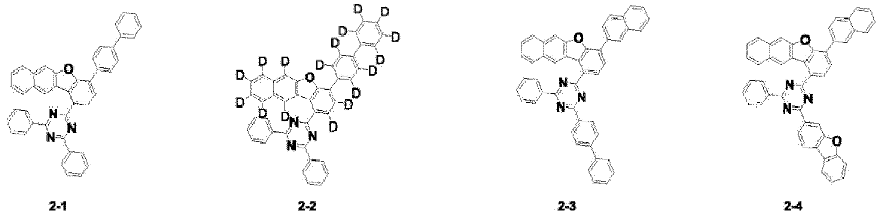

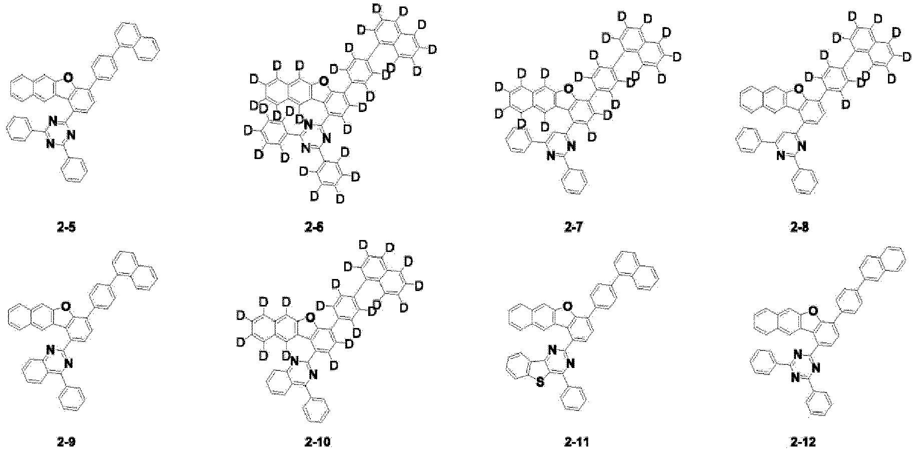

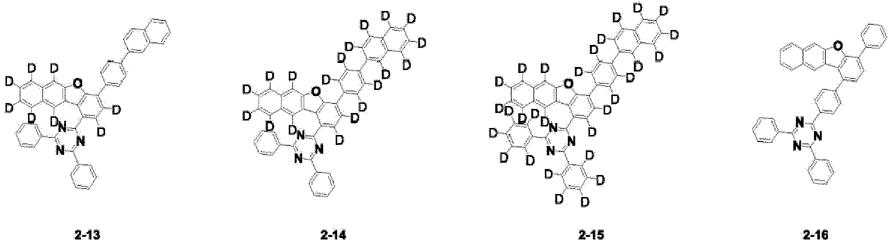

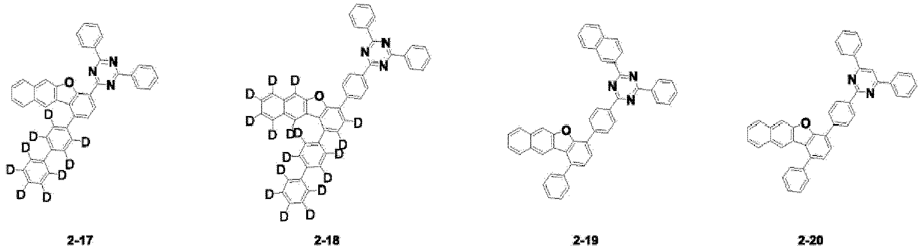

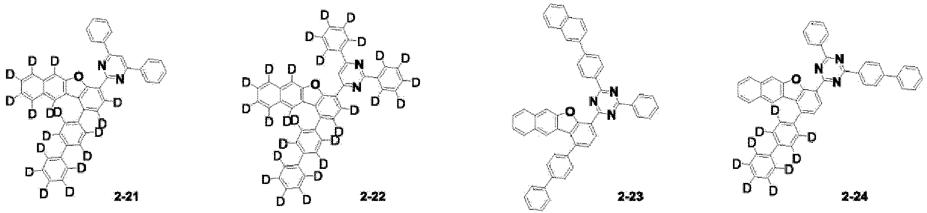

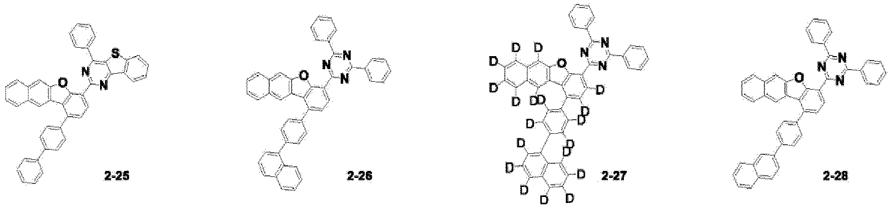

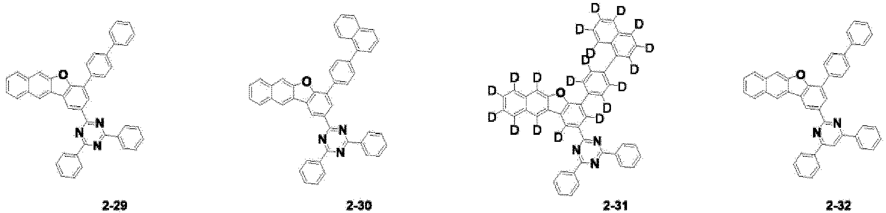

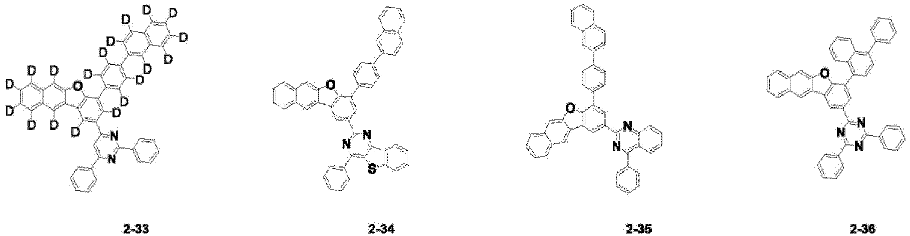

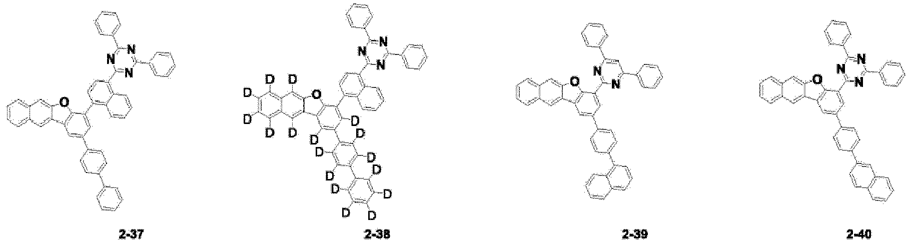

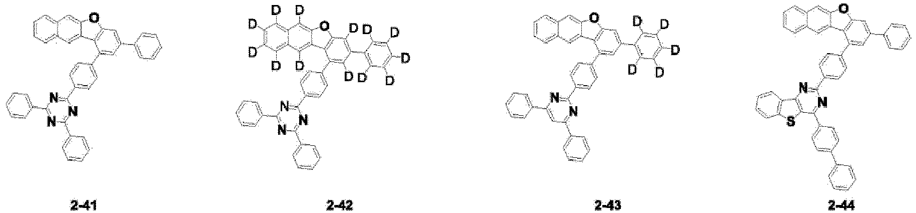

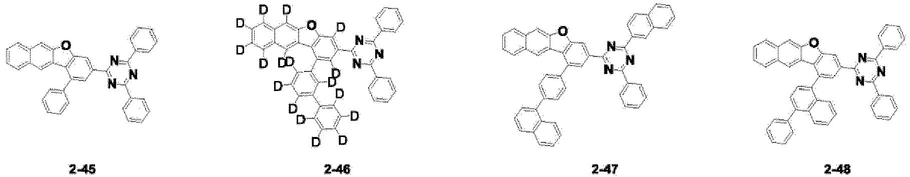

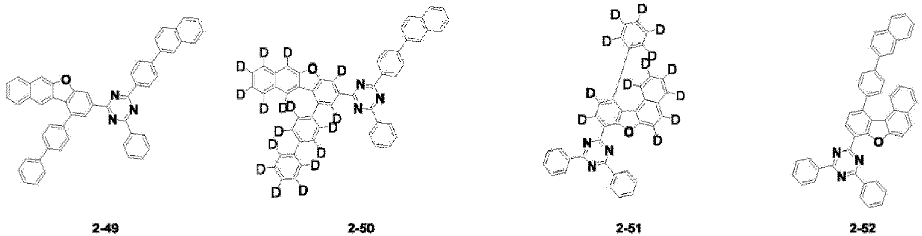

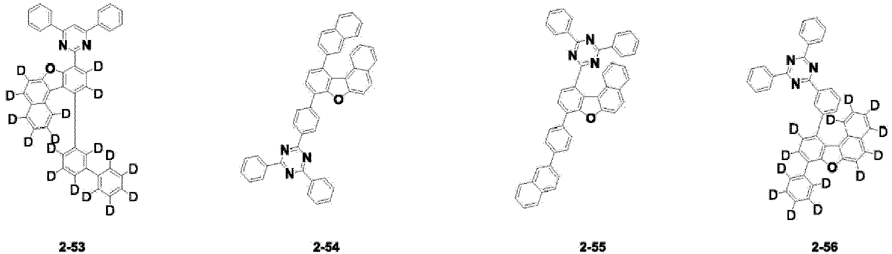

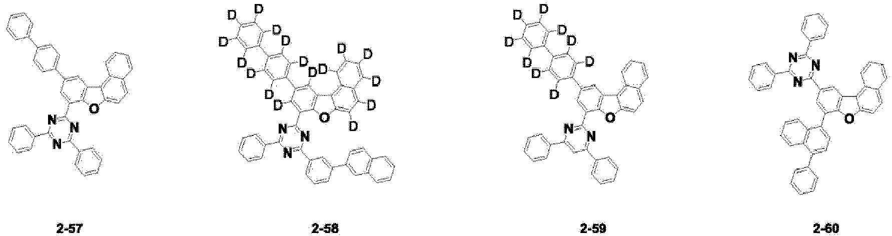

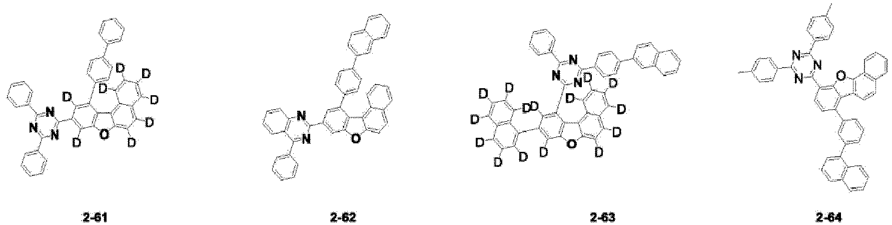

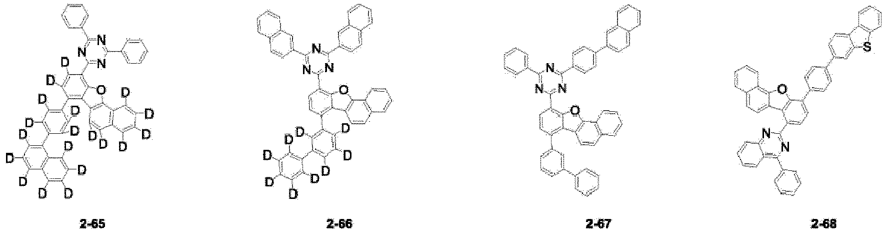

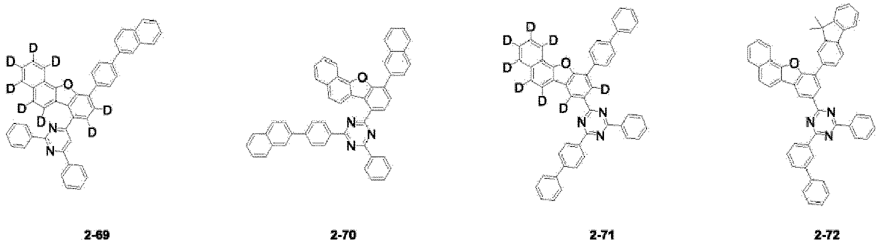

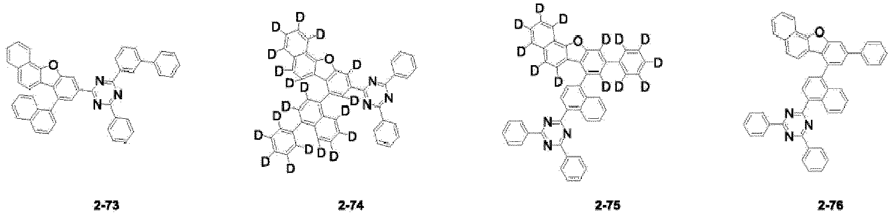

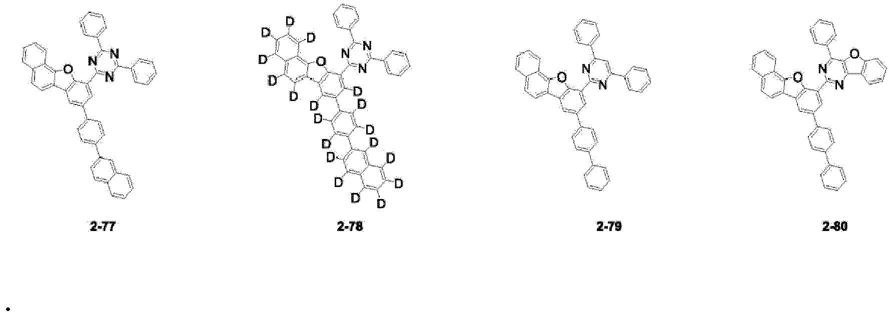

13. The organic light emitting device of claim 10, wherein Chemical Formula B is represented by any one of the following compounds:

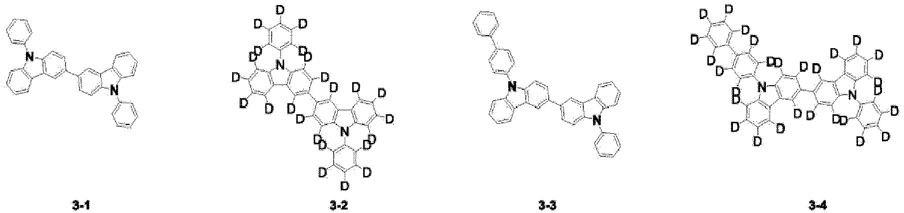

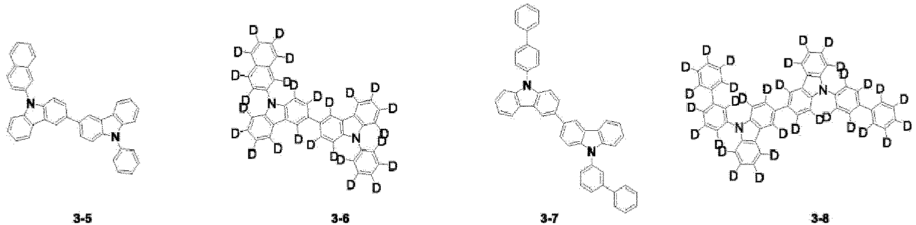

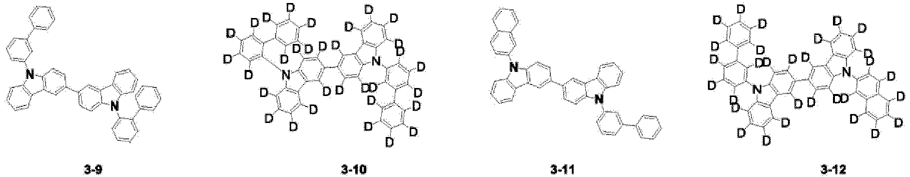

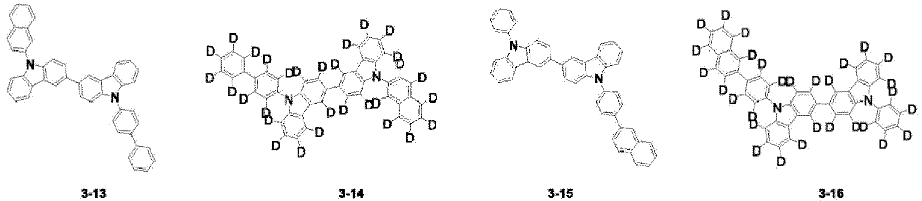

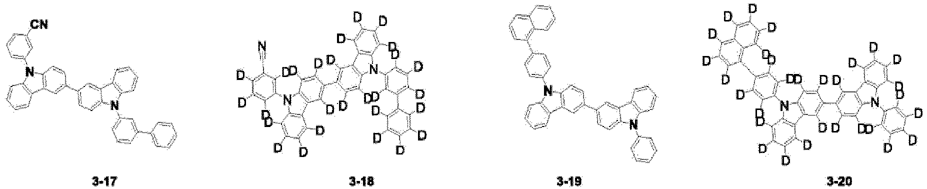

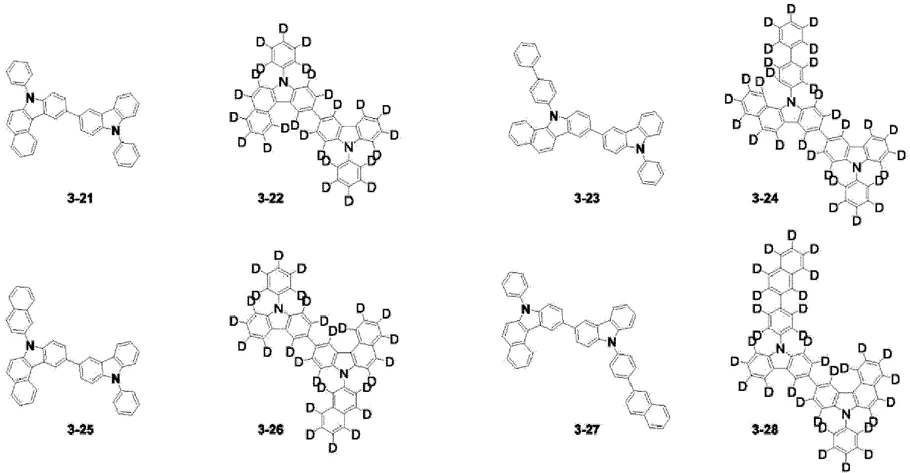

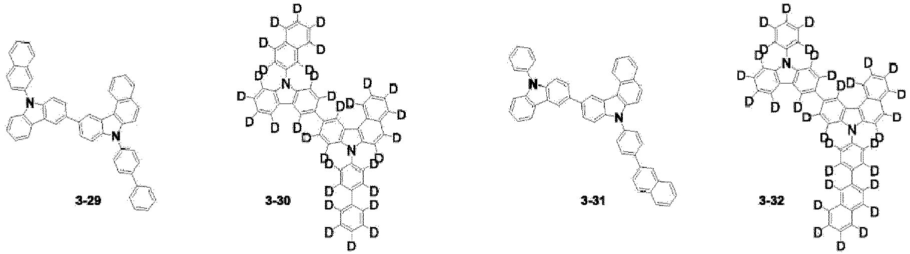

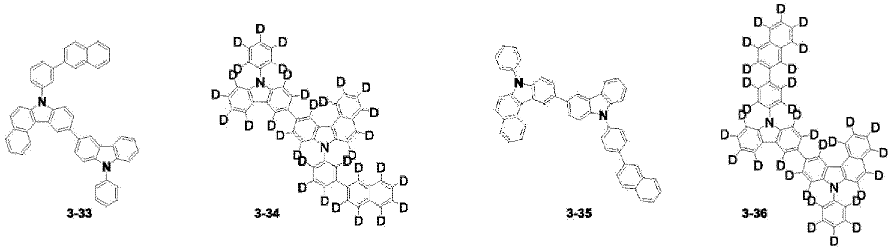

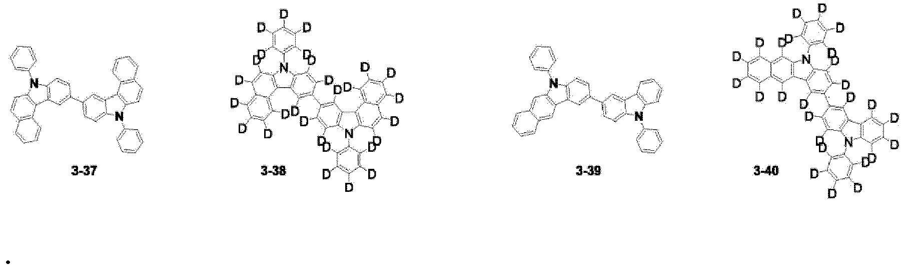

14. A composition for an organic material layer of an organic light emitting device, comprising:
a heterocyclic compound represented by Chemical Formula 1 according to any one of claims 1 to 5; and
a compound represented by the following Chemical Formula A or a compound represented by the following Chemical Formula B: 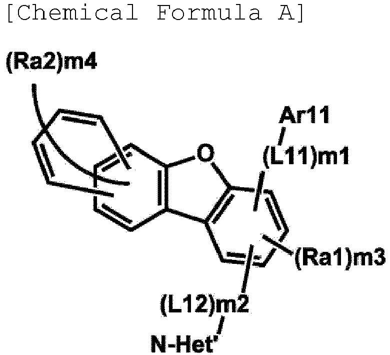 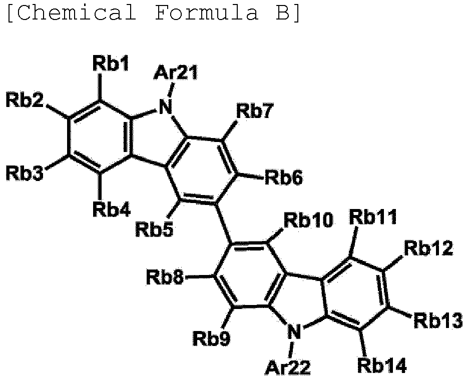 in Chemical Formulae A and B,
Ra1, Ra2 and Rb1 to Rb14 are the same as or different from each other, and are each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group, or two or more adjacent groups are bonded to each other to form a substituted or unsubstituted C6 to C60 aromatic hydrocarbon ring or a substituted or unsubstituted C2 to C60 hetero ring,
L11 and L12 are the same as or different from each other, and are each independently a direct bond; a substituted or unsubstituted C6 to C60 arylene group; or a substituted or unsubstituted C2 to C60 heteroarylene group,
N-Het' is a heteroaryl group which is substituted or unsubstituted and includes at least one N,
Ar11, Ar21 and Ar22 are the same as or different from each other, and each independently a substituted or unsubstituted C6 to C60 aryl group; or a substituted or unsubstituted C2 to C60 heteroaryl group,
m1 and m2 are each an integer from 0 to 4,
m3 is an integer from 0 to 2,
m4 is an integer from 0 to 6, and
when m1, m2 and m4 are an integer or 2 or higher, or m3 is an integer of 2, substituents in the parenthesis are the same as or different from each other.

15. The composition of claim 14, wherein a weight ratio of the heterocyclic compound represented by Chemical Formula 1 : the compound represented by Chemical Formula A or the compound represented by Chemical Formula B in the composition is 1 : 10 to 10 : 1.
